(19) Europäisches Patentamt
European Patent Office
Office européen des brevets

(11) EP 3 321 300 B1

(12) **EUROPÄISCHE PATENTSCHRIFT**

(45) Veröffentlichungstag und Bekanntmachung des Hinweises auf die Patenterteilung:
**08.01.2020 Patentblatt 2020/02**

(21) Anmeldenummer: **17199935.2**

(22) Anmeldetag: **03.11.2017**

(51) Int Cl.:
***C08G 18/66*** *(2006.01)* ***C08G 18/73*** *(2006.01)*
***C08G 18/75*** *(2006.01)* ***C08G 18/76*** *(2006.01)*
***C08G 18/08*** *(2006.01)* ***C08G 18/32*** *(2006.01)*
***C08G 18/42*** *(2006.01)*

(54) **PARTIKELFÖRMIGES FESTSTOFF-KOMPOSITMATERIAL ZUR NUKLEINSÄUREAUFREINIGUNG, ENTHALTEND MAGNETISCHE NANOPARTIKEL**

PARTICULATE SOLID COMPOSITE MATERIAL FOR NUCLEIC ACID PURIFICATION COMPRISING MAGNETIC NANOPARTICLES

MATÉRIAU COMPOSITE SOLIDE PARTICULAIRE DESTINÉ À LA PURIFICATION D'ACIDE NUCLÉIQUE, CONTENANT DES NANOPARTICULES MAGNÉTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.11.2016 DE 102016121483**

(43) Veröffentlichungstag der Anmeldung:
**16.05.2018 Patentblatt 2018/20**

(73) Patentinhaber: **AXAGARIUS GmbH & Co. KG**
**52355 Düren (DE)**

(72) Erfinder:
- **Prangenberg, Thomas**
  **53757 Sankt Augustin (DE)**
- **Riering, Helmut**
  **50169 Kerpen (DE)**

(74) Vertreter: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) Entgegenhaltungen:
**US-A1- 2005 014 001**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung liegt auf dem technischen Gebiet der Nukleinsäureaufreinigung und betrifft die Bereitstellung eines magnetischen Feststoff-Kompositmaterials auf Basis eines durch Polyaddition erhältlichen Polymers. Das Kompositmaterial lässt sich für die Aufreinigung von Nukleinsäuren in wässrigem Medium einsetzen. Gegenstand der Erfindung ist das spezielle magnetische Feststoff-Kompositmaterial, dessen Herstellverfahren, die Verwendung dieser magnetischen Feststoff-Kompositpartikel zur Isolierung von Nukleinsäuren und ein geeignetes Verfahren sowie ein Kit zur Bereitstellung von Material für die Nukleinsäureaufreinigung.

Die Analytik von Biomolekülen stellt oft sowohl in der Diagnostik als auch in biochemischen Verfahren einen wichtigen Verfahrensschritt dar. Meist sind die Isolierung und die nachfolgende Analytik von Biomolekülen aus biologischem Material, insbesondere von Nukleinsäuren, ein mehrschrittiger Prozess. In diesem Prozess werden zuerst die zu analysierenden Biomoleküle aus dem biologischen Material, wie z.B. aus Geweben, Bakterien, Zellen oder Viren, gewonnen. Dabei muss zunächst das gewünschte Biomolekül im Rahmen der sogenannten Lyse aus dem Inneren des biologischen Materials freigesetzt werden. Die Freisetzung kann beispielsweise mittels chaotroper Reagenzien, Tensiden, French-Press, Hitzeschock, Gefriertrocknung, Scherkräften, Enzymen, Ultraschall erfolgen.

In weiteren Schritten wird das freigesetzte Biomolekül von den restlichen Bestandteilen des biologischen Materials abgetrennt und isoliert. Für diesen Zweck kennt der Fachmann mehrere Verfahren, wie z.B. die Ionenaustauschchromatographie, die Affinitätschromatographie oder das sogenannte Bind-Wash-Elute-Verfahren.

Nachteilig an der Ionenaustauschchromatographie oder der Affinitätschromatographie als Reinigungsmethode für Nukleinsäuren als Biomolekül ist, dass zur Aufbereitung des Eluats die eluierte DNA umständlich entsalzt, aufkonzentriert und vom Elutionsmittel befreit werden muss, bevor sie für die nachfolgende Weiterverarbeitung oder für nachfolgende Analysen zur Verfügung stehen kann. Darüber hinaus sind chromatographische Festphasen teuer in der Herstellung, was die Ionenaustauschchromatographie unwirtschaftlich macht. Im Rahmen eines "Bind-Wash-Elute"-Verfahrens werden die Biomoleküle gegebenenfalls gemeinsam mit kontaminierenden Verbindungen aus einer flüssigen Probe auf ein Trägermaterial abgeschieden und die abgeschiedenen Bestandteile der Probe durch Einsatz von mindestens einer Waschlösung von einem Großteil der kontaminierenden Verbindungen befreit. Am Schluss wird das abgeschiedene Biomolekül vom Trägermaterial heruntergelöst.

Als Trägermaterial für die Abscheidung der Biomoleküle, insbesondere der Nukleinsäuren, eignen sich bekanntermaßen mineralische Trägerpartikel (wie beispielsweise Quarzfasern, Silica, Glas, Aluminiumoxid, Zeolith, Titandioxid, Zirkondioxid) und organische Polymerpartikel.

Zudem kennt der Fachmann magnetische Trägermaterialien, sogenannte magnetische Beads, auf die das gewünschte Biomolekül abgeschieden werden kann. Die beladenen magnetischen Beads können anschließend mit mindestens einer Waschlösung gewaschen werden. Nach Abschluss des Waschvorganges können die beladenen magnetischen Beads per Magnetseparation durch Anlegen eines Magnetfeldes gesammelt werden. Die zur Aufreinigung genutzte Waschlösung, in der sich die herausgewaschenen kontaminierenden Verbindungen befinden, kann so dann von den im angelegten Magnetfeld fixierten magnetischen Beads getrennt werden. Auf diese Weise werden die kontaminierenden Verbindungen entfernt.

Aus der Druckschrift EP 1 266 385 B1 kennt der Fachmann ein Verfahren zur Herstellung magnetischer Beads auf $SiO_2$-Basis mit einer porösen Oberfläche. Dazu werden ferro- bzw. ferrimagnetische Eisenoxidpartikel mit Durchmessern im Bereich von 75 bis 300 nm in Glycerol oder Glycol dispergiert und mit $SiO_2$ beschichtet. Anschließend werden die einzelnen beschichteten Kerne zu größeren Partikeln agglomeriert. Ein ähnliches Verfahren wird in EP 2 916 327 A1 zur Herstellung von ferrimagnetischen, silanisierten Eisenoxidpartikeln aufgezeigt. Die dabei eingesetzten Eisenoxidpartikel besitzen einen Kern aus $Fe_3O_4$ und eine Hülle aus $Fe_2O_3$.Die magnetischen Partikel werden darin in Glycol als Lösungsmittel bei erhöhten Temperaturen (250°C) hergestellt und weisen eine bevorzugte Größe von 100 nm auf.

In der Druckschrift WO 83/03920 A1 wird die Herstellung kugelförmiger Partikel auf Basis von Polystyrol und Polyacrylaten beschrieben. Es werden durch radikalisch initiierte Suspensionspolymerisation zuerst sphärische Polymerpartikel gebildet, welche anschließend unter definierten Bedingungen in einem organischen Lösungsmittel aufgequollen werden. Die aufgequollenen Polymerpartikel werden dann mit einer Fe(II)/Fe(III)-Salzlösung in Kontakt gebracht, so dass die Salze in die Partikel eindiffundieren. Damit die Salze in der Matrix adsorbieren können, werden die Polymere zudem mit zusätzlichen Nitro-, Nitroso- oder Aminogruppen funktionalisiert. Durch Zugabe von Ammoniak werden die Salze ausgefällt, so dass superparamagnetische Eisenoxidpartikel innerhalb der Polymerteilchen gebildet werden.

Die Druckschrift WO 97/04862 A1 betrifft den Einbau von magnetischen Kolloiden in kugelförmige Beads auf der Basis von Polyvinylalkoholen (PVA) als Matrixmaterial. Dabei werden die superparamagnetischen Partikel in einer wässrigen Suspension zusammen mit dem Polymer vorgelegt und mit Emulgatoren in eine apolare Phase eingebracht. Nach Ausbildung der Emulsion wird durch Zugabe entsprechender Reagenzien eine Quervernetzung der Polymerketten durchgeführt, so dass Partikel mit einem magnetischen Kern und einer Polymerhülle entstehen. Ein vergleichbarer Syntheseweg wird auch in US 4,267,234 beschrieben, worin mit Hilfe einer Suspensionspolymerisation magnetische

Beads auf Basis von Polyglutaraldehyd bereitgestellt werden.

EP 1 404 442 offenbart ebenfalls eine Synthese zur Herstellung magnetischer Polymerpartikel mit Hilfe einer wässrigen Emulsion. Dabei kann es sich bei den eingesetzten Monomeren sowohl um Metall enthaltende als auch organische Monomere handeln, welche in der wässrigen Phase löslich sind. Die Monomere werden mit den eingesetzten, ebenfalls wasserlöslichen, magnetischen Partikeln in einer Ölphase emulgiert, so dass die Partikel nach dem Polymerisationsschritt im Wesentlichen gleichmäßig in den magnetischen Beads verteilt sind.

Die Technologie der US 5,648,124 A nutzt eine andere Art des Partikelaufbaus. Die superparamagnetischen Nanoartikel werden aufgrund elektrostatischer Wechselwirkungen auf einen, bevorzugt aus einem Polymer bestehenden, Kern abgeschieden und anschließend mit einer weiteren Polymerschicht eingeschlossen.

In der Druckschrift US 7,989,614 B2 werden magnetische Beads für die Nukleinsäureaufreinigung beschrieben, die einen Kern besitzen, der mit einer Polyurethanbeschichtung versehen ist.

Gemäß Druckschrift US 8,945,509 B2 werden magnetische Beads beschrieben, auf deren Oberfläche Glycokonjugate angebunden sind. Zur Anbindung der Glycokonjugate an die hydrophoben Partikelkerne werden hydrophile Copolymerketten bestehend aus funktionalisierten Acrylaten, Polyalkylenglycolen, Copolymerketten auf Basis von Methylvinylether und Maleinsäure, oder auch Polyurethanpolyether-Copolymerketten eingesetzt. Die mit den Glycokonjugaten funktionalisierten Copolymerketten werden dazu an die Partikelkerne angebunden.

In den Druckschriften WO 2005/015216 A1 und EP 2 051 075 A1 wird die Beschichtung von Polymerpartikeln mit Epoxiden beschrieben. Die Polymerpartikel enthalten eingelagerte superparamagnetische Kristalle und besitzen funktionelle Gruppen auf der Oberfläche.

Gemäß Druckschrift WO 20061075185 A1 werden porige, oberflächenfunktionalisierte, superparamagnetische Kristalle enthaltende Polymerpartikel zur Verringerung der Auslaugung durch weitere Umsetzung mit einem Polyisocyanat und mindestens einem Diol mit einer Beschichtung versehen.

Es hat sich herausgestellt, dass die eingelagerten magnetischen Partikel der magnetischen Beads dennoch oftmals während der Anwendung aus der Trägermatrix ausgelaugt werden. Mit einer verringerten Anzahl magnetischer Nanopartikel in der Trägermatrix sind die magnetischen Beads in der Magnetseparation weniger effektiv.

Zudem ist die Herstellung magnetischer Beads mit Eignung für die Nukleinsäureaufreinigung umständlich und sollte zur Steigerung der Wirtschaftlichkeit der Magnetseparation vereinfacht werden.

Der vorliegenden Erfindung lag die Aufgabe zugrunde, magnetische Beads für die Nukleinsäureaufreinigung bereitzustellen, die eine verbesserte Handhabbarkeit und eine verbesserte Leistung in der Magnetseparation aufweisen. Die mit Nukleinsäuren beladenen, separierten magnetischen Beads sollen zudem direkt für den Einsatz in Folgeanwendungen, insbesondere in der Polymerasekettenreaktion (PCR), geeignet sein.

Zudem sollen die magnetischen Beads durch ein einfaches Herstellverfahren erhältlich und dadurch wirtschaftlich sein. Das Herstellverfahren soll einen besseren Einbau der magnetischen Partikel in die Trägermatrix der magnetischen Beads erlauben. Die magnetischen Partikel sollen in größerer Menge in der Trägermatrix vorhanden sein und weniger aus der Trägermatrix auslaugen.

Die Aufgabe wurde gelöst durch ein partikelförmiges Feststoff-Kompositmaterial zur Nukleinsäureaufreinigung, enthaltend magnetische Nanopartikel eingebettet in eine Trägermatrix auf Basis mindestens eines Polymers, das durch Polyaddition von

a) mindestens einem Isocyanat-reaktiven-Monomer A1, ausgewählt aus Verbindungen, enthaltend

- mindestens zwei funktionelle Gruppen, die jeweils mindestens ein Zerewitinoffreaktives Wasserstoffatom, und
- zusätzlich zu diesen mindestens zwei funktionellen Gruppen mindestens einen anionischen oder potentiell anionischen Rest (bevorzugt ausgewählt aus Carboxylatgruppe, Sulfonatgruppe, oder Kombinationen daraus), tragen

mit

b) mindestens einem Polyisocyanat-Monomer B

erhalten,
mit der Maßgabe, dass besagte Polyaddition in Gegenwart magnetischer Nanopartikel erfolgt.

Im Rahmen der vorliegenden Erfindung werden die Begriffe "magnetische Beads" und "partikelförmiges Feststoff-Kompositmaterial" synonym verwendet.

Die Verwendung unbestimmter Artikel (eine, ein) oder bestimmter Artikel (der, die, das) ist - falls nicht im Einzelfall anderweitig gekennzeichnet - im Sinne der vorliegenden Anmeldung nicht als beschränkende Mengenangabe aufzufassen. Gemeint ist - sofern nicht im Einzelfall anderweitig gekennzeichnet - eine Anzahl von mindestens eins (i.e. ein oder mehrere).

Ein "Nanopartikel" weist erfindungsgemäß eine mittlere Partikelgröße (Volumenmittel) von kleiner 0,3 $\mu$m auf.

Partikelgrößen festförmiger Partikel wurden mit einem Beckman Multisizer™ 3 Coulter Counter® (Software Beckman Coulter Multisizer™ 3, ©1990-2008, Version 3.53, 15.10.2008, Beckman Coulter GmbH, Krefeld) bestimmt und ausgewertet.

Als "magnetisch" wird ein Stoff definiert, der magnetisierbar ist. Ein magnetischer Stoff soll unter Einfluss eines äußeren Magnetfeldes magnetisch angezogen werden, jedoch bei Wegnahme dieses Feldes kein magnetisches Moment bzw. kaum noch ein magnetisches Moment haben, i.e. der Stoff sollte vorzugsweise eine Remanenz von nahezu Null oder von Null aufweisen, damit Partikel des Stoffes ohne Gegenwart eines äußeren magnetischen Feldes sich nicht gegenseitig anziehen und dadurch agglomerieren. Beispiele magnetischer Stoffe sind ferrimagnetische Stoffe mit einer niedrigen Curie-Temperatur oder superparamagnetische Stoffe, wie z.B. in EP 0 106 873 beschrieben.

Eine "Trägermatrix" ist definiert als festförmige Materie, in welche Partikel eines festförmigen Stoffes, wie im vorliegenden Fall magnetische Nanopartikel, eingebettet sind. Die Trägermatrix umgibt dabei besagte Partikel, wobei nicht ausgeschlossen ist, dass zusätzlich besagte Partikel auch auf der Oberfläche der Trägermatrix lokalisiert sind. Trägermatrix, magnetische Nanopartikel sowie gegebenenfalls weitere Bestandteile bilden das erfindungsgemäße partikelförmige Feststoff-Kompositmaterial.

"Auf Basis" bedeutet im Rahmen der vorliegenden Erfindung, dass das die Trägermatrix konstituierende Material mehr als 50 Gew.-%, insbesondere mehr als 60 Gew.-%, besonders bevorzugt mehr als 70 Gew.-%, ganz besonders bevorzugt mehr als 80 Gew.-%, am bevorzugtesten mehr als 90 Gew.-%, besagtes Polymer enthält.

Ein "Zerewitinoff-reaktives Wasserstoffatom" einer Funktionalität wird im Rahmen der vorliegenden Erfindung als ein azides Wasserstoffatom oder "aktives" Wasserstoffatom definiert. Ein solches kann in an sich bekannter Weise durch eine Reaktivität mit einem entsprechenden Grignard-Reagenz ermittelt werden. Die Menge an Zerewitinoff-aktiven Wasserstoffatomen wird typischerweise über die Methanfreisetzung gemessen, die gemäß der folgenden Reaktionsgleichung bei einer Reaktion der zu überprüfenden Substanz R-XH (die Funktionalität -XH bindet an den Rest des Moleküls R) mit Methylmagnesiumbromid ($CH_3$-MgBr) erfolgt:

$$CH_3\text{-}MgBr + R\text{-}XH \rightarrow CH_4 + Mg\,(XR)Br$$

Zerewitinoff-aktive Wasserstoffatome stammen insbesondere von C-H aziden organischen Gruppen, -OH, -SH, $-NH_2$ oder-NHR' mit R' als organischem Rest.

Ein "anionischer Rest" ist erfindungsgemäß eine Funktionalität, die definitionsgemäß von Funktionalitäten mit Zerewitinoff-aktivem Wasserstoffatom verschieden ist und mindestens eine anionische Ladung trägt, die über mindestens zwei Sauerstoffatome delokalisiert vorliegt. Ein "potentiell anionischer Rest" ist dementsprechend eine Funktionalität, die bei einer pH-Wert-Änderung einen anionischen Rest bildet, insbesondere bei einer pH-Wert-Erhöhung.

Eine chemische Verbindung gilt als "organisch", wenn sie mindestens ein Kohlenstoffatom und ein daran kovalent gebundenes Wasserstoffatom enthält.

Ein besonders bevorzugter Aufbau des partikelförmigen Feststoff-Kompositmaterials weist besagte Trägermatrix auf, in die zumindest magnetische Nanopartikel eingebettet sind, wobei das die Trägermatrix konstituierende Material mehr als 50 Gew.-%, insbesondere mehr als 60 Gew.-%, besonders bevorzugt mehr als 70 Gew.-%, ganz besonders bevorzugt mehr als 80 Gew.-%, am bevorzugtesten mehr als 90 Gew.-%, besagtes Polymer enthält.

Zur Bereitstellung besonders geeigneter Kompositmaterialien sind nachfolgend aufgeführte, bevorzugte Ausführungsformen des erfindungsgemäßen Kompositmaterials unabhängig von dem zuvor gekennzeichneten bevorzugten Aufbau des partikelförmigen Feststoff-Kompositmaterials zu berücksichtigen. Allerdings ist es wiederum bevorzugt, mindestens eine der nachfolgenden bevorzugten Ausführungsformen des Kompositmaterials mit dem zuvor genannten bevorzugten oder besonders bevorzugten Aufbau des partikelförmigen Feststoff-Kompositmaterials zu kombinieren.

Die Partikel des erfindungsgemäßen partikelförmigen Feststoff-Kompositmaterials weisen bevorzugt eine mittlere Partikelgröße (Volumenmittel) von 0,5 $\mu$m bis 50 $\mu$m, insbesondere von 0,6 $\mu$m bis 30 $\mu$m, ganz besonders bevorzugt von 0,6 $\mu$m bis 10 $\mu$m, auf.

Als bevorzugte Obergrenze weisen die eingebetteten magnetischen Nanopartikel eine mittlere Partikelgröße (Volumenmittel) von höchstens 200 nm, bevorzugt von höchstens 100 nm, besonders bevorzugt von höchstens 50 nm, auf.

Als bevorzugte Untergrenze weisen die magnetischen Nanopartikel eine mittlere Partikelgröße (Volumenmittel) von mindestens 1 nm, insbesondere von mindestens 5 nm, auf. Besonders bevorzugte magnetische Nanopartikel besitzen eine mittlere Partikelgröße (Volumenmittel) im Bereich einer Kombination von zuvor definierten Ober- und Untergrenzen.

Die enthaltenen magnetischen Nanopartikel werden bevorzugt ausgewählt aus ferromagnetischen Nanopartikeln, ferrimagnetischen Nanopartikeln, superparamagnetischen Nanopartikeln oder deren Mischung, bevorzugt aus superparamagnetischen Nanopartikeln. Die Synthese superparamagnetischer Partikel kann gemäß gängiger Methoden erfolgen, wie beispielsweise in J.-C. Bacri, R. Perzynski, D. Salin, V. Cabuil, R. Massart, Ionic Ferrofluids: A crossing of chemistry and physics, J. Magn. Magn. Mater., 1990, 85, 27 - 32 beschrieben.

Bevorzugt eignen sich Metalloxide als magnetische Nanopartikel, besonders bevorzugt Eisenoxide, wie $Fe_3O_4$oder $Fe_2O_3$. Dabei ist es möglich, dass die zweiwertigen Eisenatome teilweise oder vollständig durch ein von Eisen verschiedenes zweiwertiges Metall, wie insbesondere Chrom, Cobalt, Kupfer, Magnesium, Mangan, Nickel, Vanadium und/oder Zink, ausgetauscht sind. Die magnetischen Nanopartikel umfassen besonders bevorzugt Eisenoxid, insbesondere Magnetit, Maghemit oder deren Mischung. Solche Partikel sind z.B. unter den Namen Bayoxide® kommerziell erhältlich oder lassen sich wie in der Beispielsektion beschrieben (vide infra) herstellen.

Die magnetischen Nanopartikel sind bezogen auf das Gesamtgewicht des erfindungsgemäßen Kompositmaterials bevorzugt in einer Menge von mindestens 40 Gew.-%, besonders bevorzugt in einer Menge von mindestens 50 Gew.-%, ganz besonders bevorzugt in einer Menge von mindestens 60 Gew.-%, am bevorzugtesten von mindestens 75 Gew.-%, in dem besagten Kompositmaterial enthalten.

Die magnetischen Nanopartikel können an der Oberfläche mit organischen Verbindungen modifiziert sein, die ein zahlenmittleres Molekulargewicht Mn von kleiner 1000 g/mol besitzen und mindestens einen anionischen oder potentiell anionischen Rest tragen. Zur Verwirklichung dieser Ausführungsform können bevorzugt Hydroxycarbonsäuren, besonders bevorzugt Monohydroxy-($C_3$-$C_{10}$)-carbonsäuren, insbesondere Zitronensäure, 2-Hydroxybernsteinsäure, Glycolsäure, 2-Hydroxypropansäure, 3-Hydroxypropansäure, 2-Hydroxy-2-Methylpropansäure, 2-Hydroxybutansäure, 3-Hydroxybutansäure, 4-Hydroxybutansäure, 2-Hydroxybutandisäure 2-Hydroxypentansäure, 3-Hydroxypentansäure, 4-Hydroxypentansäure, 5-Hydroxypentansäure, 2-Hydroxypentandisäure, 3-Hydroxypentandisäure, 3-Carboxy-3-hydroxypentandisäure, 2-Hydroxyhexansäure, 3-Hydroxyhexansäure, 4-Hydroxyhexansäure, 5-Hydroxyhexansäure, 6-Hydroxyhexansäure, 2-Hydroxy-1,6-hexandisäure, 3-Hydroxy-1,6-hexandisäure, 3-Carboxy-2-Hydroxy-1,6-hexandisäure, 3-Carboxy-3-Hydroxy-1,6-hexandisäure, 3-Carboxy-3-Hydroxy-1,6-hexandisäure, 2-Carboxy-4-Hydroxy-1,6-hexandisäure oder eine Kombination daraus, auf der Oberfläche des magnetischen Nanopartikels angelagert sein. Ein oberflächenmodifizierter magnetischer Nanopartikel ist ebenso ein Nanopartikel im Sinne der vorab gegebenen Definition.

Als für die Polyaddition eingesetztes Isocyanat-reaktives-Monomer A1 eignet sich insbesondere mindestens eine Verbindung enthaltend

- mindestens zwei funktionelle Gruppen, die jeweils mindestens ein Zerewitinoff-reaktives Wasserstoffatom tragen, unabhängig voneinander ausgewählt aus Hydroxylgruppe, primärer Aminogruppe, sekundärer Aminogruppe, Thiolgruppe, Ketimingruppe, Ketazingruppe, Oxazolidingruppe und
- zusätzlich zu diesen mindestens zwei funktionellen Gruppen mindestens einen anionischen oder potentiell anionischen Rest (bevorzugt ausgewählt aus Carboxylatgruppe, Sulfonatgruppe, oder Kombinationen daraus).

Dabei ist es wiederum bevorzugt, wenn die mindestens zwei funktionellen Gruppen, die jeweils mindestens ein Zerewitinoff-reaktives Wasserstöffatom tragen, unabhängig voneinander ausgewählt werden aus Hydroxylgruppe, primärer Aminogruppe, sekundärer Aminogruppe.

Bevorzugt werden als Isocyanat-reaktives-Monomer A1 Diole oder Polyole mit mindestens einem anionischen oder potentiell anionischen Rest (bevorzugt ausgewählt aus Carboxylatgruppe; Sulfonatgruppe, oder Kombinationen daraus) verwendet. Besonders bevorzugte Diole sind solche der allgemeinen Formel HO-R-OH mit R = aliphatischer, cycloaliphatischer, aromatischer oder araliphatischer Rest, an den jeweils mindestens ein anionischer oder potentiell anionischer Rest bindet.

Wie in dieser Anmeldung verwendet, soll der Begriff "aliphatisch" für gegebenenfalls substituierte, lineare oder verzweigte, Alkyl-, Alkenyl- und Alkinylgruppen stehen, in denen nicht benachbarte Methylengruppen ($-CH_2-$) durch Heteroatome, wie insbesondere Sauerstoff und Schwefel, oder durch sekundäre Aminogruppen ersetzt sein können.

Wie in dieser Anmeldung verwendet, soll der Begriff "cycloaliphatisch" für, gegebenenfalls substitutierte, carbozyklische oder heterozyklische Verbindungen stehen, die nicht zu den aromatischen Verbindungen gehören.

Als Isocyanat-reaktives-Monomer A1 wird erfindungsgemäß bevorzugt mindestens ein Diol ausgewählt, welches sich strukturell von Propan-1,2-diol, Propan-1,3-diol, 2,2-Bis(hydroxymethyl)propan, Butan-1,3-diol, Butan-1,4-diol, Pentan-1,5-diol, Hexan-1,6-diol, Octan-1,8-diol, Dodecan-1,12-diol, Neopentylglycol, 1,4-Bis-(hydroxymethyl)cyclohexan, 1,3-Bis-(hydroxymethyl)cyclohexan, 1,2-Bis-(hydroxymethyl)cyclohexan, 2-Methylpropan-1,3-diol oder 2-Methylpentan-2,4-diol, 2-Ethyl-2-butylpropandiol, Trimethylpentandiol, 2,2,4-Trimethyl-1,3-pentandiol, Cyclohexan-1,2-diol, Cyclohexan-1,4-diol, 2,2-Bis(4-hydroxycyclohexyl)propan, Resorcin, 1,2-Dihydroxybenzol ableitet, mit der Maßgabe, dass jeweils zusätzlich mindestens ein anionischer oder potentiell anionischer Rest (bevorzugt ausgewählt aus Carboxylatgruppe, Sulfonatgruppe, oder Kombinationen daraus) im Molekül vorhanden ist.

Ein erfindungsgemäß besonders bevorzugtes Isocyanat-reaktives-Monomer A1 wird ausgewählt aus 2,2-Bis(hydroxymethyl)propionsäure, 2,3-Diaminobenzoesäure, 2,4-Diaminobenzoesäure, 2,5-Diaminobenzoesäure, 2,6-Diaminobenzoesäure, 3,4-Diaminobenzoesäure, 3,5-Diaminobenzoesäure, 2,2-Di(hydroxymethyl)essigsäure, 2,2,2-Tri(hydroxymethyl)essigsäure, 2,2-Di(hydroxymethyl)propansäure , 2,2-Di(hydroxymethyl)butansäure, 2,2-Di(hydroxymethyl)pentansäure, 2,5-Dihydroxy-3-methylpentansäure, 3,5-Dihydroxy-3-methylpentansäure, 4,5-Dihydroxy-3-methylpentansäure, 3,4-Dihydroxy-3-methylpentansäure, 2,3-Dihydroxy-3-methylpentansäure, 2,4-Dihydroxy-3-methylpentansäure, 2,3-Dihydroxybenzoesäure, 2,4-Dihydroxybenzoesäure, 2,5-Dihydroxybenzoesäure, 2,6-Dihydroxybenzoesäure, 3,4-Dihydroxybenzoesäure, 3,5-Dihydroxybenzoesäure, 2,3-Dihydroxybernsteinsäure, 2,5-Diaminopentansäure, 3,5-Diaminopentansäure, 4,5-Diaminopentansäure, 2,3-Dihydroxybenzolsulfonsäure, 3,4-Dihydroxybenzolsulfonsäure, 2,4-Dihydroxybenzolsulfonsäure, 2,5-Dihydroxybenzolsulfonsäure, 3,5-Dihydroxybenzolsulfonsäure, 2,3-Diaminobenzolsulfonsäure, 3,4-Diaminobenzolsulfonsäure, 2,4-Diaminobenzolsulfonsäure, 2,5-Diaminobenzolsulfonsäure, 3,5-Diaminobenzolsulfonsäure, 3,4-Dihydroxy-2-Toluolsulfonsäure, 3,4-Diamino-2-Toluolsulfonsäure, 4,5-Dihydroxy-2-Toluolsulfonsäure, 4,5-Diamino-2-Toluolsulfonsäure, 5,6-Dihydroxy-2-Toluolsulfonsäure, 5,6-Diamino-2-Toluolsulfonsäure, 3,5-Dihydroxy-2-Toluolsulfonsäure, 3,5-Diamino-2-Toluolsulfonsäure, 3,6-Dihydroxy-2-Toluolsulfonsäure, 3,6-Diamino-2-Toluolsulfonsäure, 4,6-Dihydroxy-2-Toluolsulfonsäure, 4,6-Diamino-2-Toluolsulfonsäure, 2,4-Dihydroxy-3-Toluolsulfonsäure, 2,4-Diamino-3-Toluolsulfonsäure, 2,5-Dihydroxy-3-Toluolsulfonsäure, 2,5-Diamino-3-Toluolsulfonsäure, 2,6-Dihydroxy-3-Toluolsulfonsäure, 2,6-Diamino-3-Toluolsulfonsäure, 4,5-Dihydroxy-3-Toluolsulfonsäure, 4,5-Diamino-3-Toluolsulonsäure, 4,6-Dihydroxy-3-Toluolsulfonsäure, 4,6-Diamino-3-Toluolsulfonsäure, 5,6-Dihydroxy-3-Toluolsulfonsäure, 5,6-Diamino-3-Toluolsulfonsäure, 2,3-Dihydroxy-4-Toluolsulfonsäure, 2,3-Diamino-4-Toluolsulfonsäure, 2,5-Dihydroxy-4-Toluolsulfonsäure, 2,5-Diamino-4-Toluolsulfonsäure, 2,6-Dihydroxy-4-Toluolsulfonsäure, 2,6-Diamino-4-Toluolsulfonsäure, 3,5-Dihydroxy-4-Toluolsulfonsäure, 3,5-Diamino-4-Toluolsulfonsäure, 3,6-Dihydroxy-4-Toluolsulfonsäure, 3,6-Diamino-4-Toluolsulfonsäure, 5,6-Dihydroxy-4-Toluolsulfonsäure, 5,6-Diamino-4-Toluolsulfonsäure oder Mischungen aus mindestens zwei dieser Verbindungen.

Es ist erfindungsgemäß bevorzugt, wenn neben dem Isocyanat-reaktiven Monomer A1 zusätzlich mindestens ein Isocyanat-reaktives Monomer A2 für die Polyaddition zur Herstellung des besagten Polymers der Trägermatrix eingesetzt wird, das ausgewählt wird aus nichtionischen Verbindungen enthaltend mindestens zwei funktionelle Gruppen, die jeweils mindestens ein Zerewitinoff-reaktives Wasserstoffatom tragen.

Besonders bevorzugt wird mindestens ein Isocyanat-reaktives Monomer A2 ausgewählt aus organischem Polyol, organischem Polyetherpolyol, organischem Polyesterpolyol, Polycarbonatpolyol, organischem Polyetherestercarbonatpolyol, organischem Aminoalkohol, organischem Polyamin, organischem Polyalkylenamin, oder Kombinationen daraus, ganz besonders bevorzugt ausgewählt aus organischem Diol, organischem Diamin, organischem Polyalkylenglykol, organischem Polyetherdiol, organischem Polyesterdiol, Polycarbonatdiol, Polyetherestercarbonatdiol oder Kombinationen daraus.

Bevorzugte organische Diole werden ausgewählt aus organischen Polyetherdiolen, $C_2$-$C_6$-Alkandiol (Ethylenglycol, Propan-1,2-diol, Propan-1,3-diol, Butan-1,3-diol, Butan-1,4-diol, Pentan-1,5-diol, Hexan-1,6-diol), Octan-1,8-diol, Dodecan-1,12-diol, Neopentylglycol, 1,4-Bis-(hydroxymethyl)cyclohexan, 1,3-Bis-(hydroxymethyl)cyclohexan, 1,2-Bis-(hydroxymethyl)cyclohexan, 2-Methylpropan-1,3-diol, 2-Methylpentan-2,4-diol, 2-Ethyl-2-butylpropandiol, Trimethylpentandiol, 2,2,4-Trimethyl-1,3-pentandiol, Cyclohexan-1,2-diol, Cyclohexan-1,4-diol, 2,2-Bis(4-hydroxycyclohexyl)propan, 1,3-Dihydroxyaceton, Dihydroxyacetondimer oder Kombinationen mindestens zweier vorgenannter Verbindungen.

Bevorzugte Polyetherdiole werden wiederum ausgewählt aus Diethylenglycol, Triethylenglycol, Tetraethylenglycol, Polyethylenglycol, Dipropylenglycol, Tripropylenglycol, Tetrapropylenglycol, Polypropylenglycol, Dibutylenglycol, Tributylenglycol, Tetrabutylenglycol, Polybutylenglycol oder Kombinationen mindestens zweier vorgenannter Verbindungen.

Bevorzugt geeignete Polyesterpolyole werden ausgewählt aus Poly[di(ethylenglycol)adipat], Polycaprolactondiol, Polyethylenterephthalat, Polypropylenterephthalat, Polybutylenterephthalat, Polyethylenisophthalat, Polypropylenisophthalat, Polybutylenisophthalat, Polyethylen-1,4-cyclohexylendimethylenterephthalat, Polytetramethylenetherglycolterephthalat oder Kombinationen mindestens zweier vorgenannter Verbindungen.

Beispiele geeigneter organischer Polyamine als Monomere A2 sind Ethylendiamin, 1,3-Diaminopropan, 1,4-Diaminobutan, Pentamethylendiamin, Hexamethylendiamin Diethylentriamin, Dipropylentriamin, Isophorondiamin, 1,4-Cyclohexyldiamin, Piperazin, oder Polyethylenimin. Die besagten Aminverbindungen können auch in blockierter Form, insbesondere als Ketimine, Ketazine, Oxazolidine oder als Ammoniumsalze eingesetzt werden.

Es stellte sich als besonders geeignet heraus, wenn das Isocyanat-reaktive Monomer A2 ein zahlenmittleres Molekulargewicht Mn von 200 g/mol bis 10000 g/mol (bevorzugt von 400 g/mol bis 2500 g/mol) aufweist.

Im Rahmen der vorliegenden Erfindung wird, falls nicht anders angegeben, das zahlenmittlere Molekulargewicht Mn durch Gelpermeationschromatographie (GPC) in Tetrahydrofuran bei 23°C bestimmt. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2×PSS SDV linear M, 8×300 mm, 5 μm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der am Anmeldetag dieser Patentanmeldung gültigen DIN 55672-1:2016-03 festgelegt.

Besonders bevorzugt wird Monomer A2 ausgewählt aus organischen Polyetherpolyolen, organischen Polyesterpolyolen, organischen Polycarbonatpolyolen, organischen Polyetherestercarbonatpolyolen, Polyhydroxyolefinen,

oder Kombinationen aus mindestens zwei Verbindungen davon.

Ganz besonders bevorzugt weist das Isocyanat-reaktive Monomer A2 ein zahlenmittleres Molekulargewicht Mn von 200 g/mol bis 10000 g/mol (bevorzugt von 400 g/mol bis 2500 g/mol) auf und ist ausgewählt aus organischen Polyalkylenglykolen, organischen Polyetherdiolen, organischen Polyesterdiolen, oder Kombinationen daraus.

Die erfindungsgemäßen Kompositpartikel, die aus besagten Isocyanat-reaktiven Monomeren A2 mit o.g. Molekulargewicht hergestellt werden, weisen eine gute Elastizität und eine besonders gute Einbettungseffizienz der magnetischen Nanopartikel auf.

Es ist erfindungsgemäß bevorzugt, wenn als Isocyanat-reaktives Monomer A2 für die Polyaddition zur Herstellung des besagten Polymers der Trägermatrix

- mindestens ein erstes Isocyanat-reaktives Monomer A2a mit zwei funktionellen Gruppen, die jeweils mindestens ein Zerewitinoff-reaktives Wasserstoffatom tragen, unabhängig voneinander ausgewählt aus Hydroxylgruppen, Aminogruppen, Thiolgruppen, Ketimingruppen, Ketazingruppen, Oxazolidingruppen oder Kombinationen daraus und
- mindestens ein zweites Isocyanat-reaktives Monomer A2b mit mehr als zwei funktionellen Gruppen (bevorzugt drei funktionellen Gruppen), die jeweils mindestens ein Zerewitinoff-reaktives Wasserstoffatom trägen, unabhängig voneinander ausgewählt aus Hydroxylgruppen, Aminogruppen, Thiolgruppen, Ketimingruppen, Ketazingruppen, Oxazolidingruppen oder Kombinationen daraus,
  eingesetzt werden.

Dabei wird wiederum das Isocyanat-reaktive Monomer A2a gemäß obiger Maßgabe bevorzugt ausgewählt aus organischen Polyetherpolyolen, organischen Polyesterpolyolen, organischen Polycarbonatpolyolen, organischen Polyetherestercarbonatpolyolen, Polyhydroxyolefinen, oder Kombinationen aus mindestens zwei Verbindungen davon.

Dabei sind gemäß obiger Maßgabe die für das Monomer A2 zuvor genannten organischen Polyetherpolyole, organischen Polyesterpolyole, organischen Polycarbonatpolyole, organischen Polyetherestercarbonatpolyole, Polyhydroxyolefine ebenso bevorzugt.

Ganz besonders bevorzugt weist das Isocyanat-reaktive Monomer A2a ein zahlenmittleres Molekulargewicht Mn von 200 g/mol bis 10000 g/mol (bevorzugt von 400 g/mol bis 2500 g/mol) auf und ist ausgewählt aus organischen Polyalkylenglykolen, organischen Polyetherdiolen, organischen Polyesterdiolen, oder Kombinationen daraus.

Als bevorzugtes Isocyanat-reaktives Monomer A2 oder A2b wird mindestens ein Alkohol mit drei Hydroxylgruppen eingesetzt, insbesondere 1,3,5-Trihydroxybenzol, 1,2,3-Trihydroxybenzol, 1,2,4-Trihydroxybenzol, 1,2,5-Trihydroxybenzol, 1,3,4-Trihydroxybenzol, Propan-1,2,3-triol, Propan-1,1,1-triol, Propan-1,1,2-triol, Propan-1,1,3-triol, Propan-1,2,2-triol, Ethan-1,1,2-triol, Butan-1,2,3-triol, Butan-1,2,4-triol, Pentan-1,2,3-triol, Pentan-1,2,4-triol, Pentan-1,2,5-triol, Pentan-1,3,5-triol, Pentan-2,3,4-triol, 2-Methylbutan-1,2,4-triol, 2-Methylbutan-1,2,3-triol, 2-Methylbutan-1,1,4-triol, Hexan-1,2,3-triol, Hexan-1,2,4-triol, Hexan-1,2,5-triol, Hexan-1,2,6-triol, Hexan-1,3,4-triol, Hexan-1,3,5-triol, Hexan-1,3,6-triol, Hexan-1,1,6-triol, Cyclohexan-1,2,3-triol, Cyclohexan-1,2,4-triol, Cyclohexan-1,2,5-triol, Cyclohexan-1,1,2-triol, Cyclohexan-1,1,3-triol, Cyclohexan-1,1,4-triol, Cyclohexan-1,3,5-triol, oder Kombinationen daraus. Ganz besonders bevorzugt wird das Monomer A2b ausgewählt aus insbesondere 1,3,5-Trihydroxybenzol, Propan-1,2,3-triol oder Kombinationen daraus

Als geeignetes Polyisocyanat-Monomer B finden aromatische, araliphatische, aliphatische oder cycloaliphatische Polyisocyanate jeweils mit einer NCO-Funktionalität $\geq 2$ Verwendung. Die NCO-Funktionalität kann im Polyisocyanat-Monomer B auch als sogenannte verkappte NCO-Funktionalität vorhanden sein. Dies sind z.B. Harnstoffe, Biurete, Allophanate, Uretdione, Isocyanurate, Carbodiimide.

Unter "araliphatisch" werden solche Verbindungen verstanden, die sowohl einen Arylrest als auch einen an den Arylrest bindenden aliphatischen Molekülteil besitzen. Unter "araliphatischen Polyisocyanaten" sind solche Polyisocyanate zu verstehen, die ein araliphatisches Molekülfragment besitzen, wobei mindestens eine Isocyanatfunktionalität an den aliphatischen Teil des araliphatischen Molekülfragments bindet.

Als Polyisocyanat-Monomer B geeignete Diisocyanate sind beliebige durch Phosgenierung oder nach phosgenfreien Verfahren, beispielsweise durch thermische Urethanspaltung, zugängliche Diisocyanate, deren Isocyanatgruppen entweder über gegebenenfalls verzweigte aliphatische Reste oder an einem gegebenenfalls weiter substituierten Aromaten gebunden vorliegen. Vorzugsweise lassen sich Diisocyanate der allgemeinen Formel (I)

$$O=C=N-R-N=C=O \qquad (I)$$

verwenden, worin R für einen cycloaliphatischen ($C_{3-15}$)-Kohlenwasserstoffrest, aromatischen ($C_{6-15}$)-Kohlenwasserstoffrest, araliphatischen ($C_{6-18}$)-Kohlenwasserstoffrest oder aliphatischen ($C_{3-15}$)-Kohlenwasserstoffrest steht.

Ein bevorzugtes Polyisocyanat-Monomer wird ausgewählt aus 1,2-Ethandiisocyanat, 1,3-Propandiisocyanat, 1,4-Butandiisocyanat, 1,5-Pentandiisocyanat (PDI), 1,6-Hexandiisocyanat (Hexamethylendiisocyanat, HDI), 4-Isocyanatomethyl-1,8-octandiisocyanat (Triisocyanatononan, TIN), 4,4'-Methylenbis(cyclohexylisocyanat), 3,5,5-Trimethyl-1-isocyanato-3-isocyanatomethylcyclohexan (Isophorondiisocyanat, IPDI), 2,4- und/oder 2,6-Methylcyclohexyldiisocyanat ($H_6$TDI) sowie ω,ω'-Diisocyanato-1,3-dimethylcyclohexan ($H_6$XDI), 4,4'-Diisocyanatodicyclohexylmethan, Tetramethylendiisocyanat, 2-Methyl-pentamethylendiisocyanat, 2,2,4-Trimethylhexamethylendiisocyanat (THDI), Dodecamethylendiisocyanat, 1,4-Diisocyanatocyclohexan, 4,4'-Diisocyanato-3,3'-dimethyl-dicyclohexylmethan 2,2-Bis-(4-isocyanatocyclohexyl)-propan, 3-Isocyanatomethyl-1-methyl-1-isocyanatocyclohexan (MCI), 1,3-Diisooctylcyanato-4-methyl-cyclohexan, 1,3-Diisocyanato-2-methyl-cyclohexan, 2,4-Toluoldiisocyanat, 2,6-Toluoldiisocyanat, Methylendiphenyldiisocyanat (MDI), Naphtyldiisocyanat (NDI), 1,3-Bis(isocyanatomethyl)benzol (m-Xylylendiisocyanat, m-XDI), 1,4-Bis(isocyanatomethyl)benzol (p-Xylylendiisocyanat, p-XDI), 1,3-Bis(2-isocyanatopropan-2-yl)benzol (m-Tetramethylxylylendiisocyanat, m-TMXDI), 1,4-Bis(2-isocyanatopropan-2-yl)benzol (p-Tetramethylxylylendiisocyanat, p-TMXDI), sowie beliebige Mischungen mindestens zweier dieser Diisocyanate.

Ebenso eignen sich Dimere aus den vorgenannten Diisocyanaten, Trimere aus den vorgenannten Diisocyanaten oder Kombinationen daraus.

Besonders bevorzugt wird als Polyisocyanat-Monomer B mindestens ein zyklisches Polyisocyanat, Insbesondere mindestens eine Verbindung der Formel (I), wobei R für cycloaliphatischen ($C_{3-15}$)-Kohlenwasserstoffrest, aromatischen ($C_{6-15}$)-Kohlenwasserstoffrest, araliphatischen ($C_{6-18}$)-Kohlenwasserstoffrest steht. Der Einsatz zyklischer Polyisocyanate verringert die Anlagerung des erfindungsgemäßen Kompositmaterials an Oberflächen, wie z.B. Gefäßwänden. Partikel eines aus zyklischem Polyisocyanat erhältlichen Kompositmaterials lassen sich gut in einem flüssigen Medium suspendieren und sind während der Synthese und in der Anwendung, wie z.B. in Aufreinigungsverfahren für Nukleinsäuren, einfach zu handhaben.

Besonders bevorzugt wird als Polyisocyanat-Monomer B mindestens ein Polyisocyanat eingesetzt, ausgewählt aus Toluol-2,4-diisocyanat, Toluol-2,6-diisocyanat, Isophorondiisocyanat, sowie beliebigen Mischungen mindestens zweier dieser Diisocyanate.

Im Rahmen einer ganz besonders bevorzugten Ausführungsform wird das besagte Polymer durch Polyaddition von mindestens einer der folgenden Monomerkombinationen erhalten, wobei die jeweilig in der Zeile der Tabelle 1 aufgeführte Monomerkombination zumindest die Monomere A1, A2a, A2b und B umfasst.

Tabelle 1:

| A1 | A2a | A2b | B |
|---|---|---|---|
| 2,2-Bis (hydroxymethyl) propionsäure | Polyalkylenglykol mit zahlenmittlerem Molekulargewicht Mn von 200 g/mol bis 10000 g/mol (bevorzugt von 400 g/mol bis 2500 g/mol) | 1,3,5-Trihydroxybenzol | Toluol-2,4-diisocyanat |
| 2,2-Bis (hydroxymethyl) propionsäure | Polyalkylenglykol mit zahlenmittlerem Molekulargewicht Mn von 200 g/mol bis 10000 g/mol (bevorzugt von 400 g/mol bis 2500 g/mol) | Glyzerin | Toluol-2,4-diisocyanat |
| 2,2-Bis (hydroxymethyl) propionsäure | Polyalkylenglykol mit zahlenmittlerem Molekulargewicht Mn von 200 g/mol bis 10000 g/mol (bevorzugt von 400 g/mol bis 2500 g/mol) | 1,3,5-Trihydroxybenzol | Toluol-2,4-diisocyanat und Toluol-2,6-diisocyanat |
| 2,2-Bis (hydroxymethyl) propionsäure | Polyalkylenglykol mit zahlenmittlerem Molekulargewicht Mn von 200 g/mol bis 10000 g/mol (bevorzugt von 400 g/mol bis 2500 g/mol) | Glyzerin | Toluol-2,4-diisocyanat und Toluol-2,6-diisocyanat |
| 2,2-Bis (hydroxymethyl) propionsäure | Polyethylenglykol mit zahlenmittlerem Molekulargewicht Mn von 200 g/mol bis 10000 g/mol (bevorzugt von 400 g/mol bis 2500 g/mol) | 1,3,5-Trihydroxybenzol | Toluol-2,4-diisocyanat |
| 2,2-Bis (hydroxymethyl) propionsäure | Polyethylenglykol mit zahlenmittlerem Molekulargewicht Mn von 200 g/mol bis 10000 g/mol (bevorzugt von 400 g/mol bis 2500 g/mol) | Glyzerin | Toluol-2,4-diisocyanat |

(fortgesetzt)

| A1 | A2a | A2b | B |
|---|---|---|---|
| 2,2-Bis (hydroxymethyl) propionsäure | Polyethylenglykol mit zahlenmittlerem Molekulargewicht Mn von 200 g/mol bis 10000 g/mol (bevorzugt von 400 g/mol bis 2500 g/mol) | 1,3,5-Trihydroxybenzol | Toluol-2,4-diisocyanat und Toluol-2,6-diisocyanat |
| 2,2-Bis (hydroxymethyl) propionsäure | Polyethylenglykol mit zahlenmittlerem Molekulargewicht Mn von 200 g/mol bis 10000 g/mol (bevorzugt von 400 g/mol bis 2500 g/mol) | Glyzerin | Toluol-2,4-diisocyanat und Toluol-2,6-diisocyanat |
| 2,2-Bis (hydroxymethyl) propionsäure | Polyesterpolyol | 1,3,5-Trihydroxybenzol | Toluol-2,4-diisocyanat |
| 2,2-Bis (hydroxymethyl) propionsäure | Polyesterpolyol | Glyzerin | Toluol-2,4-diisocyanat |
| 2,2-Bis (hydroxymethyl) propionsäure | Polyesterpolyol | 1,3,5-Trihydroxybenzol | Toluol-2,4-diisocyanat und Toluol-2,6-diisocyanat |
| 2,2-Bis (hydroxymethyl) propionsäure | Polyesterpolyol | Glyzerin | Toluol-2,4-diisocyanat und Toluol-2,6-diisocyanat |
| 2,2-Bis (hydroxymethyl) propionsäure | Poly[di(ethylenglykol)adipat] | 1,3,5-Trihydroxybenzol | Toluol-2,4-diisocyanat |
| 2,2-Bis (hydroxymethyl) propionsäure | Poly[di(ethylenglykol)adipat] | Glyzerin | Toluol-2,4-diisocyanat |
| 2,2-Bis (hydroxymethyl) propionsäure | Poly[di(ethylenglykol)adipat] | 1,3,5-Trihydroxybenzol | Toluol-2,4-diisocyanat und Toluol-2,6-diisocyanat |
| 2,2-Bis (hydroxymethyl) propionsäure | Poly[di(ethylenglykol)adipat] | Glyzerin | Toluol-2,4-diisocyanat und Toluol-2,6-diisocyanat |
| 2,2-Bis (hydroxymethyl) propionsäure | ($C_2$-$C_6$)-Alkandiol | 1,3,5-Trihydroxybenzol | Toluol-2,4-diisocyanat |
| 2,2-Bis (hydroxymethyl) propionsäure | ($C_2$-$C_6$)-Alkandiol | Glyzerin | Toluol-2,4-diisocyanat |
| 2,2-Bis (hydroxymethyl) propionsäure | ($C_2$-$C_6$)-Alkandiol | 1,3,5-Trihydroxybenzol | Toluol-2,4-diisocyanat und Toluol-2,6-diisocyanat |
| 2,2-Bis (hydroxymethyl) propionsäure | ($C_{2-}C_6$)-Alkandiol | Glyzerin | Toluol-2,4-diisocyanat und Toluol-2,6-diisocyanat |
| 2,2-Bis (hydroxymethyl) propionsäure | Butan-1,4-diol | 1,3,5-Trihydroxybenzol | Toluol-2,4-diisocyanat |

(fortgesetzt)

| A1 | A2a | A2b | B |
|---|---|---|---|
| 2,2-Bis (hydroxymethyl) propionsäure | Butan-1,4-diol | Glyzerin | Toluol-2,4-diisocyanat |
| 2,2-Bis (hydroxymethyl) propionsäure | Butan-1,4-diol | 1,3,5-Trihydroxybenzol | Toluol-2,4-diisocyanat und Toluol-2,6-diisocyanat |
| 2,2-Bis (hydroxymethyl) propionsäure | Butan-1,4-diol | Glyzerin | Toluol-2,4-diisocyanat und Toluol-2,6-diisocyanat |
| 2,2-Bis (hydroxymethyl) propionsäure | 1,3-Dihydroxyaceton | 1,3,5-Trihydroxybenzol | Toluol-2,4-diisocyanat |
| 2,2-Bis (hydroxymethyl) propionsäure | 1,3-Dihydroxyaceton | Glyzerin | Toluol-2,4-diisocyanat |
| 2,2-Bis (hydroxymethyl) propionsäure | 1,3-Dihydroxyaceton | 1,3,5-Trihydroxybenzol | Toluol-2,4-diisocyanat und Toluol-2,6-diisocyanat |
| 2,2-Bis (hydroxymethyl) propionsäure | 1,3-Dihydroxyaceton | Glyzerin | Toluol-2,4-diisocyanat und Toluol-2,6-diisocyanat |
| 2,2-Bis (hydroxymethyl) propionsäure | Polyethylenglykol mit zahlenmittlerem Molekulargewicht Mn von 200 g/mol bis 10000 g/mol (bevorzugt von 400 g/mol bis 2500 g/mol) | Glyzerin | Isophorondiisoc yanat |
| 2,2-Bis (hydroxymethyl) propionsäure | 1,3-Dihydroxyaceton | Glyzerin | Isophorondiisoc yanat |

Das durch die Polyaddition erhaltene Polymer der Trägermatrix wird bevorzugt durch Emulsionspolymerisation, besonders bevorzugt durch Reaktion

i) mindestens eines Isocyanat-reaktiven-Monomers A, ausgewählt aus Verbindungen, enthaltend mindestens zwei funktionelle Gruppen, die jeweils mindestens ein Zerewitinoff-reaktives Wasserstoffatom tragen mit
ii) mindestens einem Polyisocyanat-Monomer B

mittels Emulsionspolymerisation einer Emulsion, enthaltend

i) in der diskontinuierlichen Phase magnetische Nanopartikel, mindestens eine polare, organische Flüssigkeit (bevorzugt mit einem cLogP-Wert < 1,5 (25°C)) und mindestens ein von der polaren, organischen Flüssigkeit verschiedenes Isocyanat-reaktives-Monomer A, ausgewählt aus Verbindungen, enthaltend mindestens zwei funktionelle Gruppen, die jeweils mindestens ein Zerewitinoff-reaktives Wasserstoffatom tragen, und
ii) in der kontinuierlichen Phase mindestens eine unpolare, organische Flüssigkeit (bevorzugt mit einem cLogP-Wert > 2 (25°C)) enthält,

in Gegenwart von Polyisocyanat-Monomer B erhalten wird, mit der Maßgabe, dass der cLogP-Wert (25°C) der polaren organischen Flüssigkeit kleiner als der cLogP-Wert (25°C) der unpolaren, organischen Flüssigkeit ist, erhalten.

Dabei sind wiederum die zuvor erwähnten bevorzugten Monomere, insbesondere in deren zuvor genannten bevorzugten Kombinationen, bevorzugt einsetzbar.

Ein Stoff ist eine "Flüssigkeit" bzw. "flüssig", wenn er zumindest bei 20°C und 1013 mbar im flüssigen Aggregatzustand vorliegt.

Zur Definition der Hydrophilie bzw. der Lipophilie von Substanzen wird oftmals der n-Oktanol-Wasser-Verteilungskoeffizient P der Substanz herangezogen, wobei üblicherweise der dekadische Logarithmus logP angegeben wird. P ist der Quotient aus der Konzentration der besagten Substanz in der n-Oktanolphase c(Oktanol) und der Konzentration der besagten Substanz in der Wasserphase c(Wasser)

$$P = c(\text{Oktanol}) / c(\text{Wasser})$$

Im Rahmen der vorliegenden Erfindung wird ein berechneter LogP-Wert, der sogenannte cLogP-Wert einer Substanz, verwendet. Zur Ermittlung der erfindungsgemäß verwendeten cLogP-Werte wurde eine in dem Artikel "ACD/Log P method description" aus Perspectives in Drug Discovery and Design, 19, 2000, Seiten 99-116 beschriebene Berechnungsmethode der Firma Advanced Chemistry Development Inc. (ACD) genutzt. Der gesamte vorgenannte Artikel und dessen Offenbarung ist ein Teil der Offenbarung dieser Patentanmeldung. Die Berechnung der cLogP-Werte (25°C) erfolgte mit der Software ACD/Percepta 14.0.0 (Build 2726) (Advanced Chemistry Development Inc.) mit dem ACD/LogP Classic Module.

Ein ganz besonders bevorzugtes partikelförmiges Feststoff-Kompositmaterial dieser Erfindung wird gemäß nachfolgend beschriebenem Verfahren erhalten. Ein weiterer Gegenstand der Erfindung ist daher ein Verfahren zur Herstellung eines partikelförmigen Feststoff-Kompositmaterials zur Nukleinsäureaufreinigung, enthaltend magnetische Nanopartikel eingebettet in eine Trägermatrix auf Basis mindestens eines durch Polyaddition erhältlichen Polymers, dadurch gekennzeichnet, dass das Verfahren zumindest folgende Schritte umfasst

a) Bereitstellen eines magnetischen Fluids in Form einer Suspension, enthaltend magnetische Nanopartikel und eine flüssige kontinuierliche Phase, enthaltend mindestens eine polare organische Flüssigkeit und bezogen auf das Gewicht des Fluids weniger als 5 Gew.-%, bevorzugt weniger als 2 Gew.-%, Wasser,

b) Mischen des magnetischen Fluids mit mindestens einem, Isocyanat-reaktiven-Monomer A1, ausgewählt aus Verbindungen, enthaltend

- mindestens zwei funktionelle Gruppen, die jeweils mindestens ein Zerewitinoff-reaktives Wasserstoffatom tragen und
- zusätzlich zu diesen mindestens zwei funktionellen Gruppen mindestens einen anionischen oder potentiell anionischen Rest (bevorzugt ausgewählt aus Carboxylatgruppe, Sulfonatgruppe, oder Kombinationen daraus),

c) bevorzugt Zugabe mindestens eines Tensids,

d) Emulgieren der Mischung der vorangehenden Schritte in einer flüssigen kontinuierlichen Phase, enthaltend mindestens eine unpolare, organische Flüssigkeit,

e) Zugabe mindestens eines Polyisocyanat-Monomers B,

f) nach Ablauf der Reaktionszeit, Abtrennen des gebildeten Kompositmaterials und gegebenenfalls Waschen,

mit der Maßgabe, dass der cLogP-Wert (25°C) der polaren organischen Flüssigkeit kleiner als der cLogP-Wert (25°C) der unpolaren, organischen Flüssigkeit ist und die flüssige, kontinuierliche Phase aus Schritt d) bezogen auf ihr Gewicht weniger als 5 Gew.-%, bevorzugt weniger als 2 Gew.-%, Wasser enthält.

Für das erfindungsgemäße Verfahren ist es wesentlich, dass die Emulsionspolymerisation in einem wasserarmen bis wasserfreien Milieu abläuft. Dies kann beispielsweise dadurch gewährleistet werden, in dem alle eingesetzten Komponenten frei oder nahezu frei von Wasser sind, d.h. weniger als 5 Gew.-%, insbesondere weniger als 2 Gew.-% Wasser enthalten.

In Schritt a) des erfindungsgemäßen Verfahrens wird ein magnetisches Fluid bereitgestellt, welches eine geringe Menge an Wasser enthält und demnach im Sinne der vorherigen Definition nahezu wasserfrei ist.

Falls nicht anders gekennzeichnet, beziehen sich Angaben einer Wassermenge auf den Gehalt an freiem Wasser. Nicht berücksichtigt ist der Gehalt an molekular gebundenem Wasser oder Kristallwasser, den einzelne Bestandteile aufweisen können. Der Wassergehalt kann beispielsweise analog DIN EN 14346:2007-03 (Verfahren B) mittels Karl-Fischer-Titration bestimmt werden.

Als ein bevorzugt für das erfindungsgemäße Verfahren geeignetes magnetisches Fluid eignet sich erfindungsgemäß ein magnetisches Fluid, in dem zuvor genannte bevorzugte magnetische Nanopartikel in besagter flüssiger kontinuierlicher Phase suspendiert vorliegen.

Das im Schritt a) eingesetzte magnetische Fluid weist eine flüssige kontinuierliche Phase auf, die mindestens eine polare organische Flüssigkeit enthält. Dabei ist es erfindungsgemäß bevorzugt, wenn besagte polare, organische Flüssigkeit einen cLogP-Wert < 1,5 (25°C), besonders bevorzugt einen cLogP-Wert < 1,0 (25°C), ganz besonders bevorzugt einen cLogP-Wert <0 (25°C), besitzt.

Besonders bevorzugt im erfindungsgemäßen Verfahren verwendbare polare organische Flüssigkeiten sind flüssige organische Verbindungen mit mindestens einer funktionellen Gruppe, ausgewählt aus Amid, Nitril, Nitro, Sulfoxid, N-Oxid, Lacton, N-Alkylpyrrolidon. Ganz besonders bevorzugt geeignete polare organische Flüssigkeiten werden ausgewählt aus N,N-Dimethylformamid, Acetonitril, N-Methyl-2-pyrrolidon, N-Methylformamid, γ-Butyrolacton, 4-Methyl-1,3-dioxolan-2-on, Dimethylsulfoxid, Sulfolan, Nitromethan oder Kombinationen aus mindestens zwei dieser Verbindungen. Am bevorzugtesten wird als polare organische Flüssigkeit zumindest N,N-Dimethylformamid eingesetzt.
Die cLogP-Werte (25°C) der ganz besonders bevorzugten polaren organischen Flüssigkeiten sind in Tabelle 2 zusammengestellt.

Tabelle 2: cLogP-Werte bevorzugter polarer organischer Flüssigkeiten

| **Substanz** | **cLogP (25°C)** |
|---|---|
| N,N-Dimethylformamid | **-1,01** |
| Acetonitril | **-0,45** |
| N-Methyl-2-pyrrolidon | **-0,40** |
| N-Methylformamid | **-0,60** |
| γ-Butyrolacton | **-0,76** |
| 4-Methyl-1,3-dioxolan-2-on | **-0,41** |
| Dimethylsulfoxid | **-1,35** |
| Sulfolan | **-0,77** |
| Nitromethan | **-0,20** |

Die polare organische Flüssigkeit ist in dem magnetischen Fluid bezogen auf das Gewicht des magnetischen Fluids in einer Gesamtmenge von 85 bis 99 Gew.-%, insbesondere von 92 Gew.-% bis 98 Gew.-%, enthalten.

Es hat sich als erfindungsgemäß vorteilhaft herausgestellt, die Polyadditionsreaktion in Gegenwart mindestens eines dafür geeigneten Katalysators stattfinden zu lassen. Zu diesem Zweck wird bevorzugt, insbesondere vor der Zugabe des Polyisocyanat-Monomers B, mindestens ein Katalysator für die Polyadditionsreaktion der Monomere A (bzw. A1 und A2) und B mit dem Ferrofluid oder der Mischung aus Schritt b) oder aus Schritt c) gemischt.

Als erfindungsgemäß einsetzbare Katalysatoren eignen sich z.B. organische Verbindungen des Zinns, Eisens, Titans oder Bismuts wie Zinn(II)salze von Carbonsäuren, z.B. Zinn-II-acetat, - ethylhexanoat und -diethylhexanoat verwendet werden. Ebenso geeignet sind Dialkyl-Zinn(IV)-Carboxylate, wie beispielsweise Dibutyl- und Dioctyl-zinndiacetat, -maleat, -bis-(2-ethylhexanoat), -dilaurat, Tributylzinnacetat, Bis(β-methoxycarbonyl- ethyl)zinndilaurat und Bis(β-acetylethyl)zinndilaurat. Gleichfalls geeignet sind Zinnoxide und -sulfide sowie -thiolate.

Weiterhin als Katalysator im Sinne der Erfindung eignen sich Derivate des Morpholins. Konkrete Beispiele für solche Morpholino-Verbindungen sind Bis(2-(2,6-dimethyl-4-morpholino)ethyl)-(2-(4-morpholino)ethyl)amin, Bis(2-(2,6-dimethyl-4-morpholino)ethyl)-(2-(2,6-diethyl-4-morpholino)ethyl)amin, Tris(2-(4-morpholino)ethyl)amin, Tris(2-(4-morpholino)propyl)amin, Tris(2-(4-morpholino)butyl)amin, Tris(2-(2,6-dimethyl-4-morpholino)ethyl)amin, Tris(2-(2,6-diethyl-4-morpholino)ethyl)amin, Tris(2-(2-methyl-4-morpholino)ethyl)amin oder Tris(2-(2-ethyl-4-morpholino)ethyl)amin, Dimethylaminopropylmorpholin, Bis-(morpholinopropyl)-methylamin, Diethylaminopropylmorpholin, Bis-(morpholino-propyl)-ethylamin, Bis-(morpholinopropyl)-propylamin, Morpholinopropylpyrrolidon oder N-Morpholinopropyl-N'-methyl-piperazin, Dimorpholinodiethylether (DMDEE) oder Di-2,6-dimethylmorpholinoethylether.

Besonders bevorzugt wird im Rahmen der vorliegenden Erfindung mindestens ein aliphatisches tertiäres Amin, insbesondere mit cyclischer Struktur, und/oder mindestens ein ungesättigtes bicyclisches Amin eingesetzt. Insbesondere wird als bevorzugter Katalysator des erfindungsgemäßen Verfahrens mindestens eine Verbindung aus Diaza-bicyclooctan (DABCO), Triethylamin, Dimethylbenzylamin, Bis-dimethyl-aminoethylether (Calalyst A 1, UCC), Tetramethylguanidin, Bis-dimethyl-aminomethyl-phenol, 2-(2-Dimethylaminoethoxy)ethanol, 2-Dimethylaminoethyl-3-dimethylaminopropylether, Bis(2-dimethylaminoethyl)ether, N,N-Dimethylpiperazin, N-(2-hydroxyethoxyethyl)-2-azanorbomane, Diazabicycloundecen (DBU), N,N,N,N-Tetramethylbutan-1,3-diamin, N,N,N,N-Tetramethylpropan-1,3-diamin, N,N,N,N-Tetramethylhexan-1,6-diamin oder Kombinationen daraus, eingesetzt. Das tertiäre Amin kann auch in oligomerisierter oder polymerisierter Form vorliegen, z.B. als N-methyliertes Polyethylenimin.

Die Katalysatoren werden vorzugsweise in einer Menge von 0,0001 bis 5 Gew.-%, vorzugsweise 0,001 bis 2 Gew.-%, bezogen auf das Gewicht der eingesetzten, gegenüber Isocyanaten reaktiven Verbindungen (i.e. Monomer A1, A2, A2a, A2b) zugesetzt.

Tenside können in Wasser Aggregate, sogenannte Micellen, bilden. Als Micellen werden diejenigen Aggregate bezeichnet, die sich aus Tensidmolekülen in wässrigen Lösungen oberhalb einer bestimmten Temperatur (Krafft-Punkt) und einer charakteristischen Konzentration bilden. Diese charakteristische Konzentration, auch "kritische Mizellbildungskonzentration" (CMC) genannt, ist im Einklang mit dem allgemeinen Verständnis im Stand der Technik die Konzentration des entsprechenden Stoffs, über welcher diese anfängt Mizellen zu bilden und jedes weitere Molekül, das dem System zugesetzt wird, in die Mizellen übergeht. Verbindungen sind erfindungsgemäß zu den Tensiden zu zählen, wenn diese in Wasser bei 20°C bei einer definierten CMC zur Bildung von Micellen fähig sind.

Der Einsatz der Tenside erfolgt erfindungsgemäß unabhängig von der Zugabe der zuvor definierten Isocyanat-reaktiven Monomere, wodurch definitionsgemäß Tenside im Sinne der Erfindung nicht zu den Isocyanat-reaktiven Verbindungen, insbesondere zu den Monomeren A1, A2, A2a und A2b, zu zählen sind.

Im Schritt c) des erfindungsgemäßen Verfahrens werden bevorzugt nichtionische Tenside zugegeben.

Es haben sich insbesondere solche nichtionischen Tenside als besonders geeignet herausgestellt, die einen HLB-Wert (nach Griffin) von 1 bis 20, insbesondere von 1 bis 10, aufweisen.

Ganz besonders bevorzugt werden nichtionische Tenside im Schritt c) des erfindungsgemäßen Verfahrens zugegeben, die mindestens eine Hydroxylgruppe tragen.

Am bevorzugtesten wird mindestens ein Sorbitansäure-Ester der folgenden Formel (II) als nichtionisches Tensid im Schritt c) des erfindungsgemäßen Verfahrens zugegeben

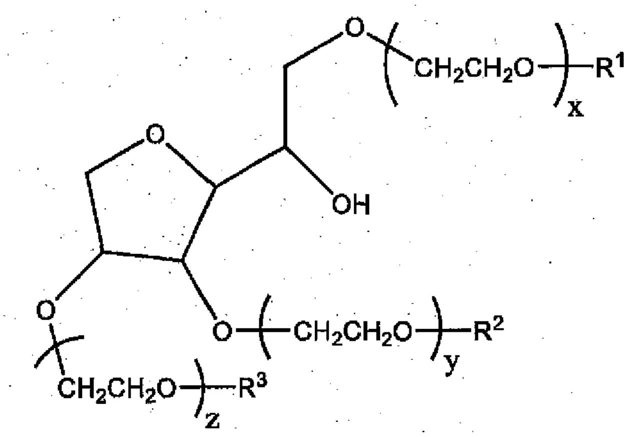

(II)

worin die Summe aus x + y + z die Zahl 0 bis 100, insbesondere 0 bis 80, ganz besonders bevorzugt Null bedeutet, $R^1$, $R^2$ und $R^3$ unabhängig voneinander stehen für ein Wasserstoffatom, eine gesättigte ($C_6$ bis $C_{20}$)-Acylgruppe, oder eine ungesättigte ($C_6$ bis $C_{20}$)-Acylgruppe mit der Maßgabe, dass mindestens einer der Reste $R^1$, $R^2$ oder $R^3$ für eine gesättigte ($C_6$ bis $C_{20}$)-Acylgruppe, oder eine ungesättigte ($C_6$ bis $C_{20}$)-Acylgruppe steht.

Besonders bevorzugt stehen gemäß Formel (II) y und z für Null, x für eine Zahl von 0 bis 20 (insbesondere x = 0), $R^1$ steht für eine gesättigte ($C_6$ bis $C_{20}$)-Acylgruppe, oder eine ungesättigte ($C_6$ bis $C_{20}$)-Acylgruppe und $R^2$ und $R^3$ bedeuten ein Wasserstoffatom.

Es sind solche Sorbitansäure-Ester der Formel (II) bevorzugt, die einen HLB-Wert (nach Griffin) von 1 bis 20, insbesondere von 1 bis 10, aufweisen.

Besonders bevorzugte nichtionische Tenside sind ausgewählt aus (jeweils gegebenenfalls ethoxyliertem) Sorbitanmonolaurat, Sorbitanmonooleat, Sorbitanmonostearat, Sorbitantrioleat. Entsprechende nichtionische Tenside sind beispielsweise unter der Bezeichnung Span 60, Span 65, Span 80,Span 85 oder Brij 25 erhältlich.

Das Tensid, insbesondere das nichtionische Tensid (bevorzugt das Tensid der Formel (II)) wird bezogen auf das Gewicht des magnetischen Fluids bevorzugt in einer Gesamtmenge von 0 Gew.-% bis 60 Gew.-%, besonders bevorzugt von 0 Gew.-% bis 30 Gew.-%, zugesetzt.

Ganz besonders bevorzugt werden die zur Herstellung des zuvor beschriebenen erfindungsgemäßen Kompositmaterials bevorzugt herangezogenen Monomere in Gegenwart eines im Schritt c) zugesetzten Tensids umgesetzt. Der Einsatz von mindestens einem nichtionischen Tensid, insbesondere von Sorbitansäure-Estern der Formel (II), ist wiederum in Kombination mit den als bevorzugt aufgeführten Monomeren (insbesondere der Tabelle 1) erfindungsgemäß bevorzugt.

Dem Gemisch aus Schritt b) bzw. Schritt c) des erfindungsgemäßen Herstellverfahrens wird vor der Emulgierung

eine unpolare organische Flüssigkeit zugefügt. Als besonders wirkungsvoll stellte es sich heraus, wenn die unpolare organische Flüssigkeit des Schritts d) einen cLogP-Wert > 2,0 (25°C) aufweist. Dabei ist es wiederum ganz besonders bevorzugt, wenn besagte polare, organische Flüssigkeit einen cLogP-Wert < 1,5 (25°C) (insbesondere einen cLogP-Wert < 1,0 (25°C), besonders bevorzugt einen cLogP-Wert < 0 (25°C)) und besagte unpolare, organische Flüssigkeit einen cLogP-Wert > 2,0 (25°C) aufweist.

Tabelle 3: cLogP-Werte bevorzugter unpolarer organischer Flüssigkeiten

| **Substanz** | **cLogP (25°C)** |
|---|---|
| Cyclohexan | **3,39** |
| Hexan | **3,94** |
| Pentan | **3,41** |
| Heptan | **4,47** |
| Octan | **5,01** |

Es ist erfindungsgemäß bevorzugt, wenn die unpolare organische Flüssigkeit ausgewählt wird aus aliphatischen Kohlenwasserstoffen, alizyklischen Kohlenwasserstoffen, Triglycerid (insbesondere pflanzlichem Öl wie beispielsweise Rapsöl), Silikonöl, pflanzliches Öl oder Kombinationen mindestens zweier dieser Verbindungen. Besonders bevorzugt wird die unpolare organische Flüssigkeit ausgewählt aus flüssigem Kohlenwasserstoff (insbesondere Paraffinöl, Petrolether, flüssige Kohlenwasserstoffe mit 5 bis 16 Kohlenstoffatomen), Silikonöl, pflanzlichem Öl oder Kombinationen daraus.

Die polare organische Flüssigkeit wird bevorzugt in einem Volumenverhältnis bezogen auf das Volumen der unpolaren organischen Flüssigkeit von 1:1 bis 1:20, bevorzugt von 1:2 bis 1:15, besonders bevorzugt von 1:5 bis 1:10 eingesetzt.

Wenngleich der Einsatz eines Tensids gemäß Schritt c) bevorzugt ist, kann der Emulgationsschritt d) bei ausreichender mechanischer Durchmischung (z.B. ausreichender Rührgeschwindigkeit) auch ohne die Gegenwart eines Tensids erfolgen.

Gemäß Schritt d) des erfindungsgemäßen Verfahrens wird das Gemisch bevorzugt unter Rühren emulgiert. Zu diesem Zweck lassen sich beispielsweise bekannte KPG-Rührer mit bekannten Rührsystemen einsetzen (z.H. Rührwerk RW 28 digital oder RW 47 digital der Firma IKA-Werke GmbH & Co. KG, Staufen). Als Rührsysteme sind dabei alle gängigen Ausführungsformen (Propellerrührer, Turbinenrührer, Scheibenrührer, Flächenrührer, Ankerrührer, Wendelrührer) einsetzbar.

Die bevorzugte Rührgeschwindigkeit ergibt sich in Abhängigkeit von dem eingesetzten Rührsystem, dem Durchmesser und der Ausführungsform des Rührers (z.B. Propellerrührer dreiblättrig oder vierblättrig) sowie der Reaktionsgefäßgeometrie. Für die in den Reaktionsbeispielen beschriebene Ausführungsform (1L-Reaktorbehälter, KPG-Rührer Modell RZR 2102 control der Fa. Heidolph, dreiblättriger Propellerrührer mit Schaftdurchmesser 10 mm, Schaftlänge 600 mm, Rührerdurchmesser 75 mm) ist es bevorzugt bei einer Rührgeschwindigkeit im Bereich von 150 U/min bis 1200 U/min, besonders bevorzugt von 200 U/min bis 600 U/min, zu rühren.

Der Emulgationsschritt d) wird bevorzugt bei einer Temperatur von 35°C bis 90°C, insbesondere von 40°C bis 80°C, ganz besonders bevorzugt von 45°C bis 75°C, durchgeführt. Ganz besonders bevorzugt werden die bevorzugten Rührparameter und die bevorzugten Temperaturen eingestellt.

Im Rahmen dieses Herstellverfahrens sind wiederum die zuvor erwähnten bevorzugten Monomere A1, A2, A2a, A2b, B, insbesondere in deren zuvor genannten bevorzugten Kombinationen, bevorzugt einsetzbar.

Im Schritt e) des Herstellungsverfahrens bevorzugt nutzbare Polyisocyanat-Monomere weisen darüber hinaus einen cLogP-Wert (25°C) von > 1,5, insbesondere von > 2,0, besonders bevorzugt von > 2,5, auf.

Tabelle 4: cLogP-Werte (25°C) bevorzugter Polyisocyanat-Monomere

| **Substanz** | **cLogP (25°C)** |
|---|---|
| Toluol-2,4-dilsocyanat (m-TDI) | **3,47** |
| Toluol-2,6-diisocyanat | **3,47** |
| Isophorondiisocyanat | **4,67** |
| Hexamethylendiisocyanat | **3,03** |

(fortgesetzt)

| Substanz | cLogP (25°C) |
|---|---|
| Methylendiphenyldilsocyanat | **4,93** |

Durch Anwendung eines speziellen Verhältnisses der funktionellen Gruppen mit Zerewitinoff-reaktiven Wasserstoffatomen aller in der Synthese eingesetzten Isocyanat-reaktiven Monomere zu den Isocyanatgruppen aller in der Synthese eingesetzten Polyisocyanat-Monomere lassen sich die Eigenschaften des Kompositmaterials in der Magnetseparation und die Durchführung des Herstellverfahrens des Kompositmaterials optimieren. Daher ist erfindungsgemäß ein Verfahren bevorzugt, das sich dadurch kennzeichnet, dass das Stoffmengenverhältnis der Stoffmenge n(Zerewitinoff) der funktionellen Gruppen mit Zerewitinoff-aktiven Wasserstoffatomen aller eingesetzten Isocyanat-reaktiven Monomere zur Stoffmenge n(NCO) der Isocyanatgruppen aller eingesetzten Polyisocyanat-Monomere 5:1 bis 1:5, besonders bevorzugt 4:1 bis 1:4, ganz besonders bevorzugt 2:1 bis 1:3, am bevorzugtesten 1.5:1 bis 1:2 beträgt.

Es ist erfindungsgemäß bevorzugt, das Polyisocyanat-Monomer B vor dessen Zugabe mit mindestens einer im Schritt d) definierten unpolaren organischen Flüssigkeit zu versetzen.

Die Zugabe des Polyisocyanat-Monomers B wird bevorzugt unter Rühren durchgeführt. Dabei gelten die für Schritt d) aufgeführten Rührgeschwindigkeiten und Temperaturen wiederum als bevorzugt.

Die Polyadditionsreaktion findet bevorzugt ebenfalls unter Rühren, besonders bevorzugt gemäß den für Schritt d) beschriebenen Rührparametern, statt.

Zudem ist es erfindungsgemäß bevorzugt, wenn die Polyadditionsreaktion bei einer Temperatur von 35°C bis 90°C, insbesondere von 40°C bis 80°C, ganz besonders bevorzugt von 45°C bis 75°C, erfolgt.

Die vom Beginn der Zugabe des Polyisocyanat-Monomers B an berechnete Reaktionsdauer der Polyadditionsreaktion beträgt 10 bis 150 Stunden, bevorzugt 24 bis 120 Stunden, besonders bevorzugt 36 bis 100 Stunden. Dabei ist es ganz besonders bevorzugt, wenn mindestens ein Katalysator für die Polyaddition verwendet wird und das Reaktionsgemisch gerührt und erwärmt wird. Dabei sind wiederum bevorzugt zuvor genannte Katalysatoren, Rührparameter und Temperaturen während der Reaktionsdauer eingestellt.

Die Reaktion kann beispielsweise nach Ablauf der Reaktionsdauer durch Zugabe von Wasser gestoppt werden.

Die erhaltenen magnetischen Kompositpartikel werden zur Aufreinlgung beispielsweise in Wasser oder verschiedenen organischen Lösemitteln, wie z.B. Ethanol oder Aceton, als Waschlösung aufgeschlämmt und (bevorzugt durch Anlegen eines Magnetfeldes) gesammelt und von dieser Waschlösung wieder entfernt.

Das zuvor beschriebene erfindungsgemäße partikelförmige Feststoff-Kompositmaterial wird bevorzugt zur Aufreinigung von Nukleinsäuren verwendet.

Ein weiterer Gegenstand der Erfindung ist daher ein Verfahren zur Aufreinigung von Nukleinsäuren aus einer Nukleinsäure-haltigen, biologischen Probe, umfassend die folgenden Schritte:

a) Bereitstellen einer flüssigen Probe, enthaltend Nukleinsäure in gelöster Form;

b) Abscheidung zumindest von Nukleinsäure aus der flüssigen Probe heraus an ein erfindungsgemäßes partikelförmiges Feststoff-Kompositmaterial (*vide supra*);

c) Abtrennen des mit Nukleinsäure beladenen, partikelförmigen Feststoff-Kompositmaterials aus Schritt b) durch Anlegen eines Magnetfeldes und gegebenenfalls Waschen der abgetrennten Partikel mit einer Waschlösung;

d) Inlösungbringen der verbliebenen Nukleinsäure aus dem gemäß Schritt c) behandelten partikelförmigen Feststoff-Kompositmaterial mittels eines Lösungspuffers und Abtrennen des partikelförmigen Feststoff-Kompositmaterial von der nukleinsäurehaltigen Lösung.

Bei dem erfindungsgemäßen Aufreinigungsverfahren handelt es sich um ein einfaches "Bind-Wash-Elute"-Verfahren. Im Rahmen eines "Bind-Wash-Elute"-Verfahrens werden Nukleinsäuren in dem erfindungsgemäßen Verfahren gemeinsam mit unerwünschten Stoffen mittels bekannter Verfahren aus der flüssigen Probe abgeschieden und die abgeschiedenen Bestandteile der Probe durch Einsatz mindestens einer Waschlösung von einem Großteil der Kontaminierung befreit. Mit dieser Methode kann auf schnelle und kostengünstige Weise eine deutliche Reduzierung der Kontaminierung erreicht werden und die eluierte Nukleinsäure, insbesondere die eluierte DNA, ist je nach gewählter Methode für die Nukleinsäureabscheidung direkt in Nachfolgeapplikationen, beispielsweise einer Polymerasekettenreaktion (PCR), einsetzbar.

Als Nukleinsäuren kommen RNA und DNA unterschiedlicher Kettenlänge in Frage, insbesondere mit mehr als fünfzehn Nukleotiden, wie beispielsweise einzel- und doppelsträngige bakterielle, virale, humane, tierische oder pflanzliche RNA oder DNA, insbesondere genomische DNA, mitochondriale DNA, Plasmide, mRNA, tRNA, rRNA, miRNA und andere kurze RNA-Spezies, insbesondere mit einer Kettenlänge von 15 bis 25 Nukleotiden. Unter "Nukleinsäure" wird erfindungsgemäß bevorzugt Desoxyribonukleinsäure (DNA) verstanden.

Zur Herstellung der flüssigen Nukleinsäure-haltigen Probe wird eine biologische Probe verwendet. Als solche biologische Probe eignet sich jedwedes biologische Material, das zumindest Nukleinsäuren enthält, wie Bakterienkulturen, tierisches oder menschliches Gewebe, Gewebeteile, Körperflüssigkeiten wie Speichel, Sputum, Liquor, Vollblut, Serum oder Plasma. Bakterien, Hefen und andere Pilze oder Viren werden ebenso unter "Probenmaterial" verstanden, wie auch PCR-Amplifikationsreaktionen, die Primer und DNA Fragmente enthalten, oder Zellkulturüberstände. Probenmaterial kann auch Umwelt- oder Lebensmittelproben umfassen. Auch künstliches Probenmaterial, z.B. mit synthetisch oder in-vitro erzeugten Nukleinsäuren fällt unter den Anwendungsbereich der vorliegenden Erfindung.

Zur Bereitstellung einer flüssigen Probe, enthaltend Nukleinsäure in gelöster Form, kann im Rahmen des Schritts a) des erfindungsgemäßen Aufreinigungsverfahrens die biologische Probe zunächst aufgeschlossen, d.h. lysiert, werden um die Nukleinsäuren aus dem Material freizusetzen. Der Aufschluss kann einen mechanischen, chemischen und/oder enzymatischen Aufschluss umfassen. Der Aufschluss der biologischen Probe wird häufig durch eine geeignete Pufferchemie unterstützt, die beispielsweise Detergenzien beinhaltet. Geeignete Lysebedingungen sind dem Fachmann bekannt.

Im Schritt b) des erfindungsgemäßen Aufreinigungsverfahrens wird die Nukleinsäure meist zusammen mit Kontaminierungen mittels eines beliebigen dem Fachmann bekannten Verfahrens aus der flüssigen Probe abgeschieden. Das erfindungsgemäße Verfahren ist dabei nicht auf ein bestimmtes Prinzip der Abscheidung beschränkt. Dem Stand der Technik sind verschiedenartige Verfahren zu entnehmen, die angewendet werden können und dem Fachmann bekannt sind. Diese umfassen beispielsweise die Verwendung chaotroper Salze, die Verwendung anti-chaotroper Salze, Ausfällungen (z.B. Fällungen mit Polyethylenglycol oder niederen Alkoholen), Filtration, die Ausnutzung hydrophober Wechselwirkungen für die Nukleinsäurebindung an ein Trägermaterial und andere Verfahren.

Eine bevorzugte Form der Abscheidung ist dadurch gekennzeichnet, dass im Schritt b) zumindest Nukleinsäure durch Bindung, insbesondere durch Adsorption oder Fällung, an die Oberfläche des erfindungsgemäßen partikelförmigen Feststoff-Kompositmaterials abgeschieden werden. Dabei ist es wiederum besonders bevorzugt, wenn die Probe im Schritt b) zur Abscheidung an das erfindungsgemäße Feststoff-Kompositmaterial mit einem Bindepuffer versetzt wird.

Der Bindepuffer enthält bevorzugt mindestens ein chaotropes Salz und/oder mindestens einen Monoalkohol mit bis zu 16 Kohlenstoffatomen.

Gemäß geltender Theorien zerstören die chaotropen Salze die geordnete Wasserstruktur um in Wasser gelöste Verbindungen. Chaotrope Salze sind demnach so definiert, dass sie Proteine denaturieren, die Löslichkeit unpolarer Substanzen in Wasser erhöhen sowie hydrophobe Wechselwirkungen zerstören. Es ist bekannt, dass Nukleinsäure in Gegenwart chaotroper Salze reversibel an Trägermaterialien, insbesondere an Silikate und an andere anorganische Trägermaterialien, bindet. Die chaotropen Salze bewirken hierbei eine Zerstörung der Hydrathülle der Nukleinsäuren und schaffen eine hydrophobe Mikroumgebung. Unter diesen Bedingungen binden sowohl Nukleinsäuren als auch einige Kontaminanten an das feste Trägermaterial, während Proteine und andere Kontaminanten nicht binden und ausgewaschen werden. Die Stärke des chaotropen Charakters eines Salzes ist in der so genannten Hofmeister-Reihe beschrieben.

In dem Bindepuffer sind chaotropes Salz bzw. chaotrope Salze bevorzugt in einer Konzentration von 1 bis 6 mol/L enthalten.

Im Rahmen der vorliegenden Erfindung werden Natriumperchlorat, Natriumiodid, Guanidiniumisothiocyanat, Guanidiniumhydrochlorid, Kaliumthiocyanat, Guanidiniumnitrat, Guanidiniumcarbonat, Harnstoff oder auch Kombination hiervon als chaotrope Salze bevorzugt verwendet.

Es ist erfindungsgemäß ganz besonders bevorzugt, wenn der Bindepuffer mindestens ein chaotropes Salz oder mindestens einen Monoalkohol mit bis zu 16 Kohlenstoffatomen oder ein organisches Polymer enthält.

Die Abscheidung von Nukleinsäure an das erfindungsgemäße Kompositmaterial kann dadurch erzielt werden, dass die Probe mit dem erfindungsgemäßen Kompositmaterial in Kontakt gebracht wird. Dabei können die Partikel des erfindungsgemäßen Kompositmaterials auch direkt zur Probe gegeben werden. Mit dem Zusatz der Partikel kann zur Verbesserung der Bindebedingungen die Probe danach noch mit Bindepuffer (*vide supra*), versetzt werden.

Als weitere bevorzugte Möglichkeit der Abscheidung gemäß Schritt b) können zumindest die Nukleinsäure aus der flüssigen Probe als Feststoff durch Fällung auf das erfindungsgemäße Kompositmaterial abgeschieden werden. Hierzu kann der flüssigen Probe ein Fällungsreagenz zugegeben werden, das die Nukleinsäure als Feststoff aus der flüssigen Probe ausfallen lässt. Das Fällungsreagenz enthält bevorzugt mindestens eine Verbindung ausgewählt aus Tripropylenglycol (bevorzugt in Kombination mit einem ($C_1$-$C_4$)-Alkohol (besonders bevorzugt Methanol, Ethanol, n-Propanol oder Isopropanol, ganz besonders bevorzugt Ethanol)), Polyethylenglycolen mit einem Molekulargewicht zwischen 300 bis 10000 g/mol, Polypropylenglycolen mit einem Molekulargewicht zwischen 300 bis 10000 g/mol, kationischen Detergentien (wie Hexadecyltrimethylammoniumbromid (CTAB), Pyridiniumsalze oder quartäre Ammoniumverbindungen mit einem langen, kettenförmigen Kohlenwasserstoffrest ($C_6$-$C_{18}$) und drei Resten, ausgewählt unter kurzen Kohlenwasserstoffresten ($C_1$-$C_3$) oder Wasserstoff), Ethanol, n-Propanol, Isopropanol oder Kombinationen daraus.

Im Schritt c) des erfindungsgemäßen Aufreinigungsverfahrens wird das nukleinsäurehaltige, partikelförmige Feststoff-Kompositmaterial durch Anlegen eines Magnetfeldes abgetrennt und gegebenenfalls durch Wäschen der abgetrennten Partikel mit einer Waschlösung weiter aufgereinigt.

Die in Schritt c) gegebenenfalls eingesetzte Waschlösung enthält bevorzugt eine organische Amin-Verbindung (*vide supra*). Die organische Amin-Verbindung wird beispielsweise unter Einhaltung vorgenannter zwingender Merkmale ausgewählt aus mindestens einer Verbindung der Gruppe, die gebildet wird aus Verbindungen mit einer Aminogruppe, Verbindungen mit mindestens zwei Aminogruppen, Verbindungen mit mindestens einer Aminogruppe und mindestens einer Hydroxylgruppe, Verbindungen mit mindestens zwei Aminogruppen und mindestens einer Hydroxylgruppe (besonders bevorzugt aus mindestens einer Verbindung der Gruppe, die gebildet wird aus Verbindungen mit einer Aminogruppe, Verbindungen mit mindestens einer Aminogruppe und mindestens einer Hydroxylgruppe).

Es ist erfindungsgemäß bevorzugt, wenn die besagte Amin-Verbindung eine Molmasse von 80 bis 500 g/mol besitzt.

Zur Verbesserung der Löslichkeit in Wasser ist es bevorzugt, wenn die Amin-Verbindung mindestens zwei Hydroxylgruppen aufweist.

Besonders geeignete organische Amin-Verbindungen weisen bevorzugt gegebenenfalls substituierte. Kohlenwasserstoffreste mit 1 bis 6 Kohlenstoffatomen auf. Als Substituenten eignen sich insbesondere Hydroxy oder Alkoxy.

Die organische Amin-Verbindung der Waschlösung wird beispielsweise ausgewählt aus Triethylamin, Triethanolamin, 2-Amino-2-(hydroxymethyl)-propan-1,3-diol (TRIS), 2,2-Bis(hydroxymethyl)-2,2',2"-nitrilotriethanol (BIS-TRIS), 1,3-Bis[tris(hydroxymethyl)methyl-amino]propan (BIS-TRIS propan), Diisopropylamin, Triisopropylamin, oder Kombinationen davon. Ganz besonders bevorzugt wird die organische Amin-Verbindung der Waschlösung ausgewählt aus Triethylamin, Triethanolamin, 2-Amino-2-(hydroxymethyl)-propan-1,3-diol (TRIS), 2,2-Bis(hydroxymethyl)-2,2',2"-nitrilotriethanol (BIS-TRIS), 1,3-Bis[tris(hydroxymethyl)methyl-amino]propan (BIS-TRIS propan), oder Kombinationen davon.

Die Waschlösung gemäß Schritt c) enthält zwingendmindestens ein von der besagten Amin-Verbindung verschiedenes, organisches Lösemittel. Das organische Lösemittel ist in der Waschlösung bevorzugt in einer Gesamtmenge von 20 bis 80 Gew.-% enthalten, bezogen auf das Gesamtgewicht der Waschlösung.

Es hat sich als vorteilhaft erwiesen, wenn die Waschlösung als bevorzugtes organisches Lösemittel mindestens einen Monoalkohol enthält. Besonders bevorzugt enthält die Waschlösung als organisches Lösemittel mindestens einen $C_1$-$C_6$-Monoalkohol, insbesondere Ethanol, Isopropanol oder Kombinationen davon. Dabei ist es wiederum ganz besonders bevorzugt, wenn die Waschlösung den $C_1$-$C_6$-Monoalkohol in einer Gesamtmenge von 20 Gew.-% bis 80 Gew.-% bezogen auf das Gesamtgewicht der Waschlösung enthält.

Im Schritt d) des erfindungsgemäßen Aufreinigungsverfahrens erfolgt das Inlösungbringen der verbliebenen Nukleinsäure aus den gemäß Schritt c) gewaschenen, abgeschiedenen Bestandteilen mittels eines Lösungspuffers und das Abtrennen des partikelförmigen Feststoff-Kompositmaterial von der nukleinsäurehaltigen Lösung.

Beim Inlösungbringen wird die verbliebene, abgeschiedene Nukleinsäure in dem Lösungspuffer gelöst und von dem magnetischen Kompositmaterial entfernt. Bevorzugte Lösungspuffer, die sich für das Inlösungbringen der verbliebenen, abgeschiedenen Nukleinsäure eignen, weisen eine geringe Ionenstärke auf. Beispielsweise eignet sich ein Lösungspuffer, der 5 bis 10 mM TRIS (gegebenenfalls in Kombination mit bis zu 1 mM Ethylendiamintetraessigsäure (EDTA)) enthält.

Das Inlösungbringen gemäß Schritt d) des erfindungsgemäßen Verfahrens erfolgt bevorzugt durch Umspülen des partikelförmigen Feststoff-Kompositmaterials mit dem Lösungspuffer und anschließender Magnetseparation der erfindungsgemäßen Kompositpartikel. Das Umspülen wird bevorzugt durch einen Fluss des Lösungspuffers durch das feste Kompositmaterial bewerkstelligt. Die Fließrichtung wird besonders bevorzugt durch Rühren, Anlegen eines Vakuums oder durch Zentrifugation vorgegeben. Bei automatisierten Plattformen kann das Umspülen auch durch wiederholtes Zu- und Abpipettieren des zugegebenen Lösungspuffers oder durch wiederholtes Auf- und Abfahren der Kammspitzen erfolgen.

Ein weiterer Erfindungsgegenstand ist ein Kit zur Aufreinigung von Nukleinsäuren umfassend ein erfindungsgemäßes partikelförmiges Feststoff-Kompositmaterial (*vide supra*) sowie mindestens einen zusätzlichen Bestandteil, ausgewählt aus einer Bedienungsanleitung zur Durchführung des zuvor beschriebenen erfindungsgemäßen Aufreinigungsverfahrens, Bindepuffer, Waschpuffer, Lösungspuffer zum Inlösungbringen der gereinigten Nukleinsäuren. Alle bevorzugten Ausführungsformen des erfindungsgemäßen Aufreinigungsverfahrens gelten ebenso für das in der Bedienungsanleitung des Kits beschriebene Aufreinigungsverfahren.

Alle bevorzugten Ausführungsformen des erfindungsgemäßen Kompositmaterials gelten ebenso für das Kit.

## Beispiele

### I. Herstellung des magnetischen Fluids

#### Beispiel 1: Herstellung Ferrofluid F1

In einem 3 L Becherglas wurden 44,86 g Eisen(III)-chlorid Hexahydrat und 16,50 g Eisen(II)-chlorid Tetrahydrat vorgelegt und unter Rühren bei 660 U/min (KPG-Rührer (Fa. Heidolph, Modell RZR 2102 control) mit dreiblättrigem Propellerrührer (VWR, Art.-Nr.: BOHLC378-20, Schaftdurchmesser 10 mm, Schaftlänge 600 mm, Rührerdurchmesser 75 mm)) in 1900 mL VE-Wasser gelöst. Über einen Tropftrichter erfolgte die tropfenweise Zugabe von 100 mL einer 25%igen Ammoniumhydroxid-Lösung. Nach 30 Minuten Reaktionsdauer wurde der gebildete Niederschlag mit Hilfe eines NdFeB-Magneten von der überstehenden Lösung abgetrennt und dreimal mit VE-Wasser gewaschen. Die Partikel wurden mit einer Ultraschallsonde (Fa. Branson, DIGITAL Sonifier®, Modell 450, Sonde 18 mm) bei einer Amplitude von 70% und einer Ultraschalldauer von 30 Minuten (Pulsdauer: 1 s on, 0,5 s off) in 600 mL VE-Wasser dispergiert. Die Dispersion wurde mit 3,5 g Natriumcitrat-Dihydrat versetzt und erneut mit der Ultraschallsonde dispergiert (Amplitude 70%, 30 Minuten, Pulsdauer: 1 s on, 0,5 s off).

Am Rotationsverdampfer wurden bei 70°C 400 mL Wasser von dem Ferrofluid abdestilliert und die Partikel wurden anschließend aus dem noch warmen Ferrofluid mit einem NdFeB-Magneten weitestgehend von der Lösung abgetrennt. Am Ende des Abtrennungsprozesses wurden 200 mL auf 80°C erwärmtes N,N-Dimethylformamid zur Verbesserung des Abtrennprozesses zugegeben. Die abgetrennten Partikel wurden viermal mit 400 mL N,N-Dimethylformamid (ebenfalls auf 80°C erwärmt) gewaschen. Die gewaschenen Partikel wurden mit der Ultraschallsonde (Amplitude 70%, 30 Minuten, Pulsdauer: 1 s on, 0,5 s off) in 300 mL N,N-Dimethylformamid dispergiert. Das so erhaltene Ferrofluid wurde 10 Minuten bei 3000 U/min zentrifugiert (Hettich Zentrifugen, ROTIXA 50 RS) und von dem Bodensatz abgetrennt. Unmittelbar vor der jeweiligen Verwendung zur Synthese der Feststoff-Kompositmaterialien wurde das Ferrofluid jeweils erneut mit der Ultraschallsonde (Amplitude 70%, 15 Minuten, Pulsdauer: 1 s on, 0,5 s off) dispergiert.

<u>Wassergehalt</u>

Zur Bestimmung des Wassergehaltes des Ferrofluides wurden 100 mL Ferrofluid bis zur vollständigen Trocknung destilliert und das Lösungsmittel in einem mit flüssigem Stickstoff gekühlten Kolben aufgefangen. Die Bestimmung des Wassergehaltes erfolgte mittels Karl-Fischer-Titration gemäß DIN EN 14346:2007-03 (Verfahren B).
Wassergehalt: 0,2 Gew.-%

<u>Feststoffrückstand</u>

Zur Bestimmung des Feststoffrückstandes wurden 47,996 g des frisch dispergierten Ferrofluids in einem Becherglas bei 150°C bis zur vollständigen Trocknung eingedampft und anschließend 30 Minuten im Trockenschrank bei 160°C getrocknet. Nach der Trocknung verblieben 2,665 g Feststoff.

#### Beispiel 2: Herstellung Ferrofluid F2

In einem 3 L Becherglas wurden 44,86 g Eisen(III)-chlorid Hexahydrat und 16,50 g Eisen(II)-chlorid Tetrahydrat vorgelegt und unter Rühren bei 660 U/min (KPG-Rührer (Fa. Heidolph, Modell RZR 2102 control) mit dreiblättrigem Propellerrührer (VWR, Art.-Nr.: BOHLC378-20, Schaftdurchmesser 10 mm, Schaftlänge 600 mm, Rührerdurchmesser 75 mm)) in 1900 mL VE-Wasser gelöst. Über einen Tropftrichter erfolgte die tropfenweise Zugabe von 100 mL einer 25%igen Ammoniumhydroxid-Lösung. Nach 30 Minuten Reaktionsdauer wurde der gebildete Niederschlag mit Hilfe eines NdFeB-Magneten von der überstehenden Lösung abgetrennt und dreimal mit VE-Wasser gewaschen. Die Partikel wurden mit einer Ultraschallsonde (Fa. Branson, DIGITAL Sonifier®, Modell 450, Sonde 18 mm) bei einer Amplitude von 70% und einer Ultraschalldauer von 60 Minuten (Pulsdauer: 1 s on, 0,5 s off) in 600 mL VE-Wasser dispergiert. Die Partikel wurden mit einem NdFeB-Magneten von der nach der Ultraschallbehandlung noch warmen Lösung (70°C) abgetrennt und viermal mit 400 mL N,N-Dimethylformamid gewaschen. Die gewaschenen Partikel wurden mit der Ultraschallsonde (Amplitude 70%, 30 Minuten, Pulsdauer: 1 s on, 0,5 s off) in 700 mL N,N-Dimethylformamid dispergiert. Unmittelbar vor der jeweiligen Verwendung zur Synthese der Feststoff-Kompositmaterialien wurde das Ferrofluid jeweils erneut mit der Ultraschallsonde (Amplitude 70%, 15 Minuten, Pulsdauer: 1 s on, 0,5 s off) dispergiert.

<u>Feststoffrückstand</u>

Zur Bestimmung des Feststoffrückstandes wurden 46,978 g des frisch dispergierten Ferrofluids in einem Becherglas bei 150°C bis zur vollständigen Trocknung eingedampft und anschließend 30 Minuten im Trockenschrank bei 160°C getrocknet. Nach der Trocknung verblieben 1,183 g Feststoff.

**II. Herstellung des Kompositmaterials**

Zur Herstellung der Kompositmaterialien K1 bis K47 gemäß Tabelle 5, werden je Kompositmaterial die in der Tabelle angegebene Herstellungsmethode und die angegebenen Einsatzmengen (falls nicht anders gekennzeichnet in Gramm) der Chemikalien verwendet.

**Tabelle 5: Synthese des Kompositmaterials**

| Komposit-material | Ferro-fluid | Monomer A1 | Monomer A2 | | | | | | | Tensid | | | | | | Monomer B | | | | Methode | | Reaktions-temperatur & Emulgier-Temperatur [°C] | |
|---|---|---|---|---|---|---|---|---|---|---|---|---|---|---|---|---|---|---|---|---|---|---|---|
| | | A1 | A2-1 | A2-2 | A2-3 | A2-4 | A2-5 | A2-6 | A2-7 | T-1 | T-2 | T-3 | T-4 | T-5 | T-6 | B-1 | B-2 | B-3 | B-4 | | Rühren [U/min] | | Dauer |
| K1 | F1 | 0,738 | 0,60 | - | - | - | - | 0,378 | - | 11,0 | - | - | - | - | - | 2,874 | - | - | - | M1 | 580 | 60 | 48 h |
| K2 | F1 | 0,939 | - | - | - | - | - | 0,378 | - | 11,0 | - | - | - | - | - | 2,874 | - | - | - | M1 | 580 | 60 | 48 h |
| K3 | F1 | 0,939 | - | - | - | - | - | - | 0,276 | 11,0 | - | - | - | - | - | 2,874 | - | - | - | M1 | 580 | 60 | 48 h |
| K4 | F1 | 0,939 | - | - | - | - | - | 0,378 | - | 11,0 | - | - | - | - | - | - | - | 3,668 | - | M1 | 580 | 60 | 48 h |
| K5 | F1 | 0,939 | - | - | - | - | - | 0,378 | - | 11,0 | - | - | - | - | - | - | - | - | 2,775 | M1 | 580 | 60 | 22 h |
| K6 | F1 | 0,738 | 0,60 | - | - | - | - | 0,378 | - | 11,0 | - | - | - | - | - | 2,003 | - | - | - | M1 | 580 | 60 | 48 h |
| K7 | F1 | 1,341 | 1,80 | - | - | - | - | 0,252 | - | 11,0 | - | - | - | - | - | 4,354 | - | - | - | M1 | 210 | 60 | 72 h |
| K8 | F1 | 1,341 | 1,80 | - | - | - | - | 0,252 | - | 11,0 | - | - | - | - | - | - | - | 5,557 | - | M1 | 210 | 60 | 96 h |
| K9 | F1 | 1,341 | 1,80 | - | - | - | - | 0,252 | - | 11,0 | - | - | - | - | - | - | - | - | 4,205 | M1 | 210 | 60 | 72 h |
| K10 | F1 | 1,341 | 1,80 | - | - | - | - | 0,252 | - | 11,0 | - | - | - | - | - | 4,354 | - | - | - | M1 | 210 | 60 | 72 h |
| K11 | F2 | 1,341 | 1,80 | - | - | - | - | 0,252 | - | 11,0 | - | - | - | - | - | 4,354 | - | - | - | M1 | 210 | 60 | 72 h |
| K12 | F1 | 1,341 | 1,80 | - | - | - | - | 0,252 | - | - | - | - | - | - | - | 4,354 | - | - | - | M1 | 210 | 60 | 96 h |
| K13 | F2 | 1,341 | 1,80 | - | - | - | - | 0,252 | - | - | - | - | - | - | - | 4,354 | - | - | - | M1 | 210 | 60 | 96 h |
| K14 | F1 | 2,012 | 2,00 | - | - | - | - | 0,315 | - | 11,0 | - | - | - | - | - | 3,483 | - | - | - | M1 | 210 | 60 | 96 h |
| K15 | F2 | 2,012 | 2,00 | - | - | - | - | 0,315 | - | 11,0 | - | - | - | - | - | 3,483 | - | - | - | M1 | 210 | 60 | 96 h |
| K16 | F1 | 2,012 | - | 0,451 | - | - | - | 0,315 | - | 11,0 | - | - | - | - | - | 3,483 | - | - | - | M1 | 210 | 60 | 72 h |
| K17 | F2 | 2,012 | - | 0,451 | - | - | - | 0,315 | - | 11,0 | - | - | - | - | - | 3,483 | - | - | - | M1 | 210 | 60 | 72 h |
| K18 | F1 | 2,012 | 2,00 | - | - | - | - | 0,315 | - | 11,0 | - | - | - | - | - | - | 3,483 | - | - | M1 | 210 | 60 | 96 h |
| K19 | F2 | 2,012 | 2,00 | - | - | - | - | 0,315 | - | 11,0 | - | - | - | - | - | - | 3,483 | - | - | M1 | 210 | 60 | 96 h |
| K20 | F1 | 2,012 | - | - | 12,50 | - | - | 0,315 | - | 11,0 | - | - | - | - | - | 3,483 | - | - | - | M1 | 210 | 60 | 72 h |
| K21 | F2 | 2,012 | - | - | 12,50 | - | - | 0,315 | - | 11,0 | - | - | - | - | - | 3,483 | - | - | - | M1 | 210 | 60 | 72 h |
| K22 | F1 | 2,012 | - | 0,360 | 2,50 | - | - | 0,315 | - | 11,0 | - | - | - | - | - | 3,483 | - | - | - | M1 | 210 | 60 | 96 h |

| | | | | | | | | | | | | | | | | | | | | | | | |
|---|---|---|---|---|---|---|---|---|---|---|---|---|---|---|---|---|---|---|---|---|---|---|---|
| **K23** | F2 | 2,012 | - | 0,360 | 2,50 | - | - | 0,315 | - | 11,0 | - | - | - | - | - | 3,483 | - | - | - | M1 | 210 | 60 | 96 h |
| **K24** | F1 | 2,012 | 2,00 | - | - | - | - | 0,315 | - | 20,0 | - | - | - | - | - | - | 3,483 | - | - | M1 | 210 | 60 | 96 h |
| **K25** | F1 | 2,012 | 2,00 | - | - | - | - | 0,315 | - | 30,0 | - | - | - | - | - | - | 3,483 | - | - | M1 | 210 | 60 | 96 h |
| **K26** | F2 | 2,012 | 2,00 | - | - | - | - | 0,315 | - | 20,0 | - | - | - | - | - | - | 3,483 | - | - | M1 | 210 | 60 | 72 h |
| **K27** | F2 | 2,012 | 2,00 | - | - | - | - | 0,315 | - | 30,0 | - | - | - | - | - | - | 3,483 | - | - | M1 | 210 | 60 | 72 h |
| **K28** | F1 | 2,012 | 2,00 | - | - | - | - | 0,315 | - | - | 11g | - | - | - | - | - | 3,483 | - | - | M1 | 210 | 60 | 72 h |
| **K29** | F1 | 2,012 | 2,00 | - | - | - | - | 0,315 | - | - | 30g | - | - | - | - | - | 3,483 | - | - | M1 | 210 | 60 | 72 h |
| **K30** | F1 | 2,012 | 2,00 | - | - | - | - | 0,315 | - | - | - | 11g | - | - | - | - | 3,483 | - | - | M1 | 210 | 60 | 96 h |
| **K31** | F1 | 2,012 | 2,00 | - | - | - | - | 0,315 | - | - | - | 30g | - | - | - | - | 3,483 | - | - | M1 | 210 | 60 | 96 h |
| **K32** | F1 | 2,012 | 2,00 | - | - | - | - | 0,315 | - | - | - | - | 11g | - | - | - | 3,483 | - | - | M1 | 210 | 60 | 72 h |
| **K33** | F1 | 2,012 | 2,00 | - | - | - | - | 0,315 | - | - | - | - | 30g | - | - | - | 3,483 | - | - | M1 | 210 | 60 | 72 h |
| **K34** | F1 | 2,012 | 2,00 | - | - | - | - | 0,315 | - | - | - | - | - | 11g | - | - | 3,483 | - | - | M1 | 210 | 60 | 96 h |
| **K35** | F1 | 2,012 | 2,00 | - | - | - | - | 0,315 | - | - | - | - | - | 30g | - | - | 3,483 | - | - | M1 | 210 | 60 | 96 h |
| **K36** | F1 | 2,012 | 1,00 (200) | - | - | - | - | 0,315 | - | 11,0 | - | - | - | - | - | 3,483 | - | - | - | M1 | 210 | 60 | 72 h |
| **K37** | F1 | 2,012 | 5,00 (1000) | - | - | - | - | 0,315 | - | 11,0 | - | - | - | - | - | 3,483 | - | - | - | M1 | 210 | 60 | 72 h |
| **K38** | F1 | 2,012 | - | - | - | 2,65 | - | 0,315 | - | 11,0 | - | - | - | - | - | 3,483 | - | - | - | M1 | 210 | 60 | 96 h |
| **K39** | F1 | 3,186 | - | - | - | - | - | - | - | 11,0 | - | - | - | - | - | 3,483 | - | - | - | M1 | 210 | 60 | 96 h |
| **K40** | F1 | 2,012 | 2,00 | - | - | - | - | 0,315 | - | 11,0 | - | - | - | - | - | 3,483 | - | - | - | M1 | 210 | 40 | 72 h |
| **K41** | F1 | 2,012 | 2,00 | - | - | - | - | 0,315 | - | 11,0 | - | - | - | - | - | 3,483 | - | - | - | M1 | 210 | 50 | 72 h |
| **K42** | F1 | 2,012 | 2,00 | - | - | - | - | 0,315 | - | 11,0 | - | - | - | - | - | 3,483 | - | - | - | M1 | 210 | 70 | 96 h |
| **K43** | F1 | 2,012 | 2,00 | - | - | - | - | 0,315 | - | 11,0 | - | - | - | - | - | 3,483 | - | - | - | M1 | 210 | 80 | 96 h |
| **K44** | F1 | 2,012 | 2,00 | - | - | - | - | 0,315 | - | - | - | - | - | - | 11g | 3,483 | - | - | - | M1 | 210 | 60 | 72 h |
| **K45** | F1 | 2,012 | - | - | - | - | 0,90 | 0,315 | - | 11,0 | - | - | - | - | - | - | 4,354 | - | - | M1 | 210 | 60 | 72 h |
| **K46** | F1 | 2,012 | - | - | - | - | 1,35 | 0,315 | - | 11,0 | - | - | - | - | - | - | 5,225 | - | - | M1 | 210 | 60 | 72 h |
| **K47** | F1 | 2,012 | 2,00 | - | - | - | - | 0,315 | - | 11,0 | - | - | - | - | - | - | 3,483 | - | - | M2 | 210 | 60 | 72 h |

Die Mengenangaben der eingesetzte Chemikalien erfolgt in Tabelle 5 falls nicht anders gekennzeichnet in Gramm.

**Folgende Chemikalien wurden eingesetzt:**

Eisen(III)-chlorid Hexahydrat (Fisher Chemical, > 97%)
Eisen(II)-chlorid Tetrahydrat (Sigma-Aldrich, puriss, ≥ 99,0%)
Ammoniumhydroxid-Lösung (Sigma-Aldrich, puriss, ~ 25%)
Natriumcitrat-Dihydrat (Sigma-Aldrich, ≥ 99%)
N,N-Dimethylformamid (VWR, 99,9%)
1,4-Diazabicyclo[2.2.2]octan (Sigma-Aldrich, ReagentPlus®, ≥ 99%)
Cyclohexan (Bernd Kraft, mind. 98%)
Weißöl WX32 (Addinol Lube Oil GmbH, medizinisches Weißöl)
Ethanol (Bernd Kraft, 99%, vergällt)

| | |
|---|---|
| Monomer A1: | 2,2-Bis(hydroxymethyl)propionsäure (Sigma-Aldrich, 98%) |
| Monomer A2-1: | Polyethylenglykol (Mn = 400 g/mol, Sigma-Aldrich), ansonsten mittlere Molmasse bei Mengenangabe erwähnt (200) = Mn = 200 g/mol; (1000) = Mn = 1000 g/mol (jeweils Sigma-Aldrich) |
| Monomer A2-2: | Butan-1,4-diol (Sigma-Aldrich, 99 %) |
| Monomer A2-3: | Poly[di(ethylenglycol)adipate] ($M_n$ ~ 2500 g/mol, Sigma-Aldrich) |
| Monomer A2-4: | Polycaprolactondiol ($M_n$ ~ 530 g/mol, Sigma-Aldrich) |
| Monomer A2-5: | 1,3-Dihydroxyacetondimer (Sigma-Aldrich, 97%) |
| Monomer A2-6: | 1,3,5-Trihydroxybenzol (Sigma-Aldrich, ≥ 99%) |
| Monomer A2-7: | Glycerin (Sigma-Aldrich, ≥ 99,5%) |
| Monomer B-1: | Toluol-2,4-diisocyanat (Sigma-Aldrich, ≥ 98,0%) |
| Monomer B-2: | Lupranat T 80 (Sigma-Aldrich) |
| Monomer B-3 | Isophorondiisocyanat (Sigma-Aldrich, 98%) |
| Monomer B-4 | Hexamethylendlisocyanat (Sigma-Aldrich, ≥ 98,0%) |
| Tensid T-1: | Span 80 (Merck, for synthesis) |
| Tensid T-2: | Span 65 (Sigma-Aldrich) |
| Tensid T-3: | Span 60 (Sigma-Aldrich) |
| Tensid T-4: | Span 83 (TCI) |
| Tensid T-5: | Span 85 (Sigma-Aldrich) |
| Tensid T-6: | Brij 52 ($M_n$ ~ 330 g/mol, Sigma-Aldrich) |

**Herstellungsmethoden gemäß Tabelle 5:**

Die Synthesen der partikelförmigen Feststoffkompositmaterialien wurden in einem 1L-Reaktorbehälter durchgeführt. Dieser ist zusammengesetzt aus einem Planflansch-Rundkolben (1L / NW100 mit Nut) und einem Planflanschdeckel (NW 100) mit 4 Stutzen (Mittelhals Hülse NS 29, 2x seitlich Hülse NS 29 schräg, 1x seitlich Hülse NS 14,5 senkrecht), einem O-Ring (Silicon, NW 100) sowie einem Schnellverschluß (NW 100). Als Rührer diente ein KPG-Rührer (Fa. Heidolph, Modell RZR 2102 control) mit einem dreiblättrigen Propellerrührer (VWR, Art.-Nr.: BOHLC378-20, Schaftdurchmesser 10 mm, Schaftlänge 600 mm, Rührerdurchmesser 75 mm), welcher durch die Mittelhalshülse in den Reaktorbehälter eingeführt wurde. Die Temperierung des Reaktorbehälters erfolgte durch ein Ölbad mit Heizplatte.

**Methode M1:**

50 mL des in Tabelle 5 angegebenen Ferrofluids, 5 mL N,N-Dimethylformamid, die angegebene Menge der Monomere A, 0,1 g 1,4-Diazabicyclo[2.2.2]octan und sofern vorhanden die angegebene Menge an Tensid wurden in einem 1L-Reaktorbehälter mit 395 mL Cyclohexan versetzt und 60 Minuten bei der in Tabelle 5 angegebenen Emulgiertemperatur und der in Tabelle 5 angegebenen Rührgeschwindigkeit gerührt. Unter Rühren wurde bei o.g. Temperatur die in Tabelle 5 angegebene Menge Monomer B in 5 mL Cyclohexan zugegeben. Die Mischung wurde über die angegebene Reaktionsdauer bei angegebener Reaktionstemperatur gerührt. Die Partikel wurden mit einem NdFeB-Magneten abgetrennt und zweimal mit Ethanol, zweimal mit Aceton und fünfmal mit Wasser gewaschen.

**Methode M2:**

50 mL des in Tabelle 5 angegebenen Ferrofluids, 5 mL N,N-Dimethylformamid, die angegebene Menge der Monomere A, 0,1 g 1,4-Diazabicyclo[2.2.2]octan und sofern vorhanden die angegebene Menge an Tensid wurden in einem 1L-Reaktorbehälter mit 395 mL Weißöl WX32 versetzt und 60 Minuten bei der in Tabelle 5 angegebenen Emulgiertemperatur und der in Tabelle 5 angegebenen Rührgeschwindigkeit gerührt. Unter Rühren wurde bei o.g. Temperatur die in Tabelle 5 angegebene Menge Monomer B in 5 mL Weißöl WX32 zugegeben. Die Mischung wurde über die angegebene Reaktionsdauer bei der angegebenen Reaktionstemperatur gerührt. Die Partikel wurden mit einem NdFeB-Magneten abgetrennt und zweimal mit Ethanol, zweimal mit Aceton und fünfmal mit Wasser gewaschen.

**III. Analyse der Kompositmaterialien**

Von den in Punkt II hergestellten Kompositmaterialien wurden der mittlere Partikeldurchmesser und der Mengengehalt an eingebetteten magnetischen Nanopartikeln bestimmt. Die Werte wurden in Tabelle 6 zusammengestellt.

**Durchführung der Bestimmung des mittleren Partikeldurchmessers:**

Die Partikelgrößen und Partikelgrößenverteilungen wurden mit einem Beckman Multisizer™ 3 Coulter Counter® (Software Beckman Coulter Multisizer™ 3, ©1990-2008, Version 3.53, 15.10.2008, Beckman Coulter GmbH, Krefeld) bestimmt und ausgewertet. Dazu wurde jeweils eine Spatelspitze der zu messenden Substanz mit 1 mL VE-Wasser versetzt und die Substanz anschließend dispergiert (2 Minuten Vortex-Mischer bei 2400 U/min (VELP Scientifica ZX4 Advanced IR Vortex Mixer, VELP Scientifica Srl, Italien) und 2 Minuten Ultraschallbad (VWR, USC300T, 80 W)). Von dieser Suspension wurden 200 µL abgenommen und mit 5 mL 10 %iger-Triton-Lösung (Triton X-100 reinst, Carl Roth GmbH & Co. KG) versetzt und homogenisiert (1 Minute Vortex-Mischer bei 2400 U/min (VELP Scientifica ZX4 Advanced IR Vortex Mixer, VELP Scientifica Srl, Italien)). Von diesen vorbereiteten Lösungen wurde jeweils 1 mL in den Coulter zu 150 mL der isotonischen Lösung gegeben (Coulter Isoton II Diluent, Art.-Nr. 8546719, Beckman Coulter GmbH, Krefeld). Die Messungen wurden entsprechend der Anweisungen des Herstellers durchgeführt. Bei den angegebenen mittleren Partikeldurchmessern der jeweiligen Reaktionsbeispiele handelt es sich um die von der Gerätesoftware angegebenen volumengemittelten Partikeldurchmesser. Gemäß dem Coulter Prinzip wird das Partikelvolumen in einen Partikeldurchmesser konvertiert. Dieser Partikeldurchmesser entspricht dem Äquivaientdurchmesser, also dem Durchmesser einer Sphäre deren Volumen dem Partikelvolumen entspricht. Das Coulter Messprinzip beruht auf der Nutzung einer. Kapillare mit einer Apertur. Jede Apertur ist geeignet Partikel zu erfassen, welche im Bereich von 2 bis 60 % des Aperturdurchmessers liegen. Typische Äperturdurchmesser liegen im Bereich von 20 bis 2000 µm. Eine 30 µm Apertur ist somit beispielsweise für die Erfassung von Partikeln im Bereich von 0,6 bis 18 µm geeignet. Für die Bestimmung der Partikelgrößen der verschiedenen Reaktionsbeispiele war daher die Nutzung unterschiedlicher Aperturen notwendig. Die Angaben der mittleren Partikeldurchmesser sind in Tabelle 6 entsprechend hinsichtlich der für die jeweilige Messung eingesetzten Apertur gekennzeichnet (Apertur: 20 µm (*), 30 µm (**), 70 µm (***), 140 µm (****), 280 µm (*****), 400 µm (******)).

**Bestimmung des Eisenoxidgehaltes**

Diese Bestimmung dient als Maß für den Anteil an magnetischen Nanopartikeln im Kompositmaterial. Zur Bestimmung des Feststoffgehaltes wurde jeweils eine Doppelbestimmung mit etwa je 500 mg Substanz durchgeführt. Dazu wurden die Proben 2 Stunden bei 120 °C vorgetrocknet und wieder auf Raumtemperatur abgekühlt. Die Proben wurden einem kontrollierten Temperaturprogramm (570 Minuten Temperaturrampe von 0 °C auf 950 °C, 120 Minuten 950 °C) ausgesetzt, wieder auf Raumtemperatur abgekühlt und es wurde die Gewichtsdifferenz von vor und nach der Temperaturbehandlung bestimmt. Die angegebenen Werte sind die Mittelwerte aus den Ergebnissen beider Bestimmungen.

**Transmissionselektronenmikroskopische Abbildungen**

Transmissionselektronenmikroskopische Abbildungen (Fig.1 & Fig.2) wurden mit einem CM300 UT FEG der Fa. Philips (heute FEI) mit 297 kV Beschleunigungsspannung und einer 2k x 2k MSC-CCD-Kamera durchgeführt. Für die Untersuchungen wurde jeweils eine Spatelspitze der zu untersuchenden Substanz in 5 mL Ethanol abs. für 2 Minuten in einem Ultraschallbad (VWR, USC300T, 80 W) dispergiert, ein Tropfen der Suspension auf ein Quantifoil TEM-Netz (200-Mesh Kupfergrid mit Kohlenstofflochfilm, R1.2/1.3) aufgebracht und das Netz anschließend 10 Minuten an Luft getrocknet.

Tabelle 6: Analyseergebnisse des Kompositmaterials (- = nicht bestimmt)

| Kompositmaterial | mittlere Partikelgröße [μm] | Eisenoxidgehalt [Gew.-%] |
|---|---|---|
| K1 | 2,17 ** | - |
| K2 | 1,54 ** | - |
| K3 | 1,73 ** (siehe Fig.1) | - |
| K4 | 0,87 ** | - |
| K5 | 28,01 ****** | - |
| K6 | 1,00 ** (siehe Fig.2) | - |
| K7 | 1,26 * | 66,3 |
| K8 | 1,34 ** | - |
| K9 | 30,03 ***** | - |
| K10 | 1,17 * | 70,4 |
| K11 | 1,02 ** | |
| K12 | 1,28 * | 76,1 |
| K13 | 4,43 *** | - |
| K14 | 1,03 ** | 87,5 |
| K15 | 1,06 ** | - |
| K16 | 1,00 ** | - |
| K17 | 2,47 * | - |
| K18 | 0,93 * | 85,4 |
| K19 | 1,42 ** | - |
| K20 | 2,80 ** | - |
| K21 | 2,51 *** | - |
| K22 | 0,87 * | 86,8 |
| K23 | 1,22 ** | - |
| K24 | 0,98 ** | - |
| K25 | 0,78 * | 89,5 |
| K26 | 1,32 ** | - |
| K27 | 1,54 ** | - |
| K28 | 1,18 ** | - |
| K29 | 1,19 ** | - |
| K30 | 1,92 *** | - |
| K31 | 3,50 *** | - |
| K32 | 1,06 ** | - |
| K33 | 13,19 *** | - |
| K34 | 1,24 ** | - |
| K35 | 1,18 ** | - |
| K36 | 0,98 ** | 88,0 |
| K37 | 1,07 ** | - |

(fortgesetzt)

| Kompositmaterial | mittlere Partikelgröße [μm] | Eisenoxidgehalt [Gew.-%] |
|---|---|---|
| **K38** | 1,00 ** | - |
| **K39** | 0,97 ** | 98,7 |
| **K40** | 0,95 ** | - |
| **K41** | 0,96 ** | - |
| **K42** | 1,00 ** | - |
| **K43** | 0,89 ** | - |
| **K44** | 0,95 ** | - |
| **K45** | 0,96 ** | - |
| **K46** | 1,10 * | - |
| **K47** | 0,92 ** | - |

**IV. Durchführung der Aufreinigung von Nukleinsäuren**

**Aufreinigung von Nukleinsäuren mit Puffersystem A:**

**Probenvorbereitung magnetische Beads:**

Von den gewaschenen magnetischen Beads aus den Reaktionsbeispielen wurden ausreichende Substanzmengen in ein 2 mL SafeSeal micro tube Gefäß (Fa. Sarstedt, Nümbrecht) gegeben, mit einer Laborzentrifuge (Eppendorf Zentrifuge 5415 C, Fa. Eppendorf, Wesseling) 2 Minuten bei 11000 U/min zentrifugiert und überstehende Flüssigkeitsreste abpipettiert. Anschließend wurden jeweils 140 mg der magnetischen Beads in 1,5 mL Eppendorf-Reaktionsgefäßen vorgelegt, mit 1 mL Reinstwasser (Millipore Direct-Q® 5 UV-R, Reinstwasser Typ 1, 0,8 μS/cm@25°C) versetzt und dispergiert (2 Minuten Vortex-Mischer bei 2400 U/min (VELP Scientifica ZX4 Advanced IR Vortex Mixer, VELP Scientifica Srl, Italien) und 2 Minuten Ultraschallbad (VWR, USC300T, 80W)). Als Referenz zu den erfindungsgemäßen Beads wurden kommerzlell erhältliche Beads verwendet. Diese Beads wurden dem NucleoMag® 96 Plant Kit (MACHEREY-NAGEL GmbH & Co. KG, Düren, Ref.-Nr. 744400.1, Kit-LOT: 1604/002) entnommen. Es handelte sich um NucleoMag® C-Beads mit einem mittleren Partikeldurchmesser von 2,29 μm. Die NucleoMag® C-Bead-Lösungen wurden analog der beschriebenen Vorgehensweise für die magnetischen Beads aus den Reaktionsbeispielen hergestellt, so dass die Konzentration der C-Beads und der magnetischen Beads aus den Reaktionsbeispielen in den eingesetzten Lösungen jeweils 140 mg/mL betrug.

**Herstellung Referenzlysat:**

Die im Folgenden genannten Puffer stammen aus dem NucleoMag® 96 Plant Kit (MACHEREY-NAGEL GmbH & Co. KG, Düren, Ref.-Nr. 744400.1) und wurden gemäß Herstellerangaben verwendet. Zur Isolierung von Nukleinsäuren wurde Blattmaterial von Weizenkeimlingen für ein Referenzlysat als Probenmaterial eingesetzt. Dazu wurden entsprechende Mengen des Blattmaterials unter flüssigem Stickstoff fein gemörsert. Das gemörserte Material wurde in ein 50 mL Reaktionsgefäß übergeführt und mit dem entsprechenden Volumen des Puffers MC1 (MACHEREY-NAGEL GmbH & Co. KG, Düren, Ref.-Nr. 744856.1) versetzt, so dass eine Konzentration von 40 mg Blattmaterial pro 0,4 mL Pufferlösung vorlag. Zu diesem Ansatz wurden je 40 mg Blattmaterial 10 μL einer RNase A Stammlösung (c(RNase) = 12 mg/mL, MACHEREY-NAGEL GmbH & Co. KG, Düren, Ref.-Nr. 744400.1) zugegeben. Die Stammlösung wurde durch Zugabe von 1,25 mL Reinstwasser zu 15 mg lyophilierter RNase hergestellt. Anschließend wurde der Ansatz für 15 Minuten bei 65 °C lysiert. Noch vorhandene feste Bestandteile des Lysatansatzes wurden durch Zentrifugation mit einer Tischzentrifuge (Hettich Rotina 420R, 10 Minuten bei 4500 x g) abgetrennt.

**Isolierung von Nukleinsäure mit Puffersystem A:**

Die Isolierung der Nukleinsäuren wurde automatisiert auf einem KingFisher™ Flex™ (Thermo Scientific Produkt-Nr. 5400630, Gerätesoftware Version 1.00.17, PC-Software Bindlt, Version 3.3) in KingFisher™ Deep-well Blocks (KingFisher™ Accessory Kit B, MACHEREY-NAGEL GmbH & Co. KG, Düren, Ref.-Nr. 744951) durchgeführt. Zur Elution

wurden KingFisher™ Elution Plates verwendet. Weitere Details und Methoden können dem KingFisher™ Flex™ Manual und dem "User Guide for Automated purification of DNA from Plant leaves and seeds with KingFisher 96/ KingFisher mL Instrument and MACHEREY-NAGEL NucleoMag 96 Plant kit" entnommen werden. Für alle Reaktionsbeispiele wurden je magnetischer Bead-Sorte Vierfach-Bestimmungen durchgeführt.

Es wurden jeweils 400 µL des jeweiligen Lysats mit je 30 µL Bead-Lösung und 400 µL Puffer MC2 (MACHEREY-NAGEL GmbH & Co. KG, Düren, Ref.-Nr. 744857.1) versetzt. Die Mischung wurde 5 Minuten bei Raumtemperatur durch Auf- und Abfahren der Kammspitzen (96 DW tip comb, KingFisher™ Accessory Kit B, MACHEREY-NAGEL GmbH & Co. KG, Düren, Ref.-Nr. 744951, Mischgeschwindigkeit "half mix") auf dem KingFisher™ Flex™ durchmischt und die Beads anschließend durch Einfahren der Magnetspitzen in die Kammspitzen für 2 Minuten magnetisch separiert. Die an den Kammspitzen angelagerten magnetischen Beads mit der daran angebundenen Nukleinsäure wurden in einen weiteren KingFisher™ Deep-well Block überführt, dessen Kavitäten mit je 600 µL Waschpuffer 1 (MC3, MACHEREY-NAGEL GmbH & Co. KG, Düren, Ref.-Nr. 744858.1) gefüllt waren. Durch Ausfahren der Magnetspitzen wurden die magnetischen Beads freigesetzt und mittels Auf- und Abfahrens der Kammspitzen für 1 Minute bei Raumtemperatur durchmischt (Mischgeschwindigkeit "half mix"). Anschließend wurden die magnetischen Beads mittels Einfahren der Magnetspitzen in die Kammspitzen 2 Minuten lang magnetisch separiert und in einen weiteren KingFisher™ Deep-well Block überführt, dessen Kavitäten mit je 600 µL Waschpuffer 2 (MC4, MACHEREY-NAGEL GmbH & Co. KG, Düren, Ref.-Nr. 744859.1) gefüllt waren. Die magnetischen Beads wurden unter den für Waschpuffer 1 beschriebenen Bedingungen freigesetzt, suspendiert, wieder separiert und in einen weiteren Deep-well Block überführt, dessen Kavitäten mit je 600 µL Waschpuffer 3 (80 %iger Ethanol) gefüllt waren. Die Dispergierung der magnetischen Beads erfolgte wiederum durch Ausfahren der Magnetspitzen und Auf- und Abfahren der Kammspitzen (Mischgeschwindigkeit "fast") für 1 Minute bei Raumtemperatur. Die Magnetspitzen wurden wieder in die Kammspitzen eingefahren, die magnetischen Beads 2 Minuten lang magnetisch separiert und in einen weiteren KingFisher™ Deep-well Block übergeführt, dessen Kavitäten mit je 600 µL Waschpuffer 4 (MC5, MACHEREY-NAGEL GmbH & Co. KG, Düren, Ref.-Nr. 744860.1) befüllt waren. Die Beads wurden bei eingefahrenen Magnetspitzen ohne Resuspendierungsschritt für 1 Minute bei Raumtemperatur inkubiert und anschließend in eine KingFisher™ Elution Plate überführt, deren Kavitäten mit je 100 µL Elutionspuffer (MC6, MACHEREY-NAGEL GmbH & Co. KG, Düren, Ref.-Nr. 744861.1) befüllt waren. Die Beads wurden durch Ausfahren der Magnetspitzen und anschließendes Auf- und Abfahren der Kammspitzen (15 s "fast", 2 min 15 s "slow") bei 55 °C resuspendiert. Durch Wiedereinfahren der Magnetspitzen wurden die Beads für 5 Minuten magnetisch separiert und durch Ausfahren der Kammspitzen aus den Eluaten entfernt. Die KingFisher™ Elution Plate wurde aus dem KingFisher™ Flex™ entnommen und zur Abtrennung von womöglich noch vorhandenen restlichen Beads auf einen NucleoMag® Separator (NucleoMag® SEP, MACHEREY-NAGEL GmbH & Co. KG, Düren, Ref.-Nr. 744900) überführt. Die jeweiligen Eluate wurden anschließend abgenommen und wie weiter beschrieben verwendet.

**Analyse der Eluate:**

Die Eluate wurden sowohl photometrisch mit einem Plattenphotometer (BioTek Synergy HT, BioTek Instruments, Inc., Winooski, USA) und entsprechend kompatiblen UV-Messplatten (UV-Star® Microplate 96 well, Greiner Bio-One GmbH, Kat.-Nr. 655801) als auch mittels Agarose-Gelelektrophorese analysiert. Als Größenstandard der Agarose-Gelelektrophorese wurde der Lambda DNA/HindIII Marker 2 (Thermo Fisher Scientific, Kat.-Nr. SM0101) verwendet. Je Eluat der Reaktionsbeispiele wurden 15 µL mit 3 µL Probenpuffer (6X DNA Loading Dye aus obigem Thermo Fisher Scientific Kit) versetzt und in einem 0,7 %igen Agarosegel elektrophoretisch bei 90 V für 30 Minuten aufgetrennt. Die Nukleinsäuren wurden mittels Ethidiumbromid unter UV-Licht und der INTAS Photodokumentation mit der INTAS GDS Software detektiert.

Zur Überprüfung der Einsetzbarkeit der isolierten Nukleinsäuren für Folgeanwendungen wurden Amplifikationen mittels TaqMan-PCR auf dem Applied Biosystems® HID 7500 Real Time PCR System (mit HID Real-Time PCR Analysis Software v1.1, ©2010 Life Technologies Corporation) durchgeführt. Dazu wurden 2 µL der Eluate mit 18 µL eines PCR-Mastermixes (SensiFast™ Probe LoRox Kit, Bioline USA Inc., Taunton, Kat.-Nr. BIO-84002, mit Primer A (Sequenz: 5'-CAA GCA GCA TGA AGA TCA AGG T-3', Konzentration 10 pmol/µL), Primer B (Sequenz: 5'-CAC ATC TGT TGG AAA GTG CTG AG-3', Konzentration 10 pmol/µL), Sonde ((FAM)-CCT CCA ATC CAG ACA CTG TAC TTY CTC TC-(TAMRA), Konzentration 10 pmol/µL mit FAM = 6-Carboxyfluorescein und TAMRA = Tetramethylrhodamin)) in MicroAMP® Optical 96-Well Reaction Platten (Thermo Fisher Scientific, Kat.-Nr. N8010560) vermischt und mit MicroAMP® Optical Adhesive Film (Thermo Fisher Scientific, Kat.-Nr. 4360954) abgedeckt. Die PCR erfolgte mit folgenden Geräteeinstellungen: 95 °C 5 Minuten, 40x Zyklen mit jeweils 10 Sekunden 95 °C und 1 Minute 60 °C.

**Isolierung von Nukleinsäure mit Puffersystem B:**

**Probenvorbereitung magnetische Beads:**

5 der magnetischen Beads aus den Reaktionsbeispielen (K3, K8, K34, K46 und K47) wurden zur Isolierung von Nukleinsäuren in Kombination mit einem anderen Puffersystem eingesetzt. Dazu wurden die magnetischen Beads analog den bereits beschriebenen Vorbereitungsschritten in einer wässrigen Lösung dispergiert bereitgestellt (140 mg/mL). Als Referenz wurden NucleoMag® C-Beads aus dem NucleoMag® 96 Plant Kit (MACHEREY-NAGEL GmbH & Co. KG, Düren, Ref.-Nr, 744400.1, Kit-LOT: 1604/002) mit einem mittleren Partikeldurchmesser von 2,29 μm sowie M-PVA C21 Beads (PerkinElmer chemagen Technologie GmbH, Baesweiler, Prod.-Nr. CMG-206, LOT-Nr. C21-0108015) mit einem Partikeldurchmesser im Bereich von 0,5 - 1,25 μm (beinhaltet >95,0% aller Partikel) eingesetzt. Die NucleoMag® C-Bead-Lösungen und die M-PVA C21 Bead-Lösungen wurden ebenfalls analog der Reaktionsbeispiele vorbereitet (140 mg/mL).

**Herstellung Referenzlysat:**

Zur Isolierung von Nukleinsäuren wurde Blattmaterial von Weizenkeimlingen für ein Referenzlysat als Probenmaterial eingesetzt. Dazu wurden entsprechende Mengen des Blattmaterials unter flüssigem Stickstoff fein gemörsert. 2,8 g des Blattmaterials wurden mit 35 mL Lysepuffer (200 mM Tris-HCl, 250 mM NaCl, 25 mM EDTA, 0,5% SDS, pH = 8) und 700 μL RNase A Stammlösung (c(RNase) = 12 mg/mL, MACHEREY-NAGEL GmbH & Co. KG, Düren, Ref.-Nr. 744400.1) versetzt. Die Stammlösung wurde durch Zugabe von 2,5 mL Reinstwasser zu 30 mg lyophilierter RNase hergestellt. Anschließend wurde der Ansatz für 30 Minuten bei 56 °C lysiert. Noch vorhandene feste Bestandteile des Lysatansatzes wurden durch Zentrifugation mit einer Tischzentrifuge (Hettich Rotina 420R, 10 Minuten bei 4500 x g) abgetrennt.

**Isolierung von Nukleinsäure mit Puffersystem B:**

Die Isolierung der Nukleinsäuren wurde analog der vorangegangenen Beispiele zur Isolierung von Nukleinsäure automatisiert auf einem KingFisher™ Flex™ durchgeführt. Für die ausgewählten Reaktionsbeispiele wurden je Bead-Sorte vierfach Bestimmungen durchgeführt.

Es wurden jeweils 400 μL des Lysats mit je 30 μL Bead-Lösung und 400 μL Bindungspuffer (Isopropanol) versetzt. Die Mischung wurde 5 Minuten bei Raumtemperatur durch Auf- und Abfahren der Kammspitzen (96 DW tip comb, KingFisher™ Accessory Kit B, MACHEREY-NAGEL GmbH & Co. KG, Düren, Ref.-Nr. 744951, Mischgeschwindigkeit "half mix") auf dem KingFisher™ Flex™ durchmischt und die Beads anschließend durch Einfahren der Magnetspitzen in die Kammspitzen für 2 Minuten magnetisch separiert.

Die an den Kammspitzen angelagerten magnetischen Beads mit der daran angebundenen Nukleinsäure wurden in einen weiteren KingFisher™ Deep-well Block überführt, dessen Kavitäten mit je 600 μL Waschpuffer 1 (2,2 M Guanidinhydrochlorid, 70% Ethanol, 10 mM Tris, pH = 6,5) gefüllt waren. Durch Ausfahren der Magnetspitzen wurden die magnetischen Beads freigesetzt und mittels Auf- und Abfahrens der Kammspitzen für 1 Minute bei Raumtemperatur durchmischt (Mischgeschwindigkeit "half mix").

Anschließend wurden die magnetischen Beads mittels Einfahren der Magnetspitzen in die Kammspitzen 2 Minuten lang magnetisch separiert und in einen weiteren KingFisher™ Deep-well Block überführt, dessen Kavitäten mit je 600 μL Waschpuffer 2 (80%iger Ethanol) gefüllt waren. Die magnetischen Beads wurden unter den für Waschpuffer 1 beschriebenen Bedingungen freigesetzt, suspendiert, wieder separiert und in einen weiteren Deep-well Block überführt, dessen Kavitäten mit je 600 μL Waschpuffer 3 (80 % iger Ethanol) gefüllt waren. Die Dispergierung der magnetischen Beads erfolgte wiederum durch Ausfahren der Magnetspitzen und Auf- und Abfahren der Kammspitzen (Mischgeschwindigkeit "fast") für 1 Minute bei Raumtemperatur. Die Magnetspitzen wurden wieder in die Kammspitzen eingefahren, die magnetischen Beads 2 Minuten lang magnetisch separiert und in eine KingFisher™ Elution Plate überführt, deren Kavitäten mit je 125 μL Elutionspuffer (5 mM Tris-HCl, pH = 8,5) befüllt waren. Die Beads wurden durch Ausfahren der Magnetspitzen und anschließendes Auf- und Abfahren der Kammspitzen (15 s "fast", 2 min 15 s "slow") bei 55 °C resuspendiert. Durch Wiedereinfahren der Magnetspitzen wurden die Beads für 5 Minuten magnetisch separiert und durch Ausfahren der Kammspitzen aus den Eluaten entfernt.

Die KingFisher™ Elution Plate wurde aus dem KingFisher™ Flex™ entnommen und zur Abtrennung von womöglich noch vorhandenen restlichen Beads auf einen NucleoMag® Separator (NucleoMag® SEP, MACHEREY-NAGEL GmbH & Co. KG, Düren, Ref.-Nr. 744900) überführt. Die jeweiligen Eluate wurden anschließend abgenommen und wie weiter beschrieben verwendet.

**Analyse der Eluate:**

Die Eluate wurden analog der vorangegangenen Reaktionsbeispiele photometrisch mit einem Plattenphotometer (BioTek Synergy HT, BioTek Instruments, Inc., Winooski, USA) und entsprechend kompatiblen UV-Messplatten (UV-Star® Microplate 96 well, Greiner Bio-One GmbH, Kat.-Nr. 655801) als auch mittels Agarose-Gelelektrophorese analysiert. Als Größenstandard der Agarose-Gelelektrophorese wurde der Lambda DNA/HindIII Marker 2 (Thermo Fisher Scientific, Kat-Nr. SM0101) verwendet. Je Eluat der Reaktionsbeispiele wurden 15 μL mit 3 μL Probenpuffer (6X DNA Loading Dye aus obigem Thermo Fisher Scientific Kit) versetzt und in einem 0,7 %igen Agarosegel elektrophoretisch bei 90 V für 30 Minuten aufgetrennt. Die Nukleinsäuren wurden mittels Ethidiumbromid unter UV-Licht und der INTAS Photodokumentation mit der INTAS GDS Software detektiert.

**3. Ergebnisse:**

Für die Einsetzbarkeit des magnetischen Kompositmaterials spielen seine Anwendungseigenschaften während der einzelnen Verfahrensschritte eine entscheidende Rolle. Als diesbezügliche Eigenschaften sind vor allem eine schnelle und homogene Dispergierbarkeit der magnetischen Beads in den unterschiedlichen Medien, ein rückstandsfreies Ablaufverhalten der verschiedenen magnetische Beads enthaltenden Lösungen an Pipetten- und Gefäßwänden, eine schnelle magnetische Abtrennbarkeit sowie ein möglichst langsames Sedimentationsverhalten der magnetischen Beads in den jeweiligen Lösungen zu nennen.

Die erfindungsgemäßen magnetischen Beads konnten zur Isolierung von Nukleinsäure in dem beschriebenen automatisierten Verfahren eingesetzt werden und weisen somit diese Eigenschaften auf. Die photometrische Untersuchung der Eluate auf den Nukleinsäure-Gehalt hin ergibt für nahezu alle untersuchten Proben eine mit der Referenz vergleichbare Anbindungsmenge an Nukleinsäuren. Die durchgeführten gelelektrophoretischen Auftrennungen der aus Weizen gewonnenen Eluate zeigen die mit den Beads aus den jeweiligen Reaktionsbeispielen isolierten Nukleinsäuren (Fig. 3, 4 und 5). Unterschiede in der Qualität der aufgereinigten Nukleinsäuren sind nicht zu erkennen, es handelt sich in allen Fällen um nicht-degradierte, hochmolekulare Nukleinsäuren. Die Mengen an isolierter Nukleinsäure liegen dabei ersichtlicherweise in vergleichbarem Rahmen zur Menge an mit den kommerziellen Beads isolierter Nukleinsäure.

Die isolierten Nukleinsäuren konnten zudem alle in der Folgeanwendung PCR (PolymeraseKettenreaktion) eingesetzt werden. Die Ergebnisse der PCR erlauben sowohl Rückschlüsse auf die anfangs vorhandene Menge an Nukleinsäure als auch auf deren Reinheit hinsichtlich inhibitorisch wirkender Verunreinigungen. In der PCR beschreibt der sog. Ct-Wert die Anzahl der benötigten Zyklen, bis das Fluoreszenzsignal einen Schwellenwert erreicht und damit den Beginn der exponentiellen Vervielfältigungsphase anzeigt. Bei einer geringen vorhandenen Menge an isolierter Nukleinsäure zu Beginn der PCR ergibt sich ein hoher Ct-Wert und bei einer großen Menge an isolierter Nukleinsäure ein niedriger Ct-Wert. Dies gilt jedoch nur bei nahezu identischen Reaktionsbedingungen (z.B. Art der Nukleinsäure, eingesetzte Primer, eingesetzte Polymerase) und wenn keine inhibitorischen Effekte vorhanden sind. Inhibitorische Effekte bedeuten eine Störung der Vervielfältigung in Form einer Verzögerung der Vervielfältigungsreaktion und führen dadurch auch bei Vorliegen einer großen Menge an isolierter Nukleinsäure zu erhöhten Ct-Werten. Inhibitorische Komponenten können z.B. aus den Beads austretende Nanopartikel sein.

Zur besseren Vergleichbarkeit der Ergebnisse wird der ΔCt-Wert herangezogen. Dieser ergibt sich aus der Differenz des Ct-Wertes der jeweiligen Probe und des Ct-Wertes der Referenzprobe ($\Delta Ct = Ct_{Probe} - Ct_{Referenz}$). Für die PCR wurden jeweils alle 4 Eluate eingesetzt, welche mit den erfindungsgemäßen Beads aus den Reaktionsbeispielen bzw. den Referenzbeads durch das automatisierte Isolierungsverfahren erhalten wurden. Aus den so bestimmten 4 Ct-Werten pro Bead-Sorte wurde jeweils der Mittelwert ($Ct_m$) gebildet. Aus diesen Mittelwerten wurde der $\Delta Ct_m$-Wert berechnet ($\Delta Ct_m = Ct_{m,Probe} - Ct_{m,Referenz}$) und für einige Reaktionsbeispiele in der folgenden Tabelle 7 zusammengefasst:

**Tabelle 7: Mittelwerte der $\Delta Ct_m$,-Werte**

| Reaktionsbeispiel | $\Delta Ct_m$ |
|---|---|
| K2 | -0,01 |
| K9 | 0,07 |
| K10 | 0,10 |
| K11 | 0,29 |
| K12 | 0,17 |
| K13 | -0,10 |
| K14 | 0,40 |
| K15 | 0,46 |
| K21 | 0,27 |

(fortgesetzt)

| Reaktionsbeispiel | $\Delta Ct_m$ |
|---|---|
| K22 | 0,48 |
| K23 | 0,41 |
| K29 | 0,46 |
| K30 | 0,42 |
| K32 | 0,38 |
| K34 | 0,10 |
| K35 | 0,30 |
| K36 | 0,12 |
| K37 | 0,48 |
| K39 | 1,39 |
| K42 | 0,20 |
| K43 | 0,17 |
| K44 | 0,38 |
| K45 | -0,36 |
| K46 | -0,46 |

Die gezeigten Werte liegen dabei überwiegend in einem Bereich von -0,5 bis +0,5 $\Delta Ct_m$. Diese Unterschiede resultieren aus geringfügig höheren bzw. niedrigeren Mengen an vorliegender isolierter Nukleinsäure zu Beginn der Amplifikationsreaktion. Unter Berücksichtigung der gezeigten Gelbilder und den darin ersichtlichen vergleichbaren Mengen an vorhandener, mit den erfindungsgemäßen Beads isolierter Nukleinsäure auf der einen Seite und der mit den Referenzbeads isolierten Nukleinsäure auf der anderen Seite, zeigt sich somit die Einsetzbarkeit der erfindungsgemäßen Beads in der PCR ohne auftretende inhibitorische Effekte.

Bei den in Tabelle 7 gezeigten Werten weist K39 einen deutlich erhöhten $\Delta Ct_m$-Wert auf. Da die Menge an isolierter Nukleinsäure gemäß Fig. 5 mit der mit den Referenzbeads isolierten Menge vergleichbar ist, liegt vermutlich eine inhibitorische Störung vor. Ein Grund dafür können austretende Nanopartikel sein. Da K39 nur mit Monomer A1 und ohne Monomer A2 bzw. A2a und/oder A2b synthetisiert wurde, unterstreicht dies die bereits erwähnte Bedeutung dieser Monomere u. A. zur Einstellung der Einbettungs- und Einkapselungseffizienz.

In Fig. 6 sind die gelelektrophoretischen Auftrennungen der Eluate gezeigt, welche unter Nutzung von Puffersystem B gewonnen wurden. Auch mit diesem Puffersystem ist die Einsetzbarkeit der getesteten erfindungsgemäßen Beads zur Isolierung von Nukleinsäure gegeben. Unterschiede in der Qualität der mit den Beads aus den Reaktionsbeispielen und den Referenzbeads isolierten und aufgereinigten Nukleinsäuren sind nicht zu erkennen, es handelt sich in allen Fällen um nicht-degradierte, hochmolekulare Nukleinsäuren.
Die Mengen an isolierter Nukleinsäure sind dabei bei K46 etwas größer im Vergleich zu den mit den NucleoMag® C-Beads (Ref) erreichten Mengen, bzw. liegen bei K3, K8, K34 und K47 zwischen den mit den NucleoMag® C-Beads und mit den M-PVA C21 Beads (Ref 1 $\mu$m) erzielten Anbindungsmengen. Dies zeigt, dass sich die Einsetzbarkeit der erfindungsgemäßen Beads nicht auf ein spezielles Puffersystem beschränkt und mit kommerziell erhältlichen Produkten vergleichbar ist.

## Patentansprüche

1. Verfahren zur Herstellung eines partikelförmigen Feststoff-Kompositmaterials zur Nukleinsäureaufreinigung, enthaltend magnetische Nanopartikel eingebettet in eine Trägermatrix auf Basis mindestens eines durch Polyaddition erhältlichen Polymers, **dadurch gekennzeichnet, dass** das Verfahren zumindest folgende Schritte umfasst

   a) Bereitstellen eines magnetischen Fluids in Form einer Suspension, enthaltend magnetische Nanopartikel und eine flüssige kontinuierliche Phase, enthaltend mindestens eine polare organische Flüssigkeit und bezogen auf das Gewicht des Fluids weniger als 5 Gew.-%, bevorzugt weniger als 2 Gew.-%, Wasser,
   b) Mischen des magnetischen Fluids mit mindestens einem, Isocyanat-reaktiven-Monomer A1, ausgewählt aus Verbindungen, enthaltend

      - mindestens zwei funktionelle Gruppen, die jeweils mindestens ein Zerewitinoffreaktives Wasserstoffatom tragen und

- zusätzlich zu diesen mindestens zwei funktionellen Gruppen mindestens einen anionischen oder potentiell anionischen Rest, bevorzugt ausgewählt aus Carboxylatgruppe, Sulfonatgruppe, oder Kombinationen daraus,

c) bevorzugt Zugabe mindestens eines Tensids,
d) Emulgieren der Mischung der vorangehenden Schritte in einer flüssigen kontinuierlichen Phase, enthaltend mindestens eine unpolare, organische Flüssigkeit,
e) Zugabe mindestens eines Polyisocyanat-Monomers B,
f) nach Ablauf der Reaktionszeit, Abtrennen des gebildeten Kompositmaterials und gegebenenfalls Waschen,

mit der Maßgabe, dass der cLogP-Wert (25°C) der polaren organischen Flüssigkeit kleiner als der cLogP-Wert (25°C) der unpolaren, organischen Flüssigkeit ist und die flüssige, kontinuierliche Phase aus Schritt d) bezogen auf ihr Gewicht weniger als 5 Gew.-%, bevorzugt weniger als 2 Gew.-%, Wasser enthält.

2. Verfahren nach Anspruch 1, **dadurch gekennzeichnet, dass** besagte polare, organische Flüssigkeit einen cLogP-Wert < 1,5 (25°C), insbesondere einen cLogP-Wert < 1,0 (25°C), und besagte unpolare, organische Flüssigkeit einen cLogP-Wert > 2,0 (25°C), insbesondere einen cLogP-Wert > 2,5 (25°C) aufweist.

3. Verfahren nach einem der vorangehenden Ansprüche, **dadurch gekennzeichnet, dass** die magnetischen Nanopartikel ausgewählt werden aus ferromagnetischen Nanopartikeln, ferrimagnetischen Nanopartikeln, oder deren Mischung, bevorzugt aus superparamagnetischen Nanopartikeln.

4. Verfahren nach einem der vorangehenden Ansprüche, **dadurch gekennzeichnet, dass** die magnetischen Nanopartikel Eisenoxid umfassen.

5. Verfahren nach einem der vorangehenden Ansprüche, **dadurch gekennzeichnet, dass** das Isocyanat-reaktive Monomer A1 ausgewählt wird aus 2,2-Bis(hydroxymethyl)propionsäure, 2,3-Diaminobenzoesäure, 2,4-Diaminobenzöesäure, 2,5-Diaminobenzoesäure, 2,6-Diaminobenzoesäure, 3,4-Diaminobenzoesäure, 3,5-Diaminobenzoesäure, 2,2-Di(hydroxymethyl)essigsäure, 2,2,2-Tri(hydroxymethyl)essigsäure, 2,2-Di(hydroxymethyl)propansäure, 2,2-Di(hydroxymethyl)butansäure, 2,2-Di(hydroxymethyl)pentansäure, 2,5-Dihydroxy-3-methylpentansäure, 3,5-Dihydroxy-3-methylpentansäure, 4,5-Dihydroxy-3-methylpentansäure, 3,4-Dihydroxy-3-methylpentansäure, 2,3-Dihydroxy-3-methylpentansäure, 2,4-Dihydroxy-3-methylpentansäure, 2,3-Dihydroxybenzoesäure, 2,4-Dihydroxybenzoesäure, 2,5-Dihydroxybenzoesäure, 2,6-Dihydroxybenzoesäure, 3,4-Dihydroxybenzoesäure, 3,5-Dihydroxybenzoesäure, 2,3-Dihydroxybernsteinsäure, 2,5-Diaminopentansäure, 3,5-Diaminopentansäure, 4,5-Diaminopentansäure, 2,3-Dihydroxybenzolsulfonsäure, 3,4-Dihydroxybenzolsulfonsäure, 2,4-Dihydroxybenzolsulfonsäure, 2,5-Dihydroxybenzolsulfonsäure, 3,5-Dihydroxybenzolsulfonsäure, 2,3-Diaminobenzolsulfonsäure, 3,4-Diaminobenzolsulfonsäure, 2,4-Diaminobenzolsulfonsäure, 2,5-Diaminobenzolsulfonsäure, 3,5-Diaminobenzoisulfonsäure, 3,4-Dihydroxy-2-Toluolsulfonsäure, 3,4-Diamino-2-Toluolsulfonsäure, 4,5-Dihydroxy-2-Toluolsulfonsäure, 4,5-Diamino-2-Toluolsulfonsäure, 5,6-Dihydroxy-2-Toluolsulfonsäure, 5,6-Diamino-2-Toluolsulfonsäure, 3,5-Dihydroxy-2-Toluolsulfonsäure, 3,5-Diamino-2-Toluolsulfonsäure, 3,6-Dihydroxy-2-Toluolsulfonsäure, 3,6-Diamino-2-Toluolsulfonsäure, 4,6-Dihydroxy-2-Toluolsulfonsäure, 4,6-Diamino-2-Toluolsulfonsäure, 2,4-Dihydroxy-3-Toluolsulfonsäure, 2,4-Diamino-3-Toluolsulfonsäure, 2,5-Dihydroxy-3-Toluolsulfonsäure, 2,5-Diamino-3-Toluolsulfonsäure, 2,6-Dihydroxy-3-Toluolsulfonsäure, 2,6-Diamino-3-Toluolsulfonsäure, 4,5-Dihydroxy-3-Toluolsulfonsäure, 4,5-Diamino-3-Toluolsulfonsäure, 4,6-Dihydroxy-3-Toluolsulfonsäure, 4,6-Diamino-3-Toluolsulfonsäure, 5,6-Dihydroxy-3-Toluolsulfonsäure, 5,6-Diamino-3-Toluolsulfonsäure, 2,3-Dihydroxy-4-Toluolsulfonsäure, 2,3-Diamino-4-Toluolsulfonsäure, 2,5-Dihydroxy-4-Toluolsulfonsäure, 2,5-Diamino-4-Toluolsulfonsäure, 2,6-Dihydroxy-4-Toluolsulfonsäure, 2,6-Diamino-4-Toluolsulfonsäure, 3,5-Dihydroxy-4-Toluolsulfonsäure, 3,5-Diamino-4-Toluolsulfonsäure, 3,6-Dihydroxy-4-Toluolsulfonsäure, 3,6-Diamino-4-Toluolsulfonsäure, 5,6-Dihydroxy-4-Toluolsulfonsäure, 5,6-Diamino-4-Toluolsulfonsäure oder Kombinationen davon.

6. Verfahren nach einem der vorangehenden Ansprüche, **dadurch gekennzeichnet, dass** als Isocyanat-reaktives Monomer neben mindestens einem Monomer A1 zusätzlich mindestens ein Monomer A2 eingesetzt wird, das ausgewählt wird aus nichtionischen Verbindungen enthaltend mindestens zwei funktionelle Gruppen, die jeweils mindestens ein Zerewitinoff-reaktives Wasserstoffatom tragen, wobei das Isocyanat-reaktive Monomer A2 insbesondere

• ausgewählt ist aus organischen Polyolen, Polyetherpolyolen, Polyesterpolyolen, Polycarbonatpolyolen, Polyetherestercarbonatpolyolen, Aminoalkoholen, organischen Polyaminen, organischen Polyalkylenaminen, oder

Kombinationen daraus, bevorzugt ausgewählt aus organischen Diolen, organischen Diaminen, organischen Polyalkylenglykolen, organischen Polyetherdiolen, organischen Polyesterdiolen, Polycarbonatdiolen, Polyetherestercarbonatdiolen oder Kombinationen daraus, und/oder
• ein zahlenmittleres Molekulargewicht Mn von 200 g/mol bis 10000 g/mol (bevorzugt von 400 g/mol bis 2500 g/mol) aufweist und vorzugsweise ausgewählt ist aus organischen Polyalkylenglykolen, organischen Polyetherdiolen, organischen Polyesterdiolen, oder Kombinationen daraus,
und/oder
• ein erstes Isocyanat-reaktives Monomer A2a mit zwei Zerewitinoff-aktiven Wasserstoffatomen und ein zweites Isocyanat-reaktives Monomer A2b mit drei Zerewitinoff-aktiven Wasserstoffatomen umfasst, wobei das erste Isocyanat-reaktive Monomer A2a ausgewählt ist aus Verbindungen mit zwei Hydroxylgruppen, Aminogruppen, Thiolgruppen, Ketimingruppen, Ketazingruppen, Oxazolidingruppen oder Kombinationen daraus und das zweite Isocyanat-reaktive Monomer A2b ausgewählt ist aus Verbindungen mit drei Hydroxylgruppen, Aminogruppen, Thiolgruppen, Ketimingruppen, Ketazingruppen, Oxazolidingruppen oder Kombinationen daraus.

**7.** Verfahren nach einem der vorangehenden Ansprüche, **dadurch gekennzeichnet, dass** als Tensid in Schritt c) mindestens ein nichtionisches Tensid, bevorzugt enthaltend mindestens eine Hydroxylgruppe, zugegeben wird.

**8.** Verfahren nach einem der vorangehenden Ansprüche, **dadurch gekennzeichnet, dass** mindestens ein Polyisocyanat-Monomer B ausgewählt ist aus organischen Diisocyanaten der nachstehenden Formel (I),

$$O=C=N-R-N=C=O \qquad (I)$$

worin R für einen cycloaliphatischen $C_{3-15}$ -Kohlenwasserstoffrest, aromatischen $C_{6-15}$ - Kohlenwasserstoffrest, araliphatischen $C_{6-18}$ -Kohlenwasserstoffrest oder aliphatischen $C_{3-15}$ -Kohlenwasserstoffrest, steht.

**9.** Verfahren nach einem der vorangehenden Ansprüche **dadurch gekennzeichnet, dass** das Stoffmengenverhältnis der Stoffmenge n(Zerewitinoff) der funktionellen Gruppen mit Zerewitinoff-aktiven Wasserstoffatomen aller eingesetzten Isocyanat-reaktiven Monomere zur Stoffmenge n(NCO) der Isocyanatgruppen aller eingesetzten Polyisocyanat-Monomere 5:1 bis 1:5, besonders bevorzugt 4:1 bis 1:4, ganz besonders bevorzugt 2:1 bis 1:3, am bevorzugtesten 1.5:1 bis 1:2, beträgt.

**10.** Partikelförmiges Feststoff-Kompositmaterial zur Nukleinsäureaufreinigung, enthaltend magnetische Nanopartikel eingebettet in eine Trägermatrix auf Basis mindestens eines Polymers, das durch Polyaddition von

a) mindestens einem Isocyanat-reaktiven-Monomer A, ausgewählt aus Verbindungen, enthaltend

- mindestens zwei funktionelle Gruppen, die jeweils mindestens ein Zerewitinoff-reaktives Wasserstoffatom, und
- zusätzlich zu diesen mindestens zwei funktionellen Gruppen mindestens einen anionischen oder potentiell anionischen Rest, bevorzugt ausgewählt aus Carboxylatgruppe, Sulfonatgruppe, oder Kombinationen daraus, tragen
mit

b) mindestens einem Polyisocyanat-Monomer B

erhalten wird,
mit der Maßgabe, dass besagte Polyaddition in Gegenwart magnetischer Nanopartikel erfolgt.

**11.** Partikelförmiges Feststoff-Kompositmaterial nach Anspruch 10, **dadurch gekennzeichnet, dass** das Polymer der Trägermatrix durch Reaktion

i) mindestens eines Isocyanat-reaktiven-Monomers A, ausgewählt aus Verbindungen, enthaltend mindestens zwei funktionelle Gruppen, die jeweils mindestens ein Zerewitinoff-reaktives Wasserstoffatom tragen mit
ii) mindestens einem Polyisocyanat-Monomer B

mittels Emulsionspolymerisation einer Emulsion, enthaltend

i) in der diskontinuierlichen Phase magnetische Nanopartikel, mindestens eine polare, organische Flüssigkeit,

bevorzugt mit einem cLogP-Wert < 1,5 (25°C) und mindestens ein von der polaren, organischen Flüssigkeit verschiedenes Isocyanat-reaktives-Monomer A, ausgewählt aus Verbindungen, enthaltend mindestens zwei funktionelle Gruppen, die jeweils mindestens ein Zerewitinoff-reaktives Wasserstoffatom tragen, und
ii) in der kontinuierlichen Phase mindestens eine unpolare, organische Flüssigkeit, bevorzugt mit einem cLogP-Wert > 2 (25°C), enthält,

in Gegenwart von Polyisocyanat-Monomer B erhalten wird, mit der Maßgabe, dass der cLogP-Wert (25°C) der polaren organischen Flüssigkeit kleiner als der cLogP-Wert (25°C) der unpolaren, organischen Flüssigkeit ist.

**12.** Partikelförmiges Feststoff-Kompositmaterial, insbesondere nach einem der Ansprüche 10 oder 11, **dadurch gekennzeichnet, dass** es gemäß einem Verfahren nach einem der Ansprüche 1 bis 9 erhalten wird.

**13.** Verwendung eines partikelförmigen Feststoff-Kompositmaterials nach einem der Ansprüche 10 bis 12 zur Aufreinigung von Nukleinsäuren.

**14.** Verfahren zur Aufreinigung von Nukleinsäuren aus einer Nukleinsäure-haltigen, biologischen Probe, umfassend die folgenden Schritte:

a) Bereitstellen einer flüssigen Probe, enthaltend Nukleinsäure in gelöster Form;
b) Abscheidung zumindest von Nukleinsäure aus der flüssigen Probe heraus an ein partikelförmiges Feststoff-Kompositmaterial nach einem der Ansprüche 10 bis 12;
c) Abtrennen des nukleinsäurehaltigen, partikelförmigen Feststoff-Kompositmaterials durch Anlegen eines Magnetfeldes und gegebenenfalls Waschen der abgetrennten Partikel mit einer Waschlösung;
d) Inlösungbringen der verbliebenen Nukleinsäure aus dem gemäß Schritt c) behandelten partikelförmigen Feststoff-Kompositmaterial mittels eines Lösungspuffers und Abtrennen des partikelförmigen Feststoff-Kompositmaterials von der nukleinsäurehaltigen Lösung.

**15.** Kit zur Aufreinigung von Nukleinsäuren umfassend ein partikelförmiges Feststoff-Kompositmaterial nach einem der Ansprüche 10 bis 12
sowie mindestens einen zusätzlichen Bestandteil, ausgewählt aus einer Bedienungsanleitung zur Durchführung eines Verfahrens nach Anspruch 14, Bindepuffer, Waschpuffer und Lösungspuffer zum Inlösungbringen der gereinigten Nukleinsäuren.

## Claims

**1.** A method for producing a particulate solid composite material for nucleic acid purification, containing magnetic nanoparticles embedded in a support matrix based on at least one polymer obtainable by polyaddition, **characterized in that** the method comprises at least the following steps:

a) providing a magnetic fluid in the form of a suspension containing magnetic nanoparticles and a liquid continuous phase containing at least one polar organic liquid and less than 5% by weight, preferably less than 2% by weight, of water, relative to the weight of the fluid,
b) mixing the magnetic fluid with at least one isocyanate-reactive monomer A1, selected from compounds containing

- at least two functional groups each having at least one Zerewitinoff-reactive hydrogen atom, and
- in addition to these at least two functional groups, at least one anionic or potentially anionic residue, preferably selected from a carboxylate group, sulfonate group, or combinations thereof,

c) preferably adding at least one surfactant,
d) emulsifying the mixture obtained in the previous steps in a liquid continuous phase containing at least one non-polar organic liquid,
e) adding at least one polyisocyanate monomer B,
f) after the reaction time is completed, separating the composite material formed, and optionally washing it,

with the proviso that the cLog P value (25 °C) of the polar organic liquid is smaller than the cLog P value (25 °C) of the non-polar organic liquid, and the liquid continuous phase from step d) contains less than 5% by weight, preferably

less than 2% by weight, of water, relative to its weight.

2. The method according to claim 1, **characterized in that** said polar organic liquid has a cLog P value < 1.5 (25 °C), especially a cLog P value < 1.0 (25 °C), and said non-polar organic liquid has a cLog P value > 2.0 (25 °C), especially a cLog P value > 2.5 (25 °C).

3. The method according to any of the preceding claims, **characterized in that** the magnetic nanoparticles are selected from ferromagnetic nanoparticles, ferrimagnetic nanoparticles, or a mixture thereof, preferably from superparamagnetic nanoparticles.

4. The method according to any of the preceding claims, **characterized in that** the magnetic nanoparticles comprise iron oxide.

5. The method according to any of the preceding claims, **characterized in that** the isocyanate-reactive monomer A1 is selected from 2,2-bis(hydroxymethyl)propionic acid, 2,3-diaminobenzoic acid, 2,4-diaminobenzoic acid, 2,5-diaminobenzoic acid, 2,6-diaminobenzoic acid, 3,4-diaminobenzoic acid, 3,5-diaminobenzoic acid, 2,2-bis(hydroxymethyl)acetic acid, 2,2,2-tri(hydroxymethyl)acetic acid, 2,2-bis(hydroxymethyl)propionic acid, 2,2-bis(hydroxymethyl)butyric acid, 2,2-bis(hydroxymethyl)pentanoic acid, 2,5-dihydroxy-3-methylpentanoic acid, 3,5-dihydroxy-3-methylpentanoic acid, 4,5-dihydroxy-3-methylpentanoic acid, 3,4-dihydroxy-3-methyl-pentanoic acid, 2,3-dihydroxy-3-methylpentanoic acid, 2,4-dihydroxy-3-methylpentanoic acid, 2,3-dihydroxybenzoic acid, 2,4-dihydroxybenzoic acid, 2,5-dihydroxybenzoic acid, 2,6-dihydroxybenzoic acid, 3,4-dihydroxybenzoic acid, 3,5-dihydroxybenzoic acid, 2,3-dihydroxysuccinic acid, 2,5-diaminopentanoic acid, 3,5-diaminopentanoic acid, 4,5-diaminopentanoic acid, 2,3-dihydroxybenzenesulfonic acid, 3,4-dihydroxybenzenesulfonic acid, 2,4-dihydroxybenzenesulfonic acid, 2,5-dihydroxybenzenesulfonic acid, 3,5-dihydroxybenzenesulfonic acid, 2,3-diaminobenzenesulfonic acid, 3,4-diaminobenzenesulfonic acid, 2,4-diaminobenzenesulfonic acid, 2,5-diaminobenzenesulfonic acid, 3,5-diaminobenzenesulfonic acid, 3,4-dihydroxy-2-toluenesulfonic acid, 3,4-diamino-2-toluenesulfonic acid, 4,5-dihydroxy-2-toluenesulfonic acid, 4,5-diamino-2-toluenesulfonic acid, 5,6-dihydroxy-2-toluenesulfonic acid, 5,6-diamino-2-toluenesulfonic acid, 3,5-dihydroxy-2-toluenesulfonic acid, 3,5-diamino-2-toluenesulfonic acid, 3,6-dihydroxy-2-toluenesulfonic acid, 3,6-diamino-2-toluenesulfonic acid, 4,6-dihydroxy-2-toluenesulfonic acid, 4,6-diamino-2-toluenesulfonic acid, 2,4-dihydroxy-3-toluenesulfonic acid, 2,4-diamino-3-toluenesulfonic acid, 2,5-dihydroxy-3-toluenesulfonic acid, 2,5-diamino-3-toluenesulfonic acid, 2,6-dihydroxy-3-toluenesulfonic acid, 2,6-diamino-3-toluenesulfonic acid, 4,5-dihydroxy-3-toluenesulfonic acid, 4,5-diamino-3-toluenesulfonic acid, 4,6-dihydroxy-3-toluenesulfonic acid, 4,6-diamino-3-toluenesulfonic acid, 5,6-dihydroxy-3-toluenesulfonic acid, 5,6-diamino-3-toluenesulfonic acid, 2,3-dihydroxy-4-toluenesulfonic acid, 2,3-diamino-4-toluenesulfonic acid, 2,5-dihydroxy-4-toluenesulfonic acid, 2,5-diamino-4-toluenesulfonic acid, 2,6-dihydroxy-4-toluenesulfonic acid, 2,6-diamino-4-toluenesulfonic acid, 3,5-dihydroxy-4-toluenesulfonic acid, 3,5-diamino-4-toluenesulfonic acid, 3,6-dihydroxy-4-toluenesulfonic acid, 3,6-diamino-4-toluenesulfonic acid, 5,6-dihydroxy-4-toluenesulfonic acid, 5,6-diamino-4-toluenesulfonic acid, or combinations thereof.

6. The method according to any of the preceding claims, **characterized in that**, in addition to at least one monomer A1, at least one additional monomer A2 is used as isocyanate-reactive monomer, selected from non-ionic compounds containing at least two functional groups each carrying at least one Zerewitinoff-reactive hydrogen atom, whereby the isocyanate-reactive monomer A2, in particular,

• is selected from organic polyols, polyether polyols, polyester polyols, polycarbonate polyols, polyetherestercarbonate polyols, amino alcohols, organic polyamines, organic polyalkyleneamines, or combinations thereof, preferably selected from organic diols, organic diamines, organic polyalkylene glycols, organic polyetherdiols, organic polyesterdiols, polycarbonatediols, polyetherestercarbonate diols, or combinations thereof,
and/or
• has a number average molecular weight Mn of from 200 g/mol to 10,000 g/mol (preferably from 400 g/mol to 2500 g/mol), and is preferably selected from organic polyalkylene glycols, organic polyetherdiols, organic polyesterdiols, or combinations thereof,
and/or
• comprises a first isocyanate-reactive monomer A2a having two Zerewitinoff-reactive hydrogen atoms and a second isocyanate-reactive monomer A2b having three Zerewitinoff-reactive hydrogen atoms, wherein the first isocyanate-reactive monomer A2a is selected from compounds having two hydroxy groups, amino groups, thiol groups, ketimine groups, ketazine groups, oxazolidine groups, or combinations thereof, and the second isocyanate-reactive monomer A2b is selected from compounds having three hydroxy groups, amino groups, thiol groups, ketimine groups, ketazine groups, oxazolidine groups, or combinations thereof.

**7.** The method according to any of the preceding claims, **characterized in that** at least one non-ionic surfactant, preferably one containing at least one hydroxy group, is added as surfactant in step c).

**8.** The method according to any of the preceding claims, **characterized in that** at least one polyisocyanate monomer B is selected from organic diisocyanates of the following formula (I),

$$O{=}C{=}N{-}R{-}N{=}C{=}O \qquad (I),$$

wherein R represents a cycloaliphatic $C_{3-15}$ hydrocarbon residue, aromatic $C_{6-15}$ hydrocarbon residue, araliphatic $C_{6-18}$ hydrocarbon residue, or aliphatic $C_{3-15}$ hydrocarbon residue.

**9.** The method according to any of the preceding claims, **characterized in that** the molar ratio between the number of moles n(Zerewitinoff) of the functional groups having Zerewitinoff-active hydrogen atoms of all isocyanate reactive monomers used to the number of moles n(NCO) of the isocyanate groups of all polyisocyanate monomers used is from 5:1 to 1:5, more preferably from 4:1 to 1:4, even more preferably from 2:1 to 1:3, most preferably from 1.5:1 to 1:2.

**10.** A particulate solid composite material for nucleic acid purification, containing magnetic nanoparticles embedded in a support matrix based on at least one polymer that is obtained by the polyaddition of

a) at least one isocyanate-reactive monomer A selected from compounds containing

- at least two functional groups each having at least one Zerewitinoff-reactive hydrogen atom, and
- in addition to these at least two functional groups, at least one anionic or potentially anionic residue, preferably selected from a carboxylate group, sulfonate group, or combinations thereof,

with

b) at least one polyisocyanate monomer B,

with the proviso that said polyaddition occurs in the presence of magnetic nanoparticles.

**11.** The particulate solid composite material according to claim 10, **characterized in that** the polymer of the support matrix is obtained by the reaction of

i) at least one isocyanate-reactive Monomer A, selected from compounds containing at least two functional groups, each carrying at least one Zerewitinoff-reactive hydrogen atom, with
ii) at least one polyisocyanate monomer B

by means of emulsion polymerization of an emulsion containing

i) in the discontinuous phase: magnetic nanoparticles, at least one polar organic liquid, preferably having a cLog P value < 1.5 (25 °C), and at least one isocyanate-reactive monomer A other than said polar organic liquid, selected from compounds containing at least two functional groups each carrying at least one Zerewitinoff-reactive hydrogen atom, and
ii) in the continuous phase: at least one non-polar, organic liquid, preferably having a cLog P value > 2 (25 °C),

in the presence of polyisocyanate monomer B, with the proviso that the cLog P value (25 °C) of the polar organic liquid is smaller than the Log P value (25 °C) of the non-polar organic liquid.

**12.** A particulate solid composite material, especially one according to either of claims 10 or 11, **characterized by** being obtained using a method according to any of claims 1 to 9.

**13.** Use of a particulate solid composite material according to any of claims 10 to 12 for purifying nucleic acids.

**14.** A method for the purification of nucleic acids from a nucleic acid-containing biological sample, comprising the following steps:

a) providing a liquid sample containing nucleic acids in solubilized form;
b) depositing at least nucleic acids from the liquid sample onto a particulate solid composite material according

to any of claims 10 to 12;
c) separating the nucleic acid-containing particulate solid composite material by applying a magnetic field, and optionally washing the separated particles with a wash solution;
d) solubilizing the remaining nucleic acid from the particulate solid composite material treated according to step c) using a solution buffer, and separating the particulate solid composite material from the nucleic acid-containing solution.

**15.** A kit for purifying nucleic acids comprising a particulate solid composite material according to any of claims 10 to 12, and at least one additional component selected from a user manual for performing the method according to claim 14, binding buffers, wash buffers, and solution buffers for solubilizing the purified nucleic acids.

## Revendications

**1.** Procédé de production d'un matériau composite solide particulaire destiné à la purification d'acide nucléique, contenant des nanoparticules magnétiques incorporées dans une matrice de support à base d'au moins un polymère pouvant être obtenu par polyaddition, **caractérisé en ce que** le procédé comprend au moins les étapes suivantes consistant à :

a) fournir un fluide magnétique sous la forme d'une suspension, contenant des nanoparticules magnétiques et une phase liquide continue contenant au moins un liquide organique polaire et, par rapport au poids du fluide, moins de 5 % en poids, de préférence moins de 2 % en poids, d'eau,
b) mélanger le fluide magnétique avec au moins un monomère réactif à un isocyanate A1 choisi parmi des composés contenant

- au moins deux groupes fonctionnels, qui portent chacun au moins un atome d'hydrogène réactif de type Zerewitinoff et
- en plus de ces groupes fonctionnels au moins au nombre de deux, au moins un radical anionique ou potentiellement anionique, de préférence choisi parmi un groupe carboxylate, un groupe sulfonate ou une combinaison de ceux-ci,

c) ajouter de préférence au moins un tensioactif,
d) émulsionner le mélange des étapes précédentes en une phase liquide continue contenant au moins un liquide organique non polaire,
e) ajouter au moins un monomère de polyisocyanate B,
f) après achèvement du temps de réaction, séparer le matériau composite formé, et facultativement le laver,

à la condition que la valeur cLogP (25°C) du liquide organique polaire soit inférieure à la valeur cLogP (25°C) du liquide organique non polaire et que la phase liquide continue de l'étape d), par rapport à son poids, contienne moins de 5 % en poids, de préférence moins de 2 % en poids, d'eau.

**2.** Procédé selon la revendication 1, **caractérisé en ce que** ledit liquide organique polaire a une valeur cLogP < 1,5 (25°C), en particulier une valeur cLogP < 1,0 (25°C), et ledit liquide organique non polaire a une valeur cLogP > 2,0 (25°C), en particulier une valeur cLogP > 2,5 (25°C).

**3.** Procédé selon l'une des revendications précédentes, **caractérisé en ce que** les nanoparticules magnétiques sont choisies parmi des nanoparticules ferromagnétiques, des nanoparticules ferrimagnétiques, ou un mélange de celles-ci, de préférence parmi des nanoparticules superparamagnétiques.

**4.** Procédé selon l'une des revendications précédentes, **caractérisé en ce que** les nanoparticules magnétiques comprennent de l'oxyde de fer.

**5.** Procédé selon l'une des revendications précédentes, **caractérisé en ce que** le monomère réactif à un isocyanate A1 est choisi parmi l'acide 2,2-bis(hydroxyméthyl)propionique, l'acide 2,3-diaminobenzoïque, l'acide 2,4-diaminobenzoïque, l'acide 2,5-diaminobenzoïque, l'acide 2,6-diaminobenzoïque, l'acide 3,4-diaminobenzoïque, l'acide 3,5-diaminobenzoïque, l'acide 2,2-di(hydroxyméthyl)acétique, l'acide 2,2,2-tri (hydroxyméthyl)acétique, l'acide 2,2-di(hydroxyméthyl)propanoïque, l'acide 2,2-di(hydroxyméthyl)butanoïque, l'acide 2,2-di(hydroxyméthyl) pentanoïque, l'acide 2,5-dihydroxy-3-méthylpentanoïque, l'acide 3,5-dihydroxy-3-méthylpentanoïque, l'acide 4,5-dihydroxy-3-méthylpentanoïque, l'acide 3,4-dihydroxy-3-méthylpentanoïque, l'acide 2,3-dihydroxy-3-méthylpentanoïque, l'acide 2,4-dihydroxy-3-méthylpentanoïque, l'acide 2,3-dihydroxybenzoïque, l'acide 2,4-dihydroxybenzoïque, l'acide 2,5-dihydroxybenzoïque, l'acide 2,6-dihydroxybenzoïque, l'acide 3,4-dihydroxybenzoïque, l'acide 3,5-dihydroxybenzoïque, l'acide 2,3-dihydroxysuccinique, l'acide 2,5-diaminopentanoïque, l'acide 3,5-diaminopentanoïque, l'acide 4,5-diaminopentanoïque, l'acide 2,3-dihydroxybenzènesulfonique, l'acide 3,4-dihydroxybenzènesulfonique, l'acide 2,4-dihydroxybenzènesulfonique, l'acide 2,5-dihydroxybenzènesulfonique, l'acide 3,5-dihydroxybenzènesulfonique, l'acide 2,3-diaminobenzènesulfonique, l'acide 3,4-diaminobenzènesulfonique, l'acide 2,4-diaminobenzènesulfonique, l'acide 2,5-diaminobenzènesulfonique, l'acide 3,5-diaminobenzènesulfonique, l'acide 3,4-dihydroxy-2-toluènesulfonique, l'acide 3,4-diamino-2-toluènesulfonique, l'acide 4,5-dihydroxy-2-toluènesulfonique, l'acide 4,5-diamino-2-toluènesulfonique, l'acide 5,6-dihydroxy-2-toluènesulfonique, l'acide 5,6-diamino-2-toluènesulfonique, l'acide 3,5-dihydroxy-2-toluènesulfonique, l'acide 3,5-diamino-2-toluènesulfonique, l'acide 3,6-dihydroxy-2-toluènesulfonique, l'acide 3,6-diamino-2-toluènesulfonique, l'acide 4,6-dihydroxy-2-toluènesulfonique, l'acide 4,6-diamino-2-toluènesulfonique, l'acide 2,4-dihydroxy-3-toluènesulfonique, l'acide 2,4-diamino-3-toluènesulfonique, l'acide 2,5-dihydroxy-3-toluènesulfonique, l'acide 2,5-diamino-3-toluènesulfonique, l'acide 2,6-dihydroxy-3-toluènesulfonique, l'acide 2,6-diamino-3-toluènesulfonique, l'acide 4,5-dihydroxy-3-toluènesulfonique, l'acide 4,5-diamino-3-toluènesulfonique, l'acide 4,6-dihydroxy-3-toluènesulfonique, l'acide 4,6-diamino-3-toluènesulfonique, l'acide 5,6-dihydroxy-3-toluènesulfonique, l'acide 5,6-diamino-3-toluènesulfonique, l'acide 2,3-dihydroxy-4-toluènesulfonique, l'acide 2,3-diamino-4-toluènesulfonique, l'acide 2,5-dihydroxy-4-toluènesulfonique, l'acide 2,5-diamino-4-toluènesulfonique, l'acide 2,6-dihydroxy-4-toluènesulfonique, l'acide 2,6-diamino-4-toluènesulfonique, l'acide 3,5-dihydroxy-4-toluènesulfonique, l'acide 3,5-diamino-4-toluènesulfonique, l'acide 3,6-dihydroxy-4-toluènesulfonique, l'acide 3,6-diamino-4-toluènesulfonique, l'acide 5,6-dihydroxy-4-toluènesulfonique, l'acide 5,6-diamino-4-toluènesulfonique, ou des combinaisons de ceux-ci.

**6.** Procédé selon l'une des revendications précédentes, **caractérisé en ce qu'**on utilise en tant que monomère réactif à un isocyanate, en plus d'au moins un monomère A1, au moins un monomère A2, qui est choisi parmi des composés non ioniques contenant au moins deux groupes fonctionnels, qui portent chacun au moins un atome d'hydrogène réactif de type Zerewitinoff, où le monomère réactif à un isocyanate A2, en particulier

- est choisi parmi des polyols organiques, des polyéther polyols, des polyester polyols, des polycarbonate polyols, des polyéthersestercarbonate polyols, des aminoalcools, des polyamines organiques, des polyalkylène amines organiques, ou des combinaisons de ceux-ci, choisi de préférence parmi des diols organiques, des diamines organiques, des polyalkylène glycols organiques, des polyéther diols organiques, des polyester diols organiques, des polycarbonate diols, des polyétherestercarbonate diols, ou des combinaisons de ceux-ci, et/ou
- présente une masse moléculaire moyenne en nombre Mn de 200 g/mole à 10 000 g/mole (de préférence de 400 g/mole à 2 500 g/mole), et est de préférence choisi parmi des polyalkylène glycols organiques, des polyéther diols organiques, des polyesterdiols organiques ou des combinaisons de ceux-ci, et/ou
- comprend un premier monomère réactif à un isocyanate A2a ayant deux atomes d'hydrogène actifs de type Zerewitinoff et un second monomère réactif à un isocyanate A2b ayant trois atomes d'hydrogène actifs de type Zerewitinoff, où le premier monomère réactif à un isocyanate A2a est choisi parmi des composés comportant deux groupes hydroxyle, groupes amino, groupes thiol, groupes cétimine, groupes cétazine, groupes oxazolidine, ou des combinaisons de ceux-ci, et le second monomère réactif à un isocyanate A2b est choisi parmi des composés comportant trois groupes hydroxyle, groupes amino, groupes thiol, groupes cétimine, groupes cétazine, groupes oxazolidine, ou des combinaisons de ceux-ci.

**7.** Procédé selon l'une des revendications précédentes, **caractérisé en ce que** l'on ajoute au moins un tensioactif non ionique en tant que tensioactif à l'étape c), contenant de préférence au moins un groupe hydroxyle.

**8.** Procédé selon l'une des revendications précédentes, **caractérisé en ce qu'**au moins un monomère de polyisocyanate B est choisi parmi des diisocyanates organiques de formule (I) suivante,

$$O = C = N\text{-}R\text{-}N = C = O \qquad (I)$$

dans laquelle R est un radical hydrocarbure cycloaliphatique en $C_3$ à $C_{15}$, un radical hydrocarbure aromatique en $C_6$ à $C_{15}$, un radical hydrocarbure araliphatique en $C_6$ à $C_{18}$, ou un radical hydrocarbure aliphatique en $C_3$ à $C_{15}$.

**9.** Procédé selon l'une des revendications précédentes, **caractérisé en ce que** le rapport molaire de la quantité de matière n(Zerewitinoff) des groupes fonctionnels avec atomes d'hydrogène actifs de type Zerewitinoff de tous les monomères réactifs à un isocyanate à la quantité de matière n(NCO) des groupes isocyanate de tous les monomères

de polyisocyanate utilisés est de 5:1 à 1 5, de préférence de 4:1 à 1:4, de manière très préférée de 2:1 à 1:3, de manière la plus préférée de 1,5:1 à 1:2.

**10.** Matériau composite solide particulaire destiné à la purification d'acides nucléiques, contenant des nanoparticules magnétiques incorporées dans une matrice de support à base d'au moins un polymère qui est obtenu par polyaddition

a) d'au moins un monomère réactif à un isocyanate A choisi parmi des composés contenant

- au moins deux groupes fonctionnels, chacun ayant au moins un atome d'hydrogène réactif de type Zerewitinoff, et
- en plus de ces groupes fonctionnels au moins au nombre de deux, au moins un radical anionique ou potentiellement anionique, choisi de préférence parmi un groupe carboxylate, un groupe sulfonate, ou des combinaisons de ceux-ci,
avec

b) au moins un monomère B de polyisocyanate,
à la condition que ladite polyaddition se produise en présence de nanoparticules magnétiques.

**11.** Matériau composite solide particulaire selon la revendication 10, **caractérisé en ce que** le polymère de la matrice de support est obtenu par réaction

i) d'au moins un monomère réactif à un isocyanate A, choisi parmi des composés contenant au moins deux groupes fonctionnels, portant chacun au moins un atome d'hydrogène réactif de type Zerewitinoff avec
ii) au moins un monomère de polyisocyanate B

par l'intermédiaire d'une polymérisation en émulsion d'une émulsion, contenant

i) dans la phase discontinue des nanoparticules magnétiques, au moins un liquide organique polaire, de préférence ayant une valeur cLogP $< 1,5$ (25°C) et au moins un monomère réactif à un isocyanate A différent du liquide polaire organique choisi parmi des composés contenant au moins deux groupes fonctionnels portant chacun au moins un atome d'hydrogène réactif de type Zerewitinoff, et
ii) dans la phase continue, au moins un liquide organique non polaire, de préférence ayant une valeur cLogP $> 2$ (25°C),

en présence d'un monomère de polyisocyanate B, à la condition que la valeur cLogP (25°C) du liquide organique polaire soit inférieure à la valeur cLogP (25°C) du liquide organique non polaire.

**12.** Matériau composite solide particulaire, en particulier selon l'une des revendications 10 ou 11, **caractérisé en ce qu'**il est obtenu selon un procédé selon l'une des revendications 1 à 9.

**13.** Utilisation d'un matériau composite solide particulaire selon l'une quelconque des revendications 10 à 12 pour la purification d'acides nucléiques.

**14.** Procédé de purification d'acides nucléiques à partir d'un échantillon biologique contenant des acides nucléiques, comprenant les étapes suivantes consistant à :

a) fournir un échantillon liquide contenant de l'acide nucléique sous une forme dissoute ;
b) faire se déposer au moins un acide nucléique provenant de l'échantillon liquide sur un matériau composite solide particulaire selon l'une quelconque des revendications 10 à 12 ;
c) séparer le matériau composite solide particulaire contenant de l'acide nucléique en appliquant un champ magnétique, et éventuellement laver les particules séparées avec une solution de lavage ;
d) dissoudre l'acide nucléique restant à partir du matériau composite solide particulaire traité selon l'étape c) au moyen d'un tampon de solution et séparer le matériau composite solide particulaire de la solution contenant de l'acide nucléique.

**15.** Kit de purification d'acides nucléiques comprenant un matériau composite solide particulaire selon l'une des revendications 10 à 12 ainsi qu'au moins un composant supplémentaire choisi dans des instructions pour la mise en œuvre d'un procédé selon la revendication 14, un tampon de liaison, un tampon de lavage et un tampon de solution

permettant la dissolution des acides nucléiques purifiés.

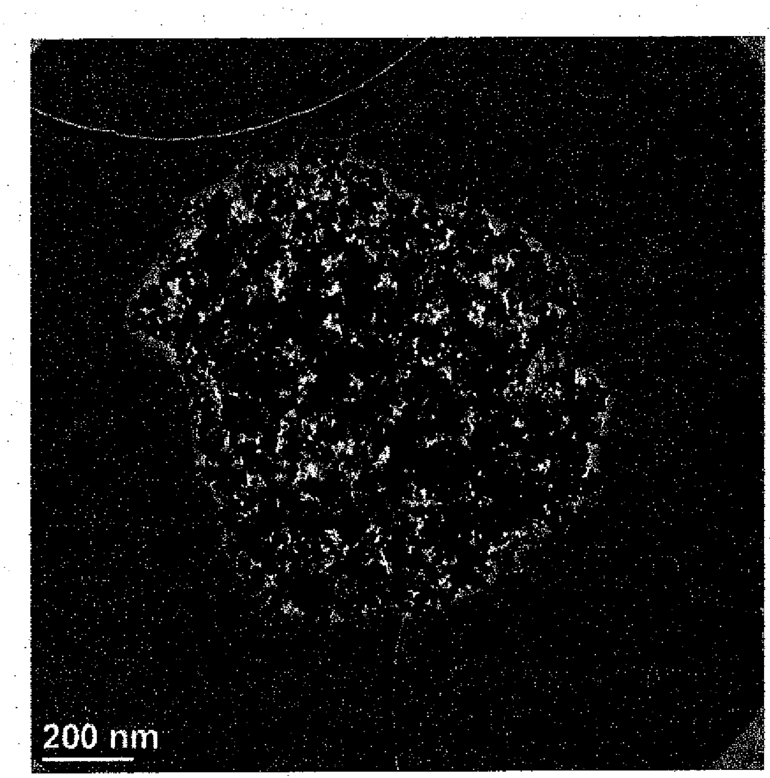


Fig.1

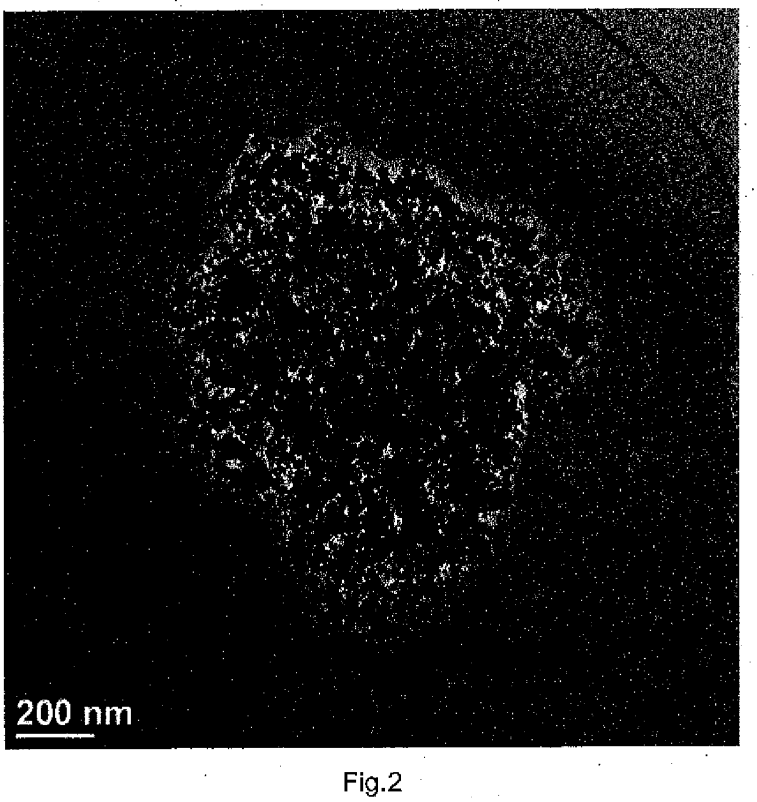


Fig.2

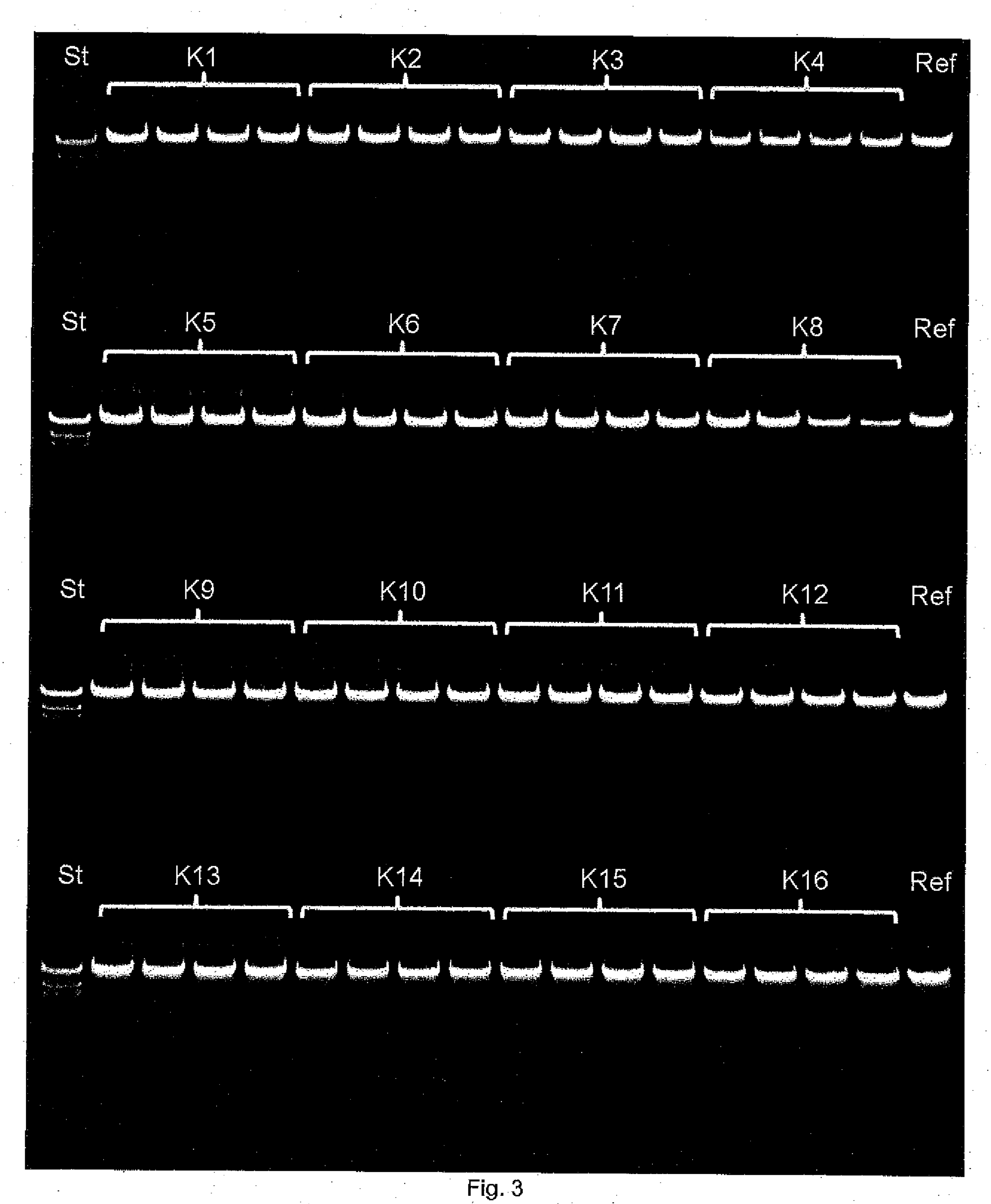
St
K1
K2
K3
K4
Ref
St
K5
K6
K7
K8
Ref
St
K9
K10
K11
K12
Ref
St
K13
K14
K15
K16
Ref

Fig. 3

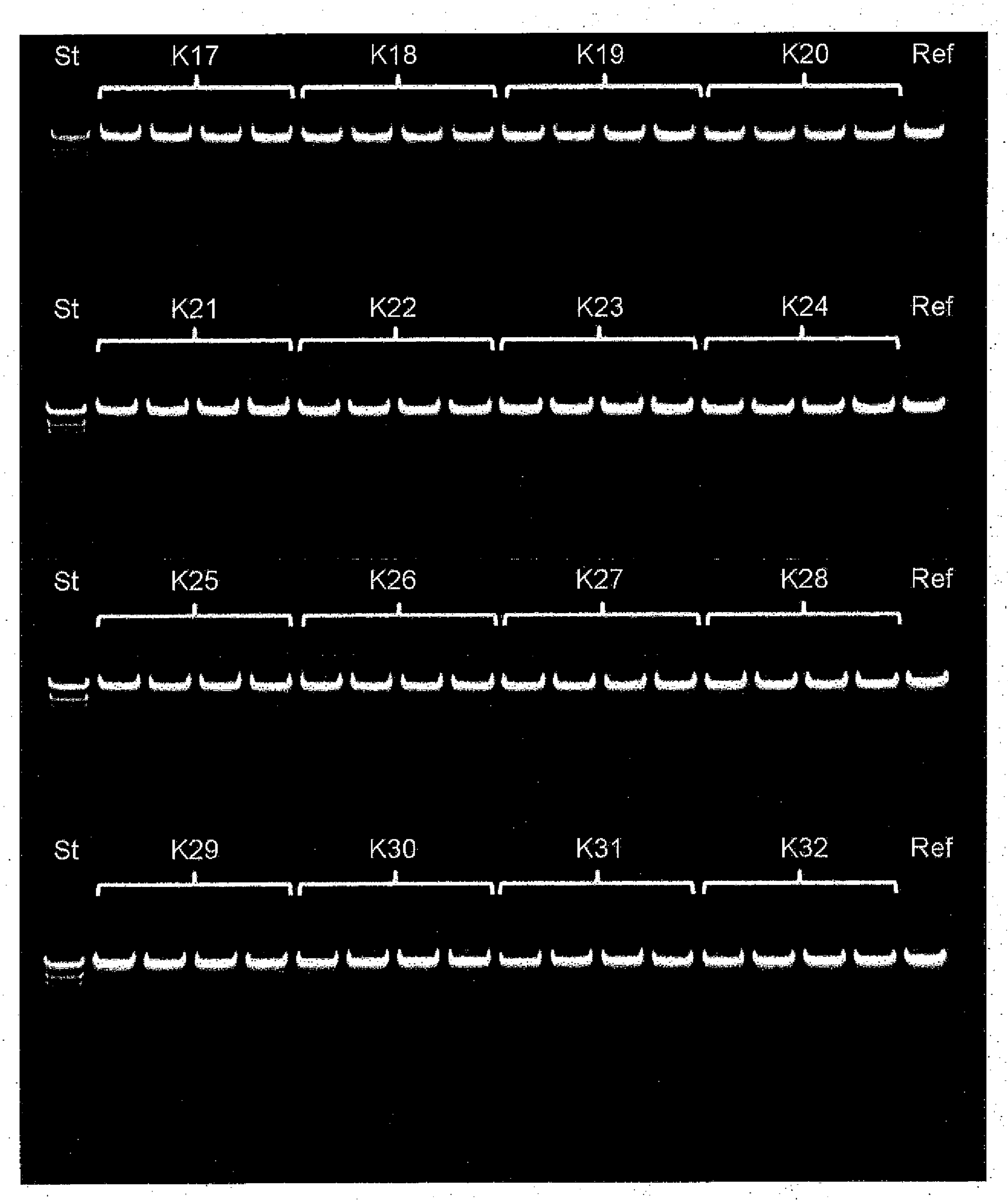
St
K17
K18
K19
K20
Ref
St
K21
K22
K23
K24
Ref
St
K25
K26
K27
K28
Ref
St
K29
K30
K31
K32
Ref

Fig. 4

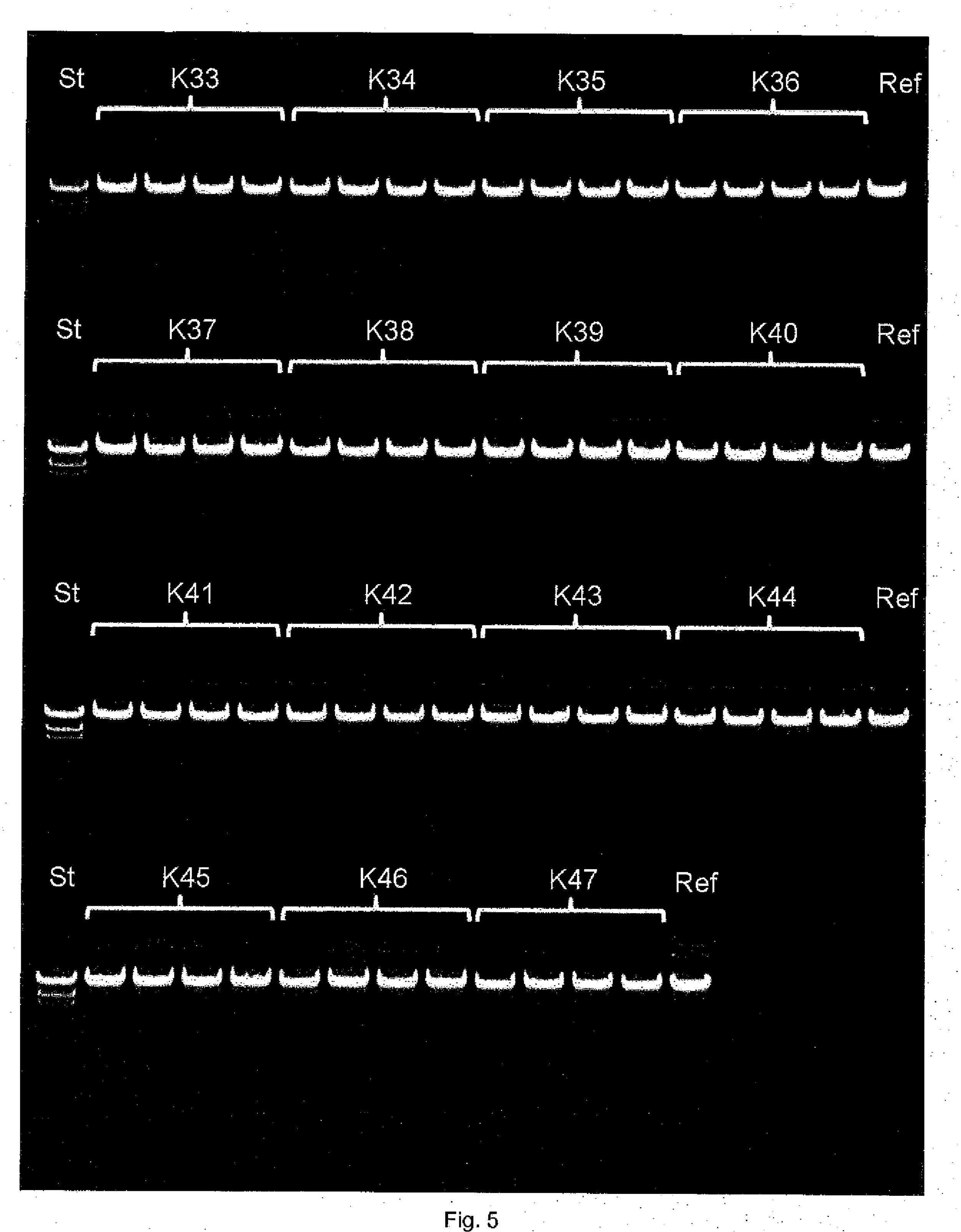
St
K33
K34
K35
K36
Ref
St
K37
K38
K39
K40
Ref
St
K41
K42
K43
K44
Ref
St
K45
K46
K47
Ref

Fig. 5

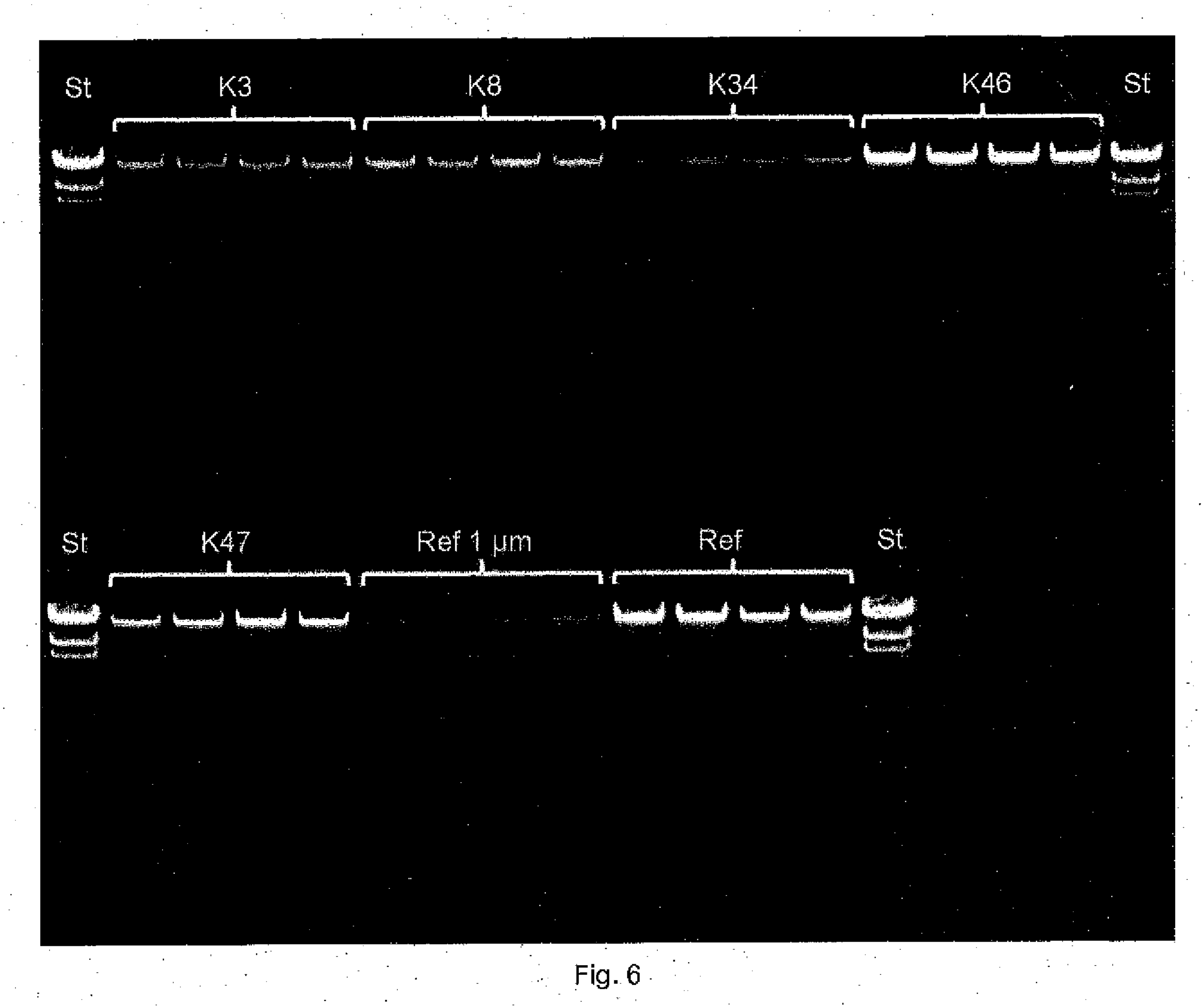


Fig. 6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1266385 B1
- EP 2916327 A1
- WO 8303920 A1
- WO 9704862 A1
- US 4267234 A
- EP 1404442 A
- US 5648124 A
- US 7989614 B2
- US 8945509 B2
- WO 2005015216 A1
- EP 2051075 A1
- WO 20061075185 A1
- EP 0106873 A


### In der Beschreibung aufgeführte Nicht-Patentliteratur


- **J.-C. BACRI ; R. PERZYNSKI ; D. SALIN ; V. CABUIL ; R. MASSART.** Ionic Ferrofluids: A crossing of chemistry and physics. *J. Magn. Magn. Mater.,* 1990, vol. 85, 27-32
- ACD/Log P method description. *Drug Discovery and Design,* 2000, vol. 19, 99-116